(19) Europäisches Patentamt
European Patent Office
Office européen des brevets

(11) Numéro de publication: **0 292 365 A2**

(12) # DEMANDE DE BREVET EUROPEEN

(21) Numéro de dépôt: 88401158.6

(22) Date de dépôt: 11.05.88

(51) Int. Cl.⁴: **C 07 D 239/94**
C 07 D 239/86,
A 61 K 31/505, C 07 C 79/46,
C 07 C 101/72

(30) Priorité: 14.05.87 FR 8706795

(43) Date de publication de la demande:
23.11.88 Bulletin 88/47

(84) Etats contractants désignés:
AT BE CH DE ES FR GB GR IT LI LU NL SE

(71) Demandeur: CENTRE NATIONAL DE LA RECHERCHE SCIENTIFIQUE
15, quai Anatole France
F-75007 Paris (FR)

UNIVERSITE RENE DESCARTES (PARIS V)
4, avenue de l'observatoire
F-75270 Paris Cédex 06 (FR)

(72) Inventeur: Renault, Jean Armand Paul
17 rue de Sèvres
F-75006 Paris (FR)

Giorgi, Sylvianne Madeleine Jeanne
7 rue Fustel de Coulanges
F-75005 Paris (FR)

Gebel, Patricia Nadine Jeanne
73 rue Roger Salengro
F-93140 Bondy (FR)

Baron, Michel Jean-Pierre
15 rue Paul Messein
F-95160 Montmorency (FR)

Paoletti, Claude Antoine
84 rue Michel Ange
F-75016 Paris (FR)

Cros, Suzanne Blanche Georgette
7 rue du Coustet
F-31520 Ramonville St-Agne (FR)

(74) Mandataire: Grosset-Fournier, Chantal Catherine et al
SC Ernest Gutmann/Yves Plasseraud 67 boulevard Haussmann
F-75008 Paris (FR)

(54) **Dérivés de l'amino-4 quinazolinedione-5,8, leur procédé de préparation et compositions pharmaceutiques les contenant.**

(57) L'invention a pour objet des composés répondant à la formule suivante :

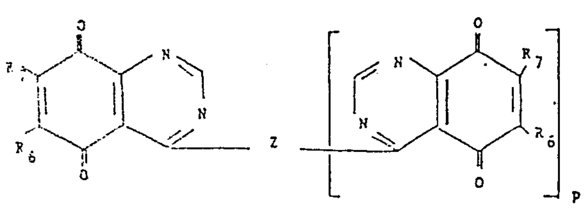

I

dans laquelle :
- p est égal à 0 ou 1,
- $R_7$ représente un atome d'hydrogène
- et $R_6$ peut représenter notamment un groupe alcoxy de 1 à 5 atomes de carbone,
et lorsque p est égal à 0, Z représente B,
. B représentant un groupe de formule
- NH - $R_4$ X
dans lequel
- $R_4$ peut représenter notamment une chaîne alkyle de 1 à 7 atomes de carbone,
- X représente notamment un atome d'hydrogène, et lorsque p est égal à 1, Z représente A...A,
. A...A représentant notamment une chaîne de formule
- NH - Y - NH -
. Y représentant notamment une chaîne alkyle linéaire de 2 à 10 atomes de carbone.

Ces composés entrent dans la préparation de compositions pharmaceutiques.

EP 0 292 365 A2

**Description**

**DERIVES DE L'AMINO-4 QUINAZOLINEDIONE-5,8, LEUR PROCEDE DE PREPARATION ET COMPOSITIONS PHARMACEUTIQUES LES CONTENANT**

L'invention concerne des dérivés de l'amino-4 quinazolinedione-5,8, leur procédé de préparation et les compositions pharmaceutiques les contenant.

Diverses molécules aromatiques ou hétérocycliques nécessitent une structure quinone pour manifester une activité antitumorale (anthracyclines, streptonigrine, lapachol, mytomycine C). Il est bien connu que l'élément structural essentiel pour l'activité antitumorale de la streptonigrine est le noyau amino-7 méthoxy-6 quinolinedione-5,8 (Rao, K.V., Cancer Chemother. Rep. Part 2, 1974, 4(4), 11).

Divers analogues du noyau sus-mentionné ont déjà fait l'objet de nombreuses études. Il a ainsi été déterminé que les dérivés du type acridinedione-1,4 (Eur. J. Med. Chem. 1981, 16, pp. 24-34) sont cytotoxiques in vitro sur la cellule leucémique L1210, mais ne présentent aucun activité antitumorale sur la souris atteinte de cette leucémie.

On a également synthétisé et testé des quinoxalinediones-5,6, des quinoxalinediones-5,8 (Eur. J. Med. Chem. 1981, 16, pp. 545-559), mais aucun de ces composés ne présente in vitro de cytotoxicité notable sur la cellule leucémique L1210.

On a également synthétisé des benzo[f] quinolinediones-7,10 (Eur. J. Med. Chem. 1983, 18, pp. 134-138) et déterminé qu'ils présentent une certaine toxicité in vitro sur les cellules L1210, mais que celle-ci reste insuffisante pour autoriser des essais in vivo.

Enfin, des études réalisées sur les quinazolinediones-5,8, notamment la bis(aziridinyl-1)-6,7 quinazolinedione-5,8 (J. Med. Chem. 1983, 26, pp. 1715-1719) ont permis de déterminer que ce dernier composé est cytotoxique in vitro sur les cellules L1210, et qu'il présente une certaine activité antitumorale in vivo sur la leucémie murine P388. Toutefois, ce composé ne présente pas d'activité antitumorale significative in vivo sur la leucémie L1210, le sarcome 180 et ne présente qu'une très faible activité in vivo sur le mélanocarcinome B16. D'autre part, ce composé n'est pas actif par voie orale.

Or, la Société Demanderesse a trouvé de façon surprenante qu'en substituant la position 4 de certaines quinazolinediones-5,8, - ne présentant elles-mêmes qu'une activité antitumorale très réduite - à l'aide de substituants appropriés, ou en dimérisant, par l'intermédiaire de la position 4, certaines quinazolinediones-5,8 par l'intermédiaire de chaînes appropriées, on obtient des dérivés de quinazolinediones-5,8 substitués en 4 ou des dimères de quinazolinediones-5,8, notamment actifs vis-à-vis d'un large spectre de tumeurs, et non toxiques aux doses auxquelles ils sont administrés, contrairement aux quinazolinediones-5,8 non substitués en 4 ou non dimérisés.

L'un des aspects de l'invention est de proposer de nouveaux composés actifs in vivo sur un large spectre de tumeurs expérimentales.

L'un des autres aspects de l'invention est de proposer de nouveaux composés qui sont actifs à des doses très inférieures au seuil de toxicité.

Un autre aspect de l'invention est de proposer de nouveaux composés présentent un bon indice thérapeutique.

Un autre aspect de l'invention est de proposer de nouveaux composés ayant des propriétés biologiques permettant leur utilisation en thérapeutique.

Un autre aspect de l'invention est de proposer de nouveaux composés actifs, non seulement par voie intraveineuse ou sous-cutanée, mais également par voie orale.

Les composés selon l'invention répondent à la formule I suivante :

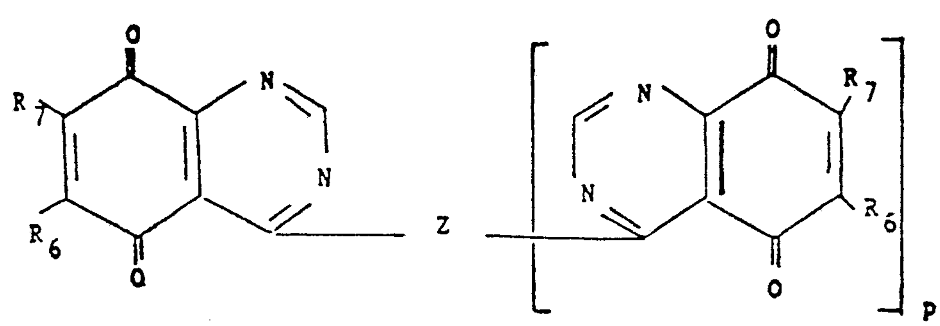

I

dans laquelle :

- p est égal à 0 ou 1,
- $R_7$ représente un atome d'hydrogène
- et $R_6$ représente :
  . un groupe amine primaire,

. un groupe amine secondaire ou tertiaire de 1 à 3 atomes de carbone,
. une pipéridine,
. une pyrrolidine,
. une diamine cyclique de 4 atomes de carbone de formule

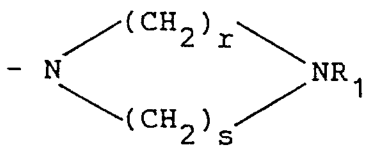

r et s étant des nombres entiers tels que le couple (r,s) représente (2,2) ou bien (r,s) représente (1,3), (3,1), ou (0,4),
dans laquelle $R_1$ représente un atome d'hydrogène ou un groupe alkyle de 1 à 3 atomes de carbone, notamment la pipérazine,
. une amine cyclique de formule

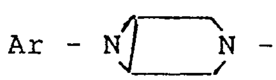

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
ou bien $R_6$ et $R_7$ sont identiques et représentent simultanément un groupe aziridine de formule

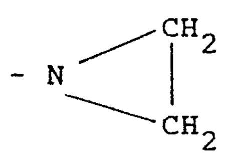 ,

ou un groupe aziridine mono, bi, tri ou tétrasubstitué par des groupes alkyle identiques ou différents, de 1 à 4 atomes de carbone, de préférence de 1 à 2 atomes de carbone,
et lorsque p est égal à 0 Z représente B, B représentant un groupe de formule
- NH - $R_4$ X
dans laquelle
- $R_4$ représente un chaîne alkyle, linéaire ou ramifiée, de 1 à 7 atomes de carbone, de préférence de 2 à 3 atomes de carbone,
- X représente
. un atome d'hydrogène,
. un groupe hydroxyl OH,
. un groupe oxycarbonyle de formule
- O - CO- $R_5$ dans laquelle $R_5$ est un groupe alkyle linéaire ou ramifié de 1 à 5 atomes de carbone, de préférence de 1 à 3 atomes de carbone,
. un groupe ester de formule - $COOR_8$ dans laquelle $R_8$ est un groupe alkyle linéaire ou ramifié de 1 à 3 atomes de carbone, ou un groupement benzyle,
. un radical de formule

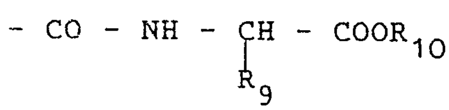

dans laquelle $R_9$ est un atome d'hydrogène ou un groupe alkyle linéaire ou ramifié, de 1 à 4 atomes de carbone, de préférence de 1 à 2 atomes de carbone, et $R_{10}$ représente un atome d'hydrogène ou un groupe alkyle identique ou non à $R_9$ comportant de préférence de 1 à 2 atomes de carbone,
. un groupe amine de formule

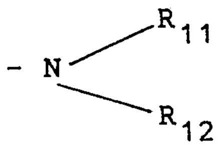

dans laquelle $R_{11}$ et $R_{12}$ sont des groupes alkyle identiques ou différents, linéaires ou ramifiés, de 1 à 3 atomes de carbone, de préférence de 1 à 2 atomes de carbone,
B représentant notamment un groupe de formule

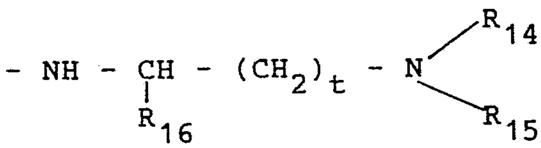

dans laquelle
t vaut de 1 à 3,
$R_{16}$ représente un atome d'hydrogène ou un groupe alkyle de 1 à 3 atomes de carbone, de préférence de 1 atome de carbone,
- $R_{14}$ et $R_{15}$ sont des groupes alkyle identiques ou différents de 1 à 3 atomes de carbone, de préférence de 1 à 2 atomes de carbone,
ou B représentant un peptide comportant de 2 à 5 acides aminés et dont la partie C-terminale se présente sous forme d'un groupe COOH ou d'un ester de formule - $COOR_{13}$ dans laquelle $R_{13}$ est un groupe alkyle de 1 à 2 atomes de carbone, ou un groupement benzyle,
et lorsque p est égal à 1, Z représente A...A, A...A représentant soit une chaîne de formule - NH - Y - NH - . Y représentant
* une chaîne alkyle linéaire de 2 à 10 atomes de carbone de préférence de 2 à 7,
* une chaîne de formule
- $(CH_2)_m$ - W - $(CH_2)_n$ -, dans laquelle m et n sont identiques ou différents et varient de 2 à 5, et W représente soit un groupe amine de formule

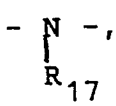, $R_{17}$

représentant un atome d'hydrogène, ou un groupe alkyle de 1 à 3 atomes de carbone, soit un groupe ammonium quaternaire de formule

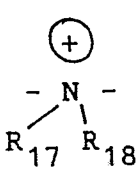

$R_{17}$ et $R_{18}$ représentant des groupes alkyle identiques ou différents de 1 à 3 atomes de carbone, lequel groupe ammonium est associé à un anion minéral ou organique physiologiquement acceptable,
* un radical choisi parmi les formules suivantes

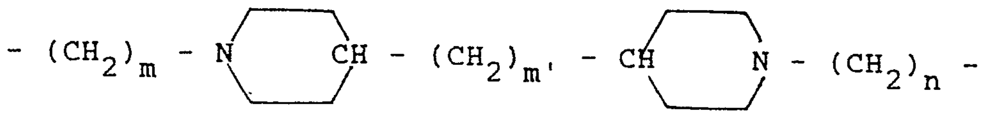

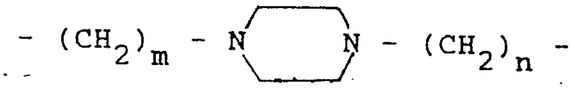

m et n identiques ou différents, variant de 2 à 5, de préférence de 2 à 3,
m′ valant de 0 à 3, et de préférence égal à 0 ou 1,

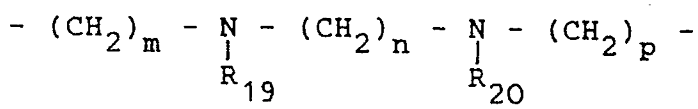

m, n, p identiques ou différents variant de 2 à 5, de préférence de 2 à 4,
$R_{19}$ et $R_{20}$ identiques ou différents représentant un atome d'hydrogène ou une chaîne alkyle de 1 à 3 atomes de carbone,
- $CH_2 - (CH_2 - O - CH_2)_q - CH_2$ -
- q variant de 1 à 5, et valant de préférence 2,
. ou A...A représentant une chaîne de formule

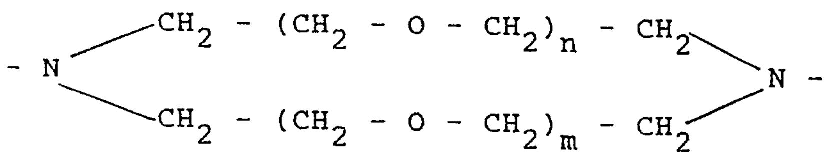

m et n, identiques ou différents, variant de 2 à 5, de préférence de 2 à 3.

Dans le cas où Y représente une chaîne de formule - $(CH_2)_m$ - W - $(CH_2)_n$ - m et n variant de 2 à 5 et W représentant un groupe ammonium quaternaire

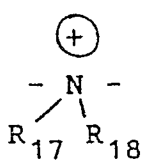

dans lequel $R_{17}$ et $R_{18}$ ont les significations indiquées ci-dessus, l'anion minéral ou organique physiologiquement acceptable est choisi notamment parmi les anions suivants : chlorhydrate, sulfate, phosphate, lactate, gluconate, isothionate, acétate, fumarate, succinate, phénoxyacétate, citrate, maléate ou méthylsulfate, etc..

Par aziridines disubstituées, on désigne aussi bien celles dont les deux atomes de carbone portent respectivement chacun un substituant, que celles dont un seul atome de carbone est disubstitué.

Parmi les composés de formule I, un groupe avantageux de composés est constitué par les monomères répondant à la formule II suivante :

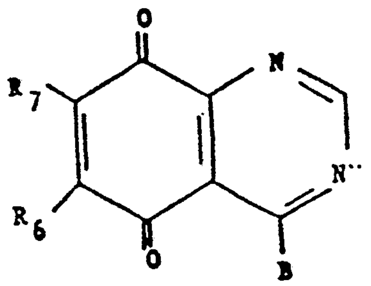

II

dans laquelle $R_6$, $R_7$ et B ont les significations indiquées ci-dessus.

Parmi les composés de formule II, un groupe avantageux de composés est constitué par ceux dans lesquels

- B représente un groupe de formule $NH-R_4-X$, dans laquelle $R_4$ représente une chaîne alkyle, linéaire ou ramifiée de 2 à 5 atomes de carbone et X représente un groupe amine de formule

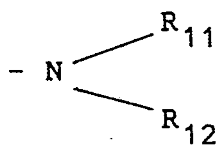

,

dans laquelle $R_{11}$ et $R_{12}$ sont des groupes alkyle identiques ou différents, linéaires ou ramifiés, de 1 à 3 atomes de carbone, de préférence de 1 à 2 atomes de carbone.

Parmi les composés de formule II, un groupe particulièrement avantageux de composés est constitué par ceux de formule II dans laquelle

- $R_6$ et $R_7$ sont identiques et représentent simultanément
  . un groupe aziridine de formule

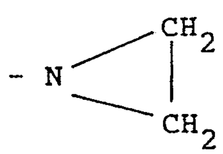

,

. ou un groupe aziridine mono, bi, tri ou tétrasubstitué par des groupes alkyle identiques ou différents, de 1 à 4 atomes de carbone, de préférence de 1 à 2 atomes de carbone.

Parmi les composés de formule II, un groupe avantageux de composés est constitué par ceux de formule II dans laquelle

- B représente un groupe de formule $NH-R_4-X$, dans laquelle $R_4$ représente une chaîne alkyle, linéaire ou ramifiée de 2 à 5 atomes de carbone et X représente un groupe amine de formule

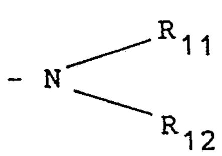

,

dans laquelle $R_{11}$ et $R_{12}$ sont des groupes alkyle identiques ou différents, linéaires ou ramifiés, de 1 à 3 atomes de carbone, de préférence de 1 à 2 atomes de carbone

et

- $R_6$ et $R_7$ sont identiques et représentent simultanément un groupe aziridine de formule

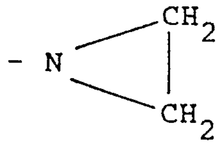

,

. ou un groupe aziridine mono, bi, tri ou tétrasubstitué par des groupes alkyle identiques ou différents, de 1 à 4 atomes de carbone, de préférence de 1 à 2 atomes de carbone.

Parmi les composés de formule II, un autre composé avantageux répond à la formule suivante :

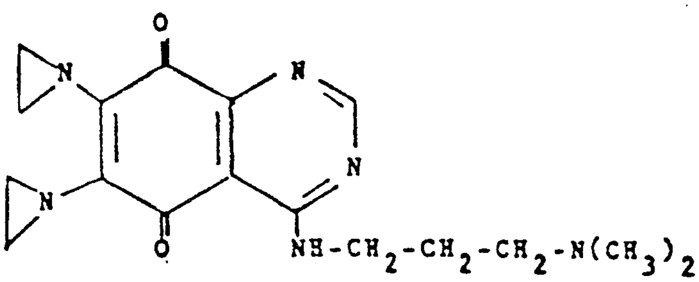

(63)

Parmi les composés de formule I, un groupe avantageux de composés est constitué par les dérivés répondant à la formule III suivante :

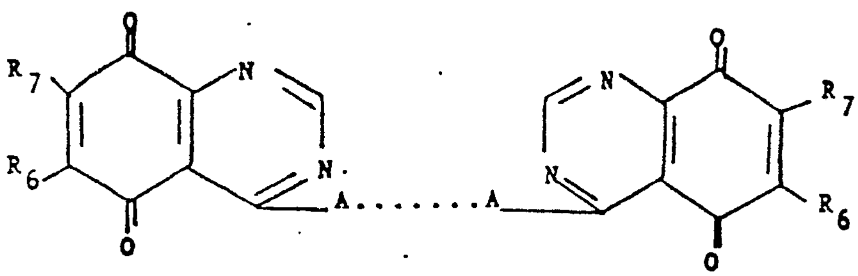

III

dans laquelle A...A, $R_6$ et $R_7$ ont les significations indiquées ci-dessus.

Parmi les composés de formule III, un groupe avantageux de composés est constitué par ceux dans lesquels

- $R_6$ et $R_7$ sont identiques et représentent simultanément un groupe aziridine de formule

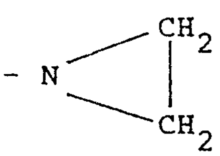

,

ou un groupe aziridine mono, bi, tri ou tétrasubstitué par des groupes alkyle, identiques ou différents, de 1 à 4 atomes de carbone, de préférence de 1 à 2 atomes de carbone.

Parmi les composés de formule III, un groupe avantageux de composés est constitué par ceux dans lesquels

- A...A représente une chaîne moléculaire de formule

- NH - Y - NH -, dans laquelle Y représente une chaîne alkyle linéaire de 2 à 10 atomes de carbone, de préférence de 2 à 7 atomes de carbone.

Parmi les composés de formule III, un groupe avantageux de composés est constitué par ceux dans lesquels

- A...A représente une chaîne moléculaire de formule

- NH - Y - NH -, dans laquelle Y représente une chaîne moléculaire de formule
- $(CH_2)_m$ - W - $(CH_2)_n$, dans laquelle m et n sont identiques ou différents et varient de 2 à 5, et W représente un groupe amine de formule

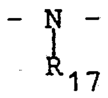

dans laquelle $R_{17}$ représente un atome d'hydrogène, ou un groupe alkyle de 1 à 3 atomes de carbone.

Parmi les composés de formule III, un groupe avantageux de composés de l'invention est constitué par ceux dans lesquels
- A...A représente une chaîne de formule -NH-Y-NH-, dans laquelle Y représente $-CH_2-(CH_2OCH_2)_qCH_2$, q variant de 1 à 5, et valant, de préférence 2.

Parmi les composés de formule III, un groupe avantageux de composés est constitué par ceux dans lesquels
- $R_6$ et $R_7$ sont identiques et représentent simultanément un group aziridine de formule

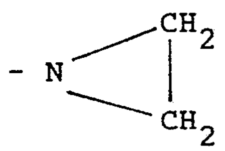 ,

. ou un groupe aziridine mono, bi, tri ou tétrasubstitué par des groupes alkyle, identiques ou différents, de 1 à 4 atomes de carbone, de préférence de 1 à 2 atomes de carbone,
et
- A...A représente une chaîne moléculaire de formule
- NH - Y - NH -, dans laquelle Y représente une chaîne alkyle linéaire de 2 à 10 atomes de carbone, de préférence de 2 à 7 atomes de carbone.

Parmi les composés de formule III, un groupe avantageux de composés est constitué par ceux dans lesquels
- $R_6$ et $R_7$ sont identiques et représentent simultanément un groupe aziridine de formule

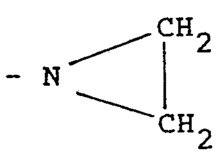 ,

. ou un groupe aziridine mono, bi, tri ou tétrasubstitué par des groupes alkyle, identiques ou différents, de 1 à 4 atomes de carbone, de préférence de 1 à 2 atomes de carbone,
et
- A...A représente une chaîne moléculaire de formule
- NH - Y - NH -, dans laquelle Y représente une chaîne moléculaire de formule
- $(CH_2)_m$ - W - $(CH_2)_n$, dans laquelle m et n sont identiques ou différents et varient de 2 à 5, et W représente un groupe amine de formule

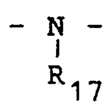

dans laquelle $R_{17}$ représente un atome d'hydrogène, ou un groupe alkyle de 1 à 3 atomes de carbone.

Parmi les composés de formule III, un groupe avantageux de composés est constitué par ceux dans lesquels
- $R_6$ et $R_7$ sont identiques et représentent simultanément un groupe aziridine de formule

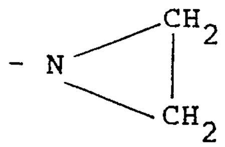 ,

. ou un groupe aziridine mono, bi, tri ou tétrasubstitué par des groupes alkyle, identiques ou différents, de 1 à 4 atomes de carbone, de préférence de 1 à 2 atomes de carbone,
et

- A...A représente une chaîne de formule -NH-Y-NH-, dans laquelle Y représente $-CH_2-(CH_2OCH_2)_q-CH_2-$, q variant de 1 à 5, et valant, de préférence 2.

Parmi les composés de formule III, un groupe avantageux de composés est constitué par ceux répondant aux formules suivantes :

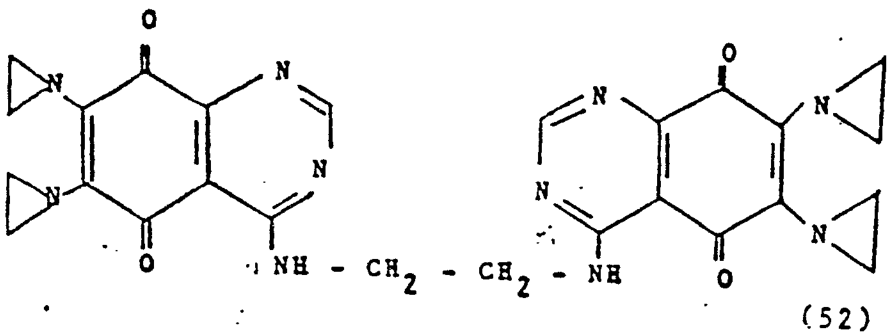

(52)

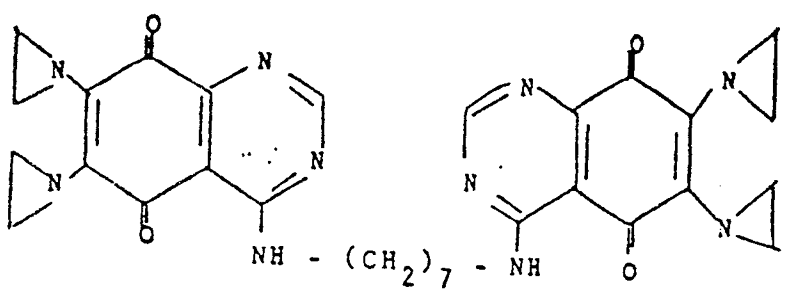

(54)

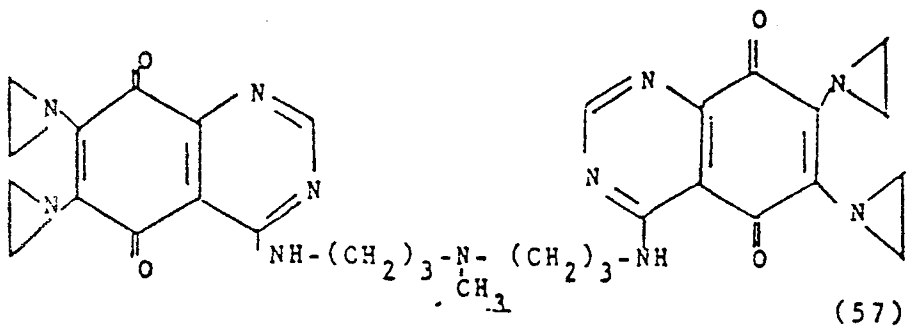

(57)

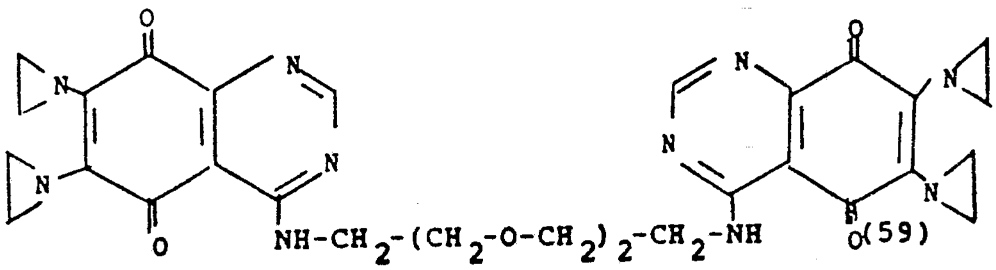

(59)

On a rassemblé dans les tableaux A et A BIS ci-après des exemples respectivement de monomères et de dimères faisant partie de l'invention et pour lesquels on donne la signification de $R_6$, $R_7$, B et A...A, ainsi que les numéros de composés correspondants.

TABLEAU A

| Type | B | $R_6$ | $R_7$ | n° du composé |
|---|---|---|---|---|
| II | $NH-CH_2-CH_2-CH_2-N(CH_3)_2$ | 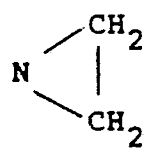 | 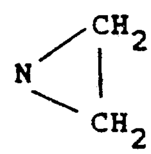 | 63 |

TABLEAU A BIS

(suite)

| Type | A....A | $R_6$ | $R_7$ | n° du composé |
|---|---|---|---|---|
| III | $NH-(CH_2)_2-NH$ | 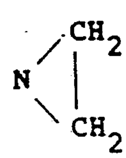 | 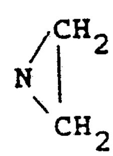 | 52 |
| " | $NH-(CH_2)_7-NH$ | " | " | 54 |
| " | $NH-(CH_2)_3-N(CH_3)-(CH_2)_3-NH$ | " | " | 57 |
| " | $NH-CH_2-(CH_2-O-CH_2)_2-CH_2-NH$ | " | " | 59 |

La préparation des composés selon l'invention est réalisée de la manière suivante :
- on oxyde à l'aide d'un oxydant, notamment $KMnO_4$ le composé de formule 1 :

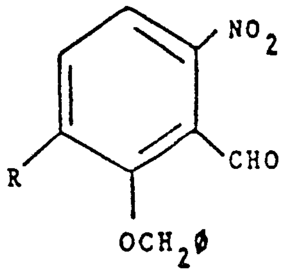

1

dans laquelle

- Ø représente un cycle benzénique,
- R représente un groupe alcoxy de 1 à 5 atomes de carbone, notamment le groupe méthoxy,

pour obtenir un composé de formule 2

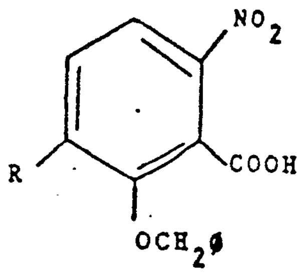

2

- on réduit le groupe $NO_2$ du composé précédent en groupe $NH_2$, notamment à l'aide de $FeSO_4$,

pour obtenir un composé de formule 3

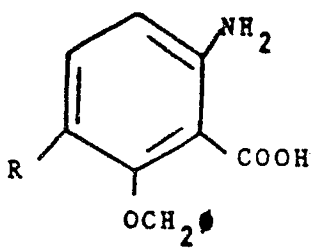

3

- on condense le composé précédent avec la triazine symétrique pour obtenir un composé de formule 4

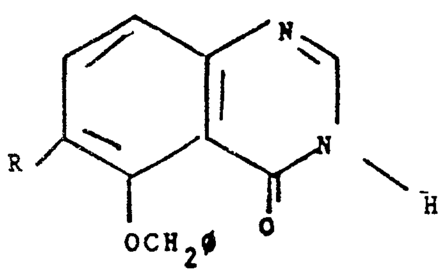

4

- on chlore le composé précédent, notamment à l'aide d'oxychlorure de phosphore, pour obtenir un composé de formule 5

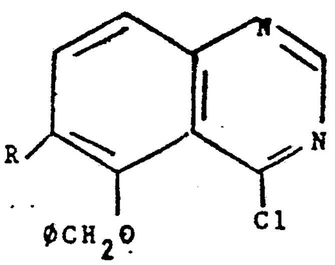

5

et dans le cas où le composé final souhaité est un monomère de formule II indiquée ci-dessus,
- on condense le composé de formule 5 sur le composé de formule BH, dans laquelle
. B représente un groupe de formule
- NH - $R_4$ X
dans laquelle
- $R_4$ représente une chaîne alkyle linéaire ou ramifiée de 1 à 7 atomes de carbone, de préférence de 2 à 3,
- X représente
. un atome d'hydrogène,
. un groupe hydroxyl OH,
. un groupe oxycarbonyle de formule
- O - CO - $R_5$ dans laquelle $R_5$ est un groupe alkyle linéaire ou ramifié de 1 à 5 atomes de carbone, de préférence de 1 à 3 atomes de carbone,
. un groupe ester de formule - $COOR_8$ dans laquelle $R_8$ est un groupe alkyle linéaire ou ramifié de 1 à 3 atomes de carbone, ou un groupe benzyle,
. un radical de formule

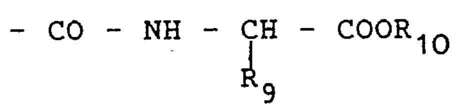

dans laquelle $R_9$ est un atome d'hydrogène ou un groupe alkyle linéaire ou ramifié, de 1 à 4 atomes de carbone, de préférence de 1 à 2, et $R_{10}$ représente un groupe alkyle identique ou non à $R_9$, de préférence de 1 à 2 atomes de carbone,
. un groupe amine de formule

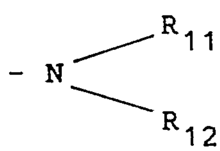

dans laquelle $R_{11}$ et $R_{12}$ sont des groupes alkyle identiques ou différents, linéaires ou ramifiés, de 1 à 3 atomes de carbone, de préférence de 1 à 2 atomes de carbone,
B représentant notamment un groupe de formule

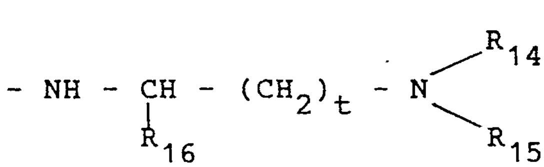

dans laquelle
t vaut de 1 à 3,
$R_{16}$ représente un atome d'hydrogène ou un groupe alkyle de 1 à 3 atomes de carbone, de préférence de 1 atome de carbone,
- $R_{14}$ et $R_{15}$ sont des groupes alkyle identiques ou différents de 1 à 3 atomes de carbone, de préférence de 1

à 2 atomes de carbone, ou B représente un peptide comportant de 2 à 5 acides aminés et dont la partie C-terminale se présente sous forme d'un groupe COOH ou d'un ester de formule - $COOR_{13}$ dans laquelle $R_{13}$ est un groupe alkyle de 1 à 2 atomes de carbone, ou un groupe benzyle, pour obtenir un composé de formule 6

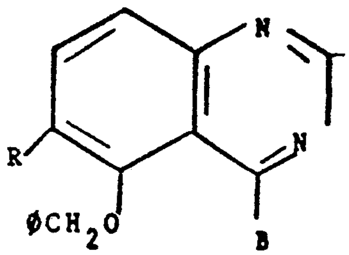

6

- on élimine le groupe benzyle du composé précédent, pour obtenir un composé de formule 7

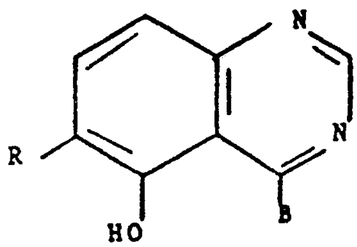

7

- on oxyde le composé précédent pour obtenir un monomère de formule IV

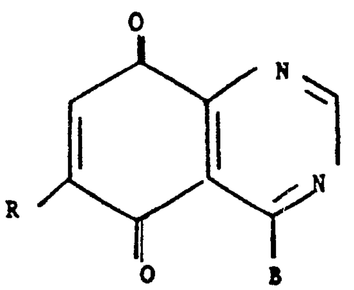

IV

- on condense le composé précédent dans lequel R représente un groupe alcoxy de 1 à 5 atomes de carbone, notamment un groupe méthoxy,

* soit sur

. de l'ammoniaque,
. une amine primaire ou une amine secondaire de 1 à 3 atomes de carbone,
. la pipéridine,
. la pyrrolidine,
. une diamine cyclique de 4 atomes de carbone de formule

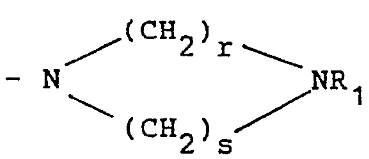

r et s étant des nombres entiers tels que le couple (r,s) représent (2,2) ou bien (r,s) représente (1,3), (3,1), (0,4) ou (4,0), dans laquelle $R_1$ représente un atome d'hydrogène ou un groupe alkyle de 1 à 3 atomes de carbone,

notamment la pipérazine,

. une amine cyclique de formule

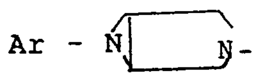

dans laquelle Ar représente un cycle benzénique non substitué ou substitué par un groupe alkyle de 1 à 2 atomes de carbone, et/ou substitué par un à deux atomes d'halogène, notamment le chlore, pour obtenir un monomère de formule II indiquée ci-dessus

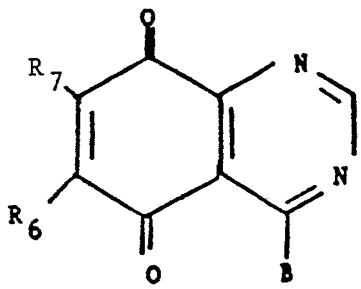

II

dans laquelle $R_7$ représente l'hydrogène

$R_6$ représente

. une amine primaire,

. un groupe amine secondaire ou tertiaire de 1 à 3 atomes de carbone,

. une pipéridine,

. une pyrrolidine,

. une diamine cyclique de 4 atomes de carbone de formule

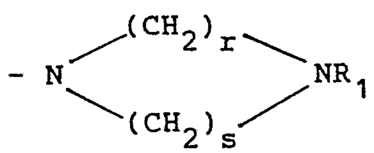

r et s étant de nombres entiers tels que le couple (r,s) représente (2,2) ou bien (r,s) représente (1,3), (3,1) ou (0,4),

dans laquelle $R_1$ représente un atome d'hydrogène ou un groupe alkyle de 1 à 3 atomes de carbone, notamment la pipérazine,

. une amine cyclique de formule

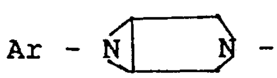

dans laquelle Ar représente un cycle benzénique non substitué ou substitué par un groupe alkyle de 1 à 2 atomes de carbone, et/ou substitué par 1 à 2 atomes d'halogène, notamment le chlore,

* soit aux moins deux équivalents d'aziridine de formule

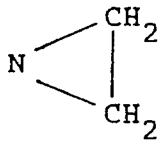

ou sur au moins deux équivalents d'aziridine mono, bi, tri ou tétrasubstituée par des groupes alkyle, identiques ou différents, de 1 à 4 atomes de carbone, pour obtenir un monomère de formule II indiquée ci-dessus, dans la formule duquel $R_6$ et $R_7$ représentent simultanément une aziridine de formule

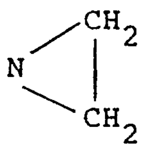

ou respectivement la même aziridine mono, bi, tri ou tétrasubstituées par des groupes alkyle de 1 à 4 atomes de carbone,

- et dans le cas où le composé final souhaité est un dimère de formule III indiquée ci-dessus, on condense le composé de formule 5 sur une molécule de formule HA..AH, dans laquelle soit A...A représente

$- NH - Y - NH -$

. Y représentant

* une chaîne alkyle linéaire de 2 à 10 atomes de carbone de préférence de 2 à 7,

* une chaîne de formule

$-(CH_2)_m - W - (CH_2)_n -$ dans laquelle m et n sont identiques ou différents et varient de 2 à 5, et W représente un groupe amine de formule

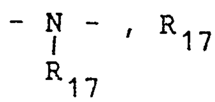

$R_{17}$ représentant un atome d'hydrogène, ou un groupe alkyle de 1 à 3 atomes de carbone,

. un radical choisi parmi les formules suivantes

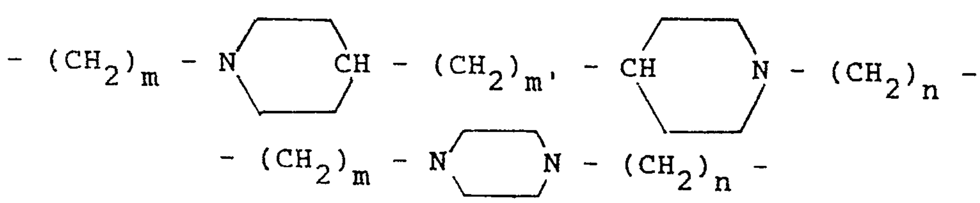

m et n identiques ou différents variant de 2 à 5, de préférence de 2 à 3,
m' valant de 0 à 3, et de préférence égal à 0 ou 1,

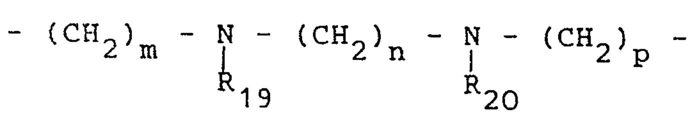

m, n, p identiques ou différents variants de 2 à 5, de préférence de 2 à 4,
$R_{19}$ et $R_{20}$ identiques ou différents représentent un atome d'hydrogène ou une chaîne alkyle de 1 à 3 atomes de carbone,

$- CH_2 - (CH_2 - O - CH_2)_q - CH_2 -$

- q variant de 1 à 5, et valant de préférence 2,

. soit A...A représente une chaîne de formule

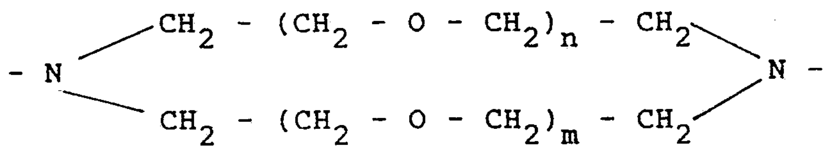

dans laquelle m et n sont identiques ou différents et varient de 2 à 5, de préférence de 2 à 3,

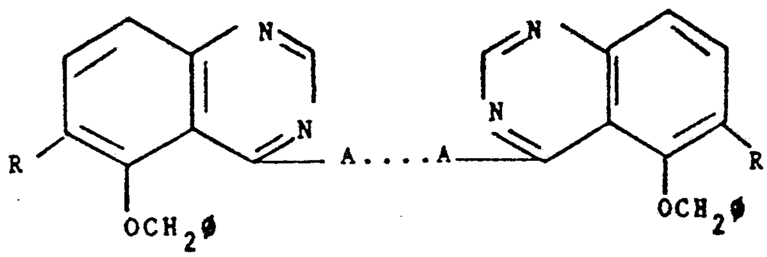

11

· 11

- puis on élimine les groupes benzyle du composé précédent pour obtenir le composé de formule 12

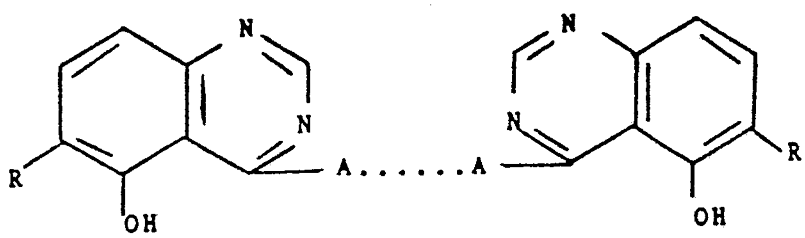

12

- on oxyde le composé précédent pour obtenir un dimère de formule V indiquée ci-dessus :

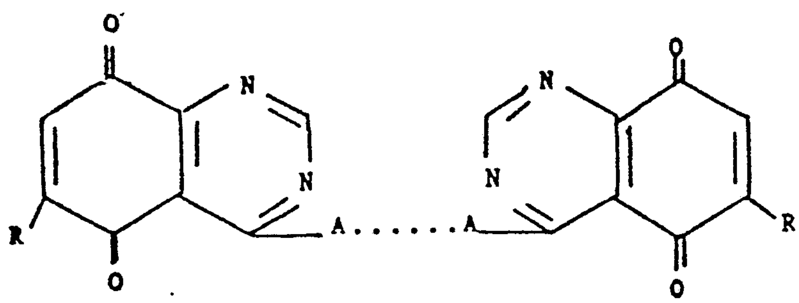

V

- on condense le composé précédent dans lequel R représente un groupe alcoxy de 1 à 5 atomes de carbone, notamment un groupe méthoxy

* soit sur
  . de l'ammoniaque,
  . une amine primaire ou secondaire de 1 à 3 atomes de carbone,
  . la pipéridine,
  . la pyrrolidine,
  . une diamine cyclique de 4 atomes de carbone de formule

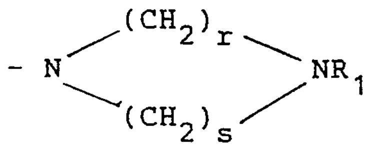

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,
. une amine cyclique de formule

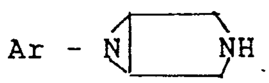

dans laquelle Ar représente un cycle benzénique non substitué ou substitué par un groupe alkyle de 1 à 2 atomes de carbone, et/ou par 1 à 2 atomes d'halogène, notamment le chlore,
pour obtenir un dimère de formule III

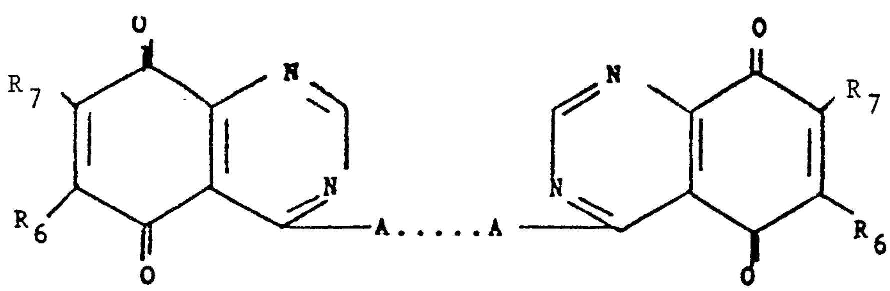

III

dans laquelle $R_7$ représente l'hydrogène
$R_6$ représente
. une amine primaire,
. un groupe amine secondaire ou tertiaire de 1 à 3 atomes de carbone,
. une pipéridine,
. une pyrrolidine,
. une diamine cyclique de 4 atomes de carbone de formule

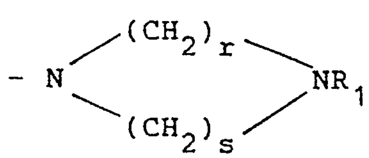

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,
. une amine cyclique de formule

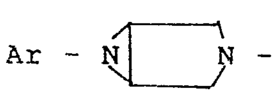

dans laquelle Ar représente un cycle benzénique non substitué ou substitué par un groupe alkyle de 1 à 2 atomes de carbone, et/ou par 1 à 2 atomes d'halogène, notamment le chlore,

. soit sur au moins quatre équivalents d'aziridine de formule

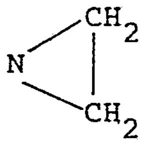

ou sur au moins quatre équivalents d'aziridine mono, bi, tri ou tétrasubstituée par des groupes alkyle, identiques ou différents, de 1 à 4 atomes de carbone,

pour obtenir un dimère de formule III, indiquée ci-dessus, dans lequel $R_6$ et $R_7$ représentent simultanément une aziridine de formule

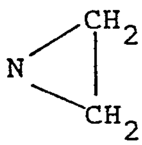

ou respectivement la même aziridine mono, bi, tri ou tétrasubstituées par des groupes alkyle de 1 à 4 atomes de carbone.

Pour préparer les composés de l'invention dans lesquels Y représente une chaîne $-NH(CH_2)_m-W-(CH_2)_n-NH-$ et W représente

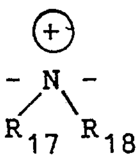

on prépare, dans un premier temps, les composés dans lesquels W représente

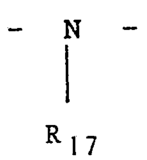 (ou 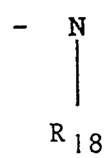) et on

et on quaternarise l'azote tertiaire indiqué ci-dessus à l'aide d'un dérivé $R_{17}X$ ou $R_{18}X$, $X^-$ représentant l'anion d'un halogène, notamment iodure ou bromure et $R_{17}$ (ou $R_{18}$) représentant un groupe alkyle identique ou différent de $R_{18}$ (respectivement $R_{17}$) de 1 à 3 atomes de carbone.

L'anion $X^-$ peut être transformé en un autre anion minéral ou organique, de préférence physiologiquement acceptable, par double décomposition avec le sel d'argent correspondant.

La préparation des composés de formule II selon l'invention est réalisée de la manière suivante :

- on oxyde à l'aide d'un oxydant, notamment $KMnO_4$ le composé de formule 1

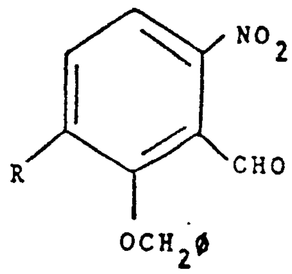

1

dans laquelle

- Ø représente un cycle benzénique,
- R représente un groupe alcoxy de 1 à 5 atomes de carbone,

pour obtenir un composé de formule 2

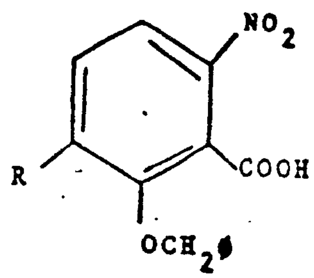

2

- on réduit le groupe $NO_2$ du composé précédent en groupe $NH_2$, notamment à l'aide de $FeSO_4$, pour obtenir un composé de formule 3

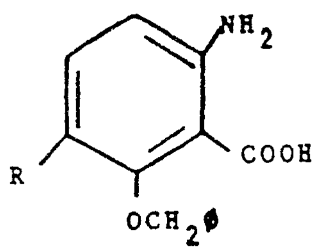

3

- on condense le composé précédent avec la triazine symétrique pour obtenir un composé de formule 4

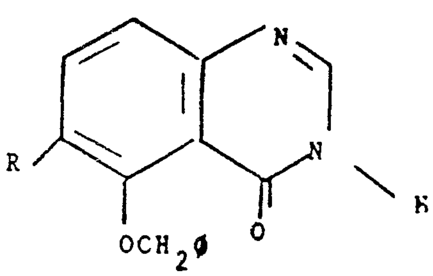

4

- on chlore le composé précédent, notamment à l'aide d'oxychlorure de phosphore, pour obtenir un composé de formule 5

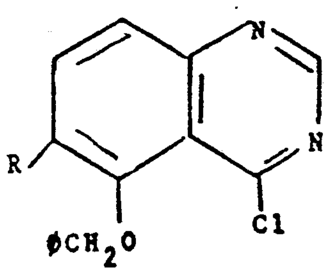

5

- on condense le composé de formule 5 sur le composé de formule BH, dans laquelle
. B représente un groupe de formule
- $NH - R_4 X$
dans laquelle
- $R_4$ représente une chaîne alkyle linéaire ou ramifiée de 1 à 7 atomes de carbone, de préférence de 2 à 3,
- X représente
. un atome d'hydrogène,
. un groupe hydroxyle OH,
. un groupe oxycarbonyle de formule
- $O - CO - R_5$ dans laquelle $R_5$ est un groupe alkyle linéaire ou ramifié de 1 à 5 atomes de carbone, de préférence de 1 à 3 atomes de carbone,
. un groupe ester de formule $-COOR_8$ dans laquelle $R_8$ est un groupe alkyle linéaire ou ramifié de 1 à 3 atomes de carbone, ou un groupe benzyle,
. un radical de formule

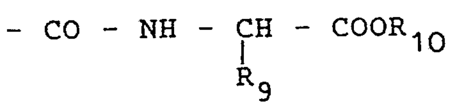

dans laquelle $R_9$ est un atome d'hydrogène ou un groupe alkyle linéaire ou ramifié, de 1 à 4 atomes de carbone, de préférence de 1 à 2, et $R_{10}$ représente un groupe alkyle identique ou non à $R_9$ comportant de préférence de 1 à 2 atomes de carbone,
. un groupe amine de formule

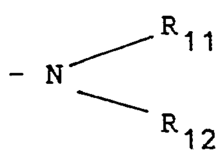

dans laquelle $R_{11}$ et $R_{12}$ sont des groupes alkyle identiques ou différents, linéaires ou ramifiés, de 1 à 3 atomes de carbone, de préférence de 1 à 2 atomes de carbone,
B représentant notamment un groupe de formule

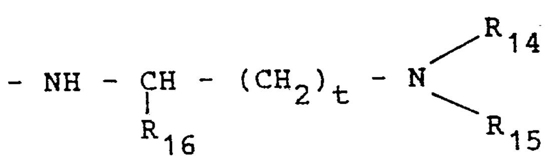

dans laquelle
t vaut de 1 à 3,
$R_{16}$ représente un atome d'hydrogène ou un groupe alkyle de 1 à 3 atomes de carbone, de préférence de 1 atome de carbone,
- $R_{14}$ et $R_{15}$ sont des groupes alkyle identiques ou différents de 1 à 3 atomes de carbone, de préférence de 1 à 2 atomes de carbone,

ou B représente un peptide comportant de 2 à 5 acides aminés et dont la partie C-terminale se présente sous forme d'un groupe COOH ou d'un ester de formule - $COOR_{13}$ dans laquelle $R_{13}$ est un groupe alkyle de 1 à 2 atomes de carbone, ou un groupe benzyle, pour obtenir un composé de formule 6

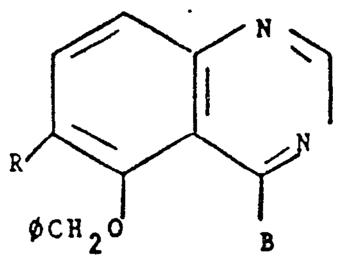

6

- on élimine le groupe benzyle du composé précédent, pour obtenir un composé de formule 7

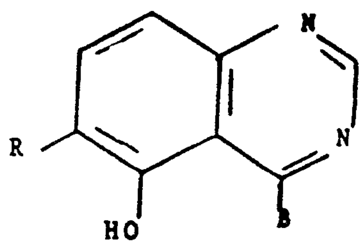

7

- on oxyde le composé précédent pour obtenir un monomère de formule IV indiquée ci-dessus

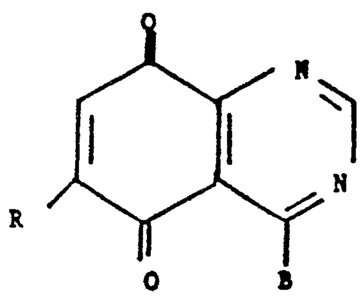

IV

- on condense le composé précédent dans lequel R représente un groupe alcoxy de 1 à 5 atomes de carbone, notamment un groupe méthoxy,

*soit sur

. de l'ammoniaque,

. une amine primaire ou secondaire de 1 à 3 atomes de carbone,

. sur la pipéridine,

. la pyrrolidine,

. une diamine cyclique de 4 atomes de carbone de formule

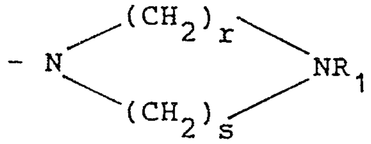

r et s étant des nombres entiers tels que le couple (r,s) représente (2,2) ou bien (r,s) représente (1,3), (3,1) ou (0,4),

dans laquelle $R_1$ représente un atome d'hydrogène ou un groupe alkyle de 1 à 3 atomes de carbone, notamment la pipérazine,

. une amine cyclique de formule

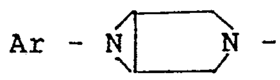

dans laquelle Ar représente un cycle benzénique non substitué ou substitué par un groupe alkyle de 1 à 2 atomes de carbone, avantageusement par un méthyle, et/ou par 1 ou 2 atomes d'halogène, notamment le chlore, pour obtenir un monomère de formule II

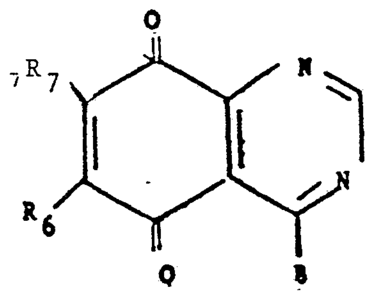

II

dans laquelle $R_7$ représente l'hydrogène
$R_6$ représente
. une amine primaire,
. une groupe amine secondaire ou tertiaire de 1 à 3 atomes de carbone,
. une pipéridine,
. une pyrrolidine,
. une diamine cyclique de 4 atomes de carbone de formule

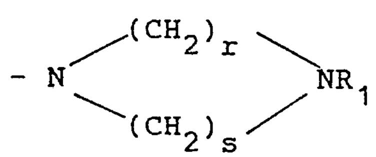

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,
. une amine cyclique de formule

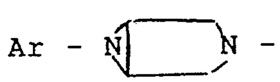

dans laquelle Ar représente un cycle benzénique non substitué ou substitué par un groupe alkyle de 1 à 2 atomes de carbone, avantageusement par un méthyle, et/ou par 1 à 2 atomes d'halogène, notamment le chlore,
* soit sur au moins deux équivalents d'aziridine de formule

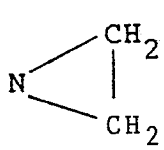

ou sur au moins deux équivalents d'une aziridine mono, bi, tri ou tétrasubstituée par des groupes alkyle de 1

à 4 atomes de carbone,
pour obtenir un monomère de formule II indiquée ci-dessus dans la formule duquel $R_6$ et $R_7$ représentent simultanément une aziridine de formule

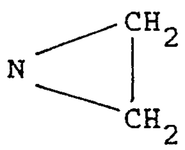

ou une aziridine mono, bi, tri ou tétrasubstituée par des groupes alkyle de 1 à 3 atomes de carbone.

La préparation des composés de formule III selon l'invention est réalisée de la manière suivante :
- on oxyde à l'aide d'un oxydant, notamment $KmnO_4$ le composé de formule 1

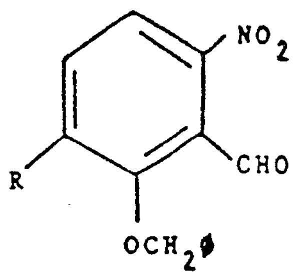

1

dans laquelle
- Ø représente un cycle benzénique,
- R représente un groupe alcoxy de 1 à 5 atomes de carbone,

pour obtenir un composé de formule 2

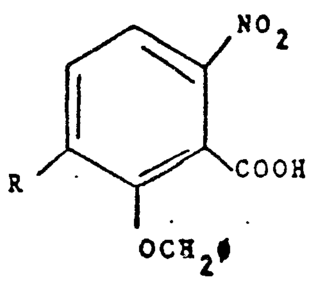

2

- on réduit le groupe $NO_2$ du composé précédent en groupe $NH_2$, notamment à l'aide de $FeSO_4$, pour obtenir un composé de formule 3

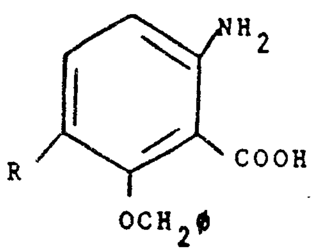

3

- on condense le composé précédent avec la triazine symétrique pour obtenir un composé de formule 4

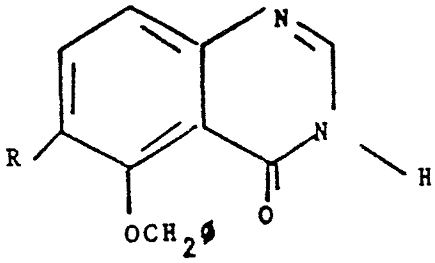

4

- on chlore le composé précédent, notamment à l'aide d'oxychlorure de phosphore, pour obtenir un composé de formule 5

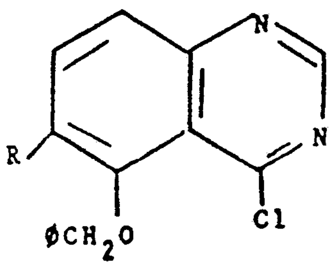

5

- on condense deux molécules de composé de formule 5 sur le composé de formule HA...AH, dans laquelle soit A...A représente

- NH - Y - NH -

. Y représentant

* une chaîne alkyle linéaire de 2 à 10 atomes de carbone de préférence de 2 à 7,

* une chaîne de formule

- $(CH_2)_m$ - W - $(CH_2)_n$ - dans laquelle m et n sont identiques ou différents et variant de 2 à 5, et W représente soit un groupe amine de formule

$$-\underset{R_{17}}{N}- \;, \; R_{17}$$

$R_{17}$ représentant un atome d'hydrogène, ou un groupe alkyle de 1 à 3 atomes de carbone,

* un radical choisi parmi les formules suivantes

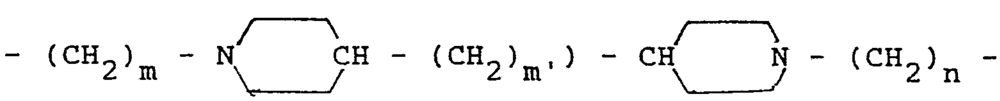

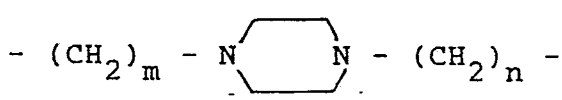

dans lesquelles m et n identiques ou différents varient de 2 à 5, de préférence de 2 à 3, m' valant de 0 à 3, et de préférence égal à 0 ou 1,

$$-(CH_2)_m-\underset{R_{19}}{\overset{|}{N}}-(CH_2)_n-\underset{R_{20}}{\overset{|}{N}}-(CH_2)_p-$$

m, n, p identiques ou différents variant de 2 à 5, de préférence 2 à 4,

$R_{19}$ et $R_{20}$ identiques ou différents représentent un atome d'hydrogène ou une chaîne alkyle de 1 à 3 atomes

de carbone,

- $CH_2 - (CH_2 - O - CH_2)_q - CH_2$ -

- q variant de 1 à 5, valant de préférence 2,

. soit A...A représente une chaîne de formule

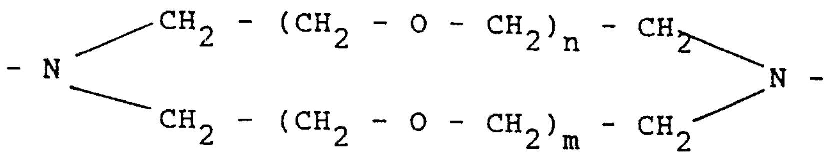

dans laquelle m et n sont identiques ou différentes et varient de 2 à 5, et valant de préférence de 2 à 3, pour obtenir le composé de formule 11

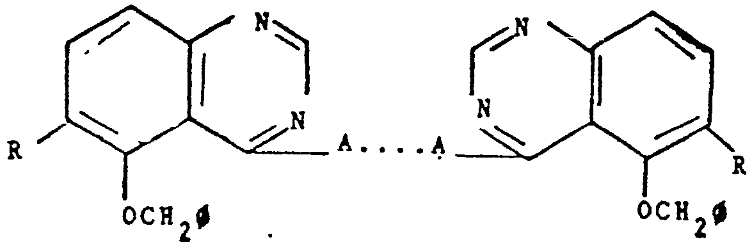

11

- puis on élimine les groupes benzyle du composé précédent pour obtenir le composé de formule 12

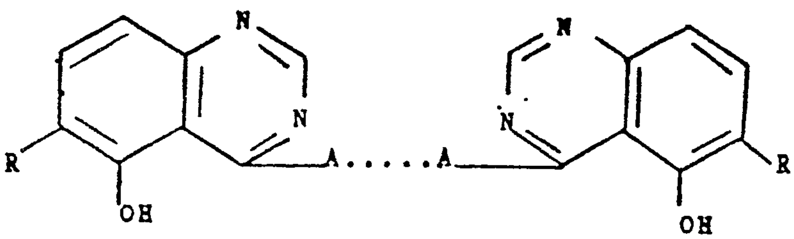

12

- on oxyde le composé précédent pour obtenir un dimère de formule V :

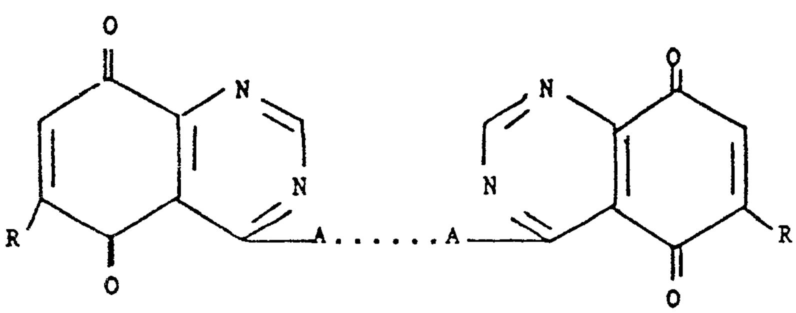

V

- on condense le composé précédent dans lequel R représente un groupe alcoxy de 1 à 5 atomes de carbone, notamment un groupe méthoxy

* soit sur

. de l'ammoniaque,

. une amine primaire ou secondaire de 1 à 3 atomes de carbone,

. la pipéridine,

. la pyrrolidine,

. une diamine cyclique de 4 atomes de carbone

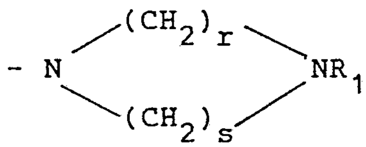

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,

. une amine cyclique de formule

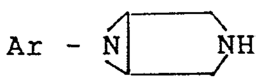

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
pour obtenir un dimère de formule III indiquée ci-dessus

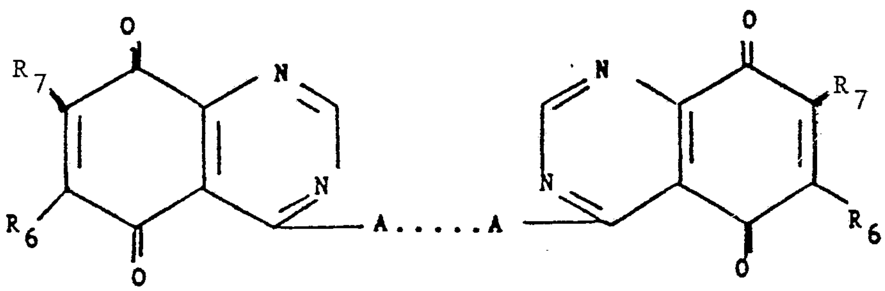

III

dans laquelle $R_7$ représente l'hydrogène
$R_6$ représente

. une amine primaire,
. un groupe amine secondaire ou tertiaire de 1 à 3 atomes de carbone,
. une pipéridine,
. une pyrrolidine,
. une diamine cyclique de 4 atomes de carbone de formule

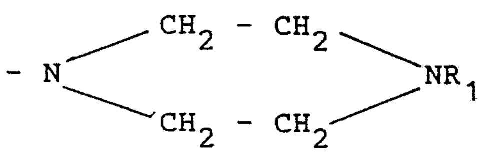

dans laquelle $R_1$ représente un atome d'hydrogène ou un groupe alkyle de 1 à 3 atomes de carbone, notamment la pipérazine,

. une amine cyclique de formule

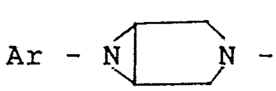

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,

* soit sur au moins quatre équivalents d'aziridine de formule

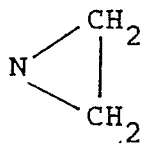

ou sur au moins quatre équivalents d'aziridine mono, bi, tri ou tétrasubstituée par des groupes alkyle de 1 à 4 atomes de carbone,

pour obtenir un dimère de formule III, dans lequel $R_6$ et $R_7$ représentent simultanément une aziridine de formule

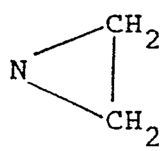

ou respectivement la même aziridine mono, bi, tri ou tétrasubstituées par des ou groupes alkyle de 1 à 4 atomes de carbone.

Pour illustrer l'invention, sans toutefois être limitatif, on a représenté sur les figures I et II, un schéma de synthèse respectivement des monomères et dimères de l'invention, dans lequel figurent les formules des composés intermédiaires, ainsi que leur numéro.

Par commodité, on désignera par composé "a" (a prenant les valeurs 1 à 7, et 11 et 12) le composé répondant à l'une des formules indiquées ci-dessus, et également représentées sur les figures I et/ ou II. Les composés 9, 10 et 14 ayant leur formule uniquement représentée sur les figures I et/ou II, car ce sont des composés de l'invention qui tombent dans la définition générale des composés de l'invention de formule I.

Les composés IV et V représentés respectivement sur les figures I et II sont des intermédiaires nouveaux qui font également partie de l'invention.

Le produit de départ entrant dans la préparation de tous les composés selon l'invention est l'aldéhyde alcoxy-3 benzyloxy-2 nitro-6 benzoïque (composé 1) de formule 1,

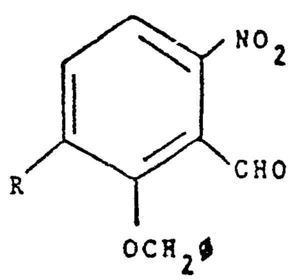

dans laquelle R représente un groupe alcoxy de 1 à 5 atomes de carbone et Ø représente un cycle benzénique.

Le composé 1, dans lequel R représente $OCH_3$, ainsi que son mode d'obtention, est décrit par Julia (cf. M. JULIA, P. MANOURY et C. VOILLAUME, Bull. Soc. Chim., 1965, 1417).

Le même procédé de synthèse s'applique lorsque R est un groupe alcoxy de 2 à 5 atomes de carbone.

Le produit de départ 1 sus-mentionné sera de préférence tel que R représente un groupe méthoxy, c'est-à-dire l'aldéhyde benzyloxy-2 méthoxy-3 nitro-6 benzoïque.

L'oxydation du produit de départ 1 en composé 2 de formule indiquée ci-dessus, (acide alcoxy-3 benzyloxy-2 nitro-6 benzoïque) notamment en acide benzyloxy-2 méthoxy-3 nitro-6 benzoïque se fait avantageusement à l'aide de $KMnO_4$.

La réduction du groupe $NO_2$ du composé 2, de formule indiquée ci-dessus, en groupe $NH_2$, pour obtenir le composé 3, de formule indiquée ci-dessus, (acide amino-6 benzyloxy-2 alcoxy-3 benzoïque) notamment

l'acide amino-6 benzyloxy-2 méthoxy-3 benzoïque est préférablement réalisée à l'aide de $FeSO_4$, en milieu alcalin.

La condensation du composé 3 précédent avec la triazine symétrique pour conduire au composé 4 de formule indiquée ci-dessus, (alcoxy-6 benzyloxy-5 dihydro-3,4 quinazolinone-4) notamment au benzyloxy-5 méthoxy-6 dihydro-3,4 quinazolinone-4 est réalisée selon la méthode décrite par Kreutzberger (A. KREUTZBERGER et M.U. UZBEK, Arch. Pharm., 1972, 305, 171).

La chloruration du composé 4 précédent est effectuée à l'aide d'un procédé connu, de préférence réalisée par l'oxychlorure de phospore en présence de triéthylamine pour conduire au composé 5 de formule indiquée ci-dessus, (alcoxy-6 benzyloxy-5 chloro-4 quinazoline) notamment au benzyloxy-5 chloro-4 méthoxy-6 quinazoline.

La chloruration peut également être effectuée par le chlorure de thionyle, ou par le pentachlorure de phosphore on présence ou non d'oxychlorure de phosphore.

La condensation du composé 5 sur BH, conduisant au composé 6 de formule indiquée ci-dessus ou la dimérisation du composé de formule 6, conduisant au composé 11, par l'intermédiaire de HA...AH se fait selon des procédés classiques.

La condensation du composé 5 sur BH, se fait avantageusement avec un excès de BH. On peut opérer en excès de triéthylamine ou d'une autre amine tertiaire aliphatique ou hétérocyclique, ou de toute autre amine ne réagissant pas avec le composé 5 telle le diamino-1,8 naphtalène.

La dimérisation du composé 5 par l'intermédiaire du composé HA....AH se fait avantageusement dans un rapport stoechiométrique de 2 moles de composé 5, pour une mole de composé HA....AH.

On peut opérer en excès de triéthylamine ou d'une autre amine tertiaire aliphatique ou hétérocyclique ou de de toute autre amine ne réagissant pas avec le composé 5, telle le diamino-1,8 naphtalène.

L'élimination des fonctions benzyles constituant les groupes oxybenzyles des composés 6 et 11 indiqués ci-dessus du type alcoxy-6 benzyloxy-5 quinazoline substitués en position 4, ou dimère de alcoxy-6 benzyloxy-5 quinazoline, notamment benzyloxy-5 méthoxy-6 quinazoline substitués en position 4 ou notamment dimère de benzyloxy-5 méthoxy-6 quinazoline est réalisée soit par action de l'acide trifluoroacétique, soit par hydrogénation catalytique en présence de charbon palladié, pour obtenir respectivement les composés de formule 7 et 11, indiquées ci-dessus, respectivement du type alcoxy-6 hydroxy-5 quinazoline substitué en position 4, (notamment hydroxy-5 méthoxy-6 quinazoline substitué en position 4) et du type dimère alcoxy-6 d'hydroxy-5 quinazoline, (notamment dimère d'hydroxy-5 méthoxy-6 quinazoline.)

Lorsque les composés 6 et 11 possèdent des fonctions labiles, telles qu'ester ou amide, on aura préférentiellement recours à la débenzylation par hydrogénation catalytique.

C'est notamment le cas des intermédiaires entrant dans la préparation des composés de l'invention référencés ci-après par les numéros suivants et dont les formules sont indiquées ci-après : 65, 67, 71 et 73.

En ce qui concerne l'hydrogénation catalytique, elle constitue aussi une méthode avantageuse, lorsque les composés 6 et 11 sont solubles dans les solvants habituellement utilisés pour l'hydrogénation catalytique.

C'est le cas des composés de l'invention dont les formules sont indiquées ci-après et référencées par 74, 62, 75, 76, 77 et 78.

On a recours à la débenzylation à l'aide d'acide trifluoroacétique, lorsqu'il est difficile ou impossible de désorber le composé obtenu du catalyseur. C'est notamment le cas du composé 68 de l'invention, dont la formule est indiquée ci-après.

L'oxydation des composés 7 et 12 est avantageusement réalisée à l'aide du réactif de Frémy (nitrosodisulfonate de potassium) (réf. H. ZIMMER, D.C. LANKIN et S.W. HORGAN, Chem. Rev., 1971, 71, 229) pour donner respectivement les composés de formule IV et V représentés sur les planches comportant les figures I et II ci-après.

La substitution de monomères ou de dimères respectivement de formule IV ou V, possédant un groupe alcoxy en position 6, par une aziridine, substituée ou non, conduit nécessairement à des monomères ou des dimères de formula II (composé 10 de la figure I) ou III (composé 14 de la figure II) dans lesquels la position 6 et la position 7 sont toutes les deux substituées par la susdite molécule d'aziridine, lorsque cette dernière est utilisée sans solvant.

La synthèse des aziridines sus-mentionnées est décrite dans l'article "aziridines" par J.A. Deyrup publié dans Small Ring Heterocycle Part I, édité par A. Haffner, J. Willey, 1983, New York.

On substitue avantageusement le composé de formule IV par deux molécules d'aziridine et le composé de formule V par quatre molécules d'aziridine.

La substitution de composés de formula IV ou V possédant un groupe alcoxy en position 6, par des molécules aminées, autres que les molécules d'aziridine sus-mentionnées, conduit à des composés de formule II (composé 9 de la figure I) ou III, uniquement substitués en position 6 par ces molécules aminées sus-mentionnées.

On substitue avantageusement un composé de formula IV par une molécule aminée définie ci-dessus et un composé de formule V par deux molécules aminées.

L'invention vise également en tant que tels les composés monomères intermédiaires qui entrent dans la préparation des composés de formule I, et qui sont nouveaux.

Ces composés répondent aux formules suivantes :

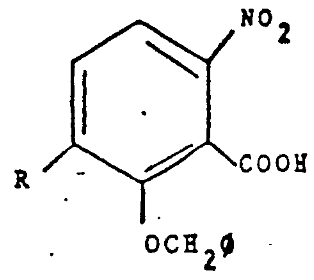

2

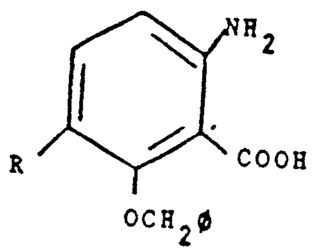

3

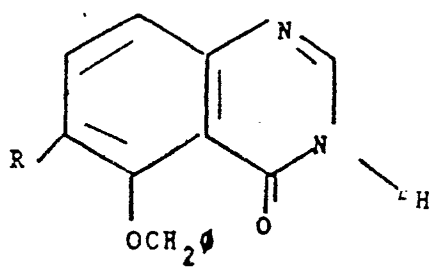

4

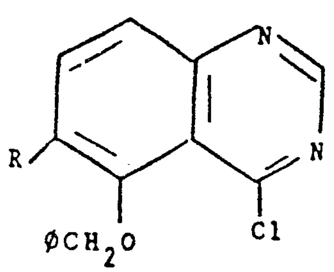

5

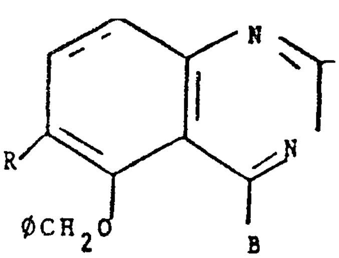

6

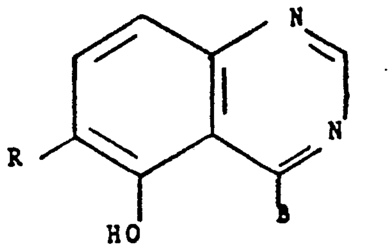

7

dans lesquels
. R représente un groupe alcoxy de 1 à 5 atomes de carbone,
. O représente un groupe benzénique et B a la signification indiquée ci-dessus.

L'invention vise également en tant que tels les composés dimères intermédiaires qui entrent dans la préparation des composés de formule I, et qui sont nouveaux.

Ces composés répondent aux formules suivantes :

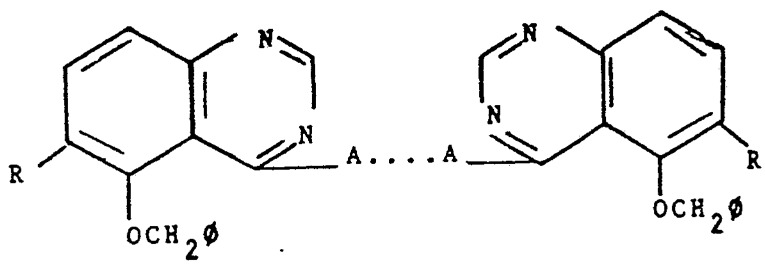

11

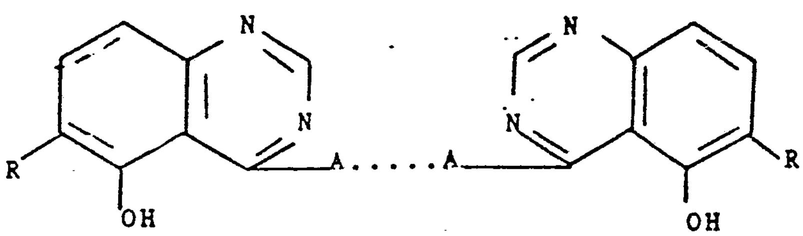

12

dans lesquels
. R représente un groupe alcoxy de 1 à 5 atomes de carbone,
. Ø représente un groupe benzénique et
. A...A a la signification indiquée ci-dessus.

Dans la mise en oeuvre du procédé d'obtention des composés de l'invention de formule II, sont nouveaux les composés de formule IV :

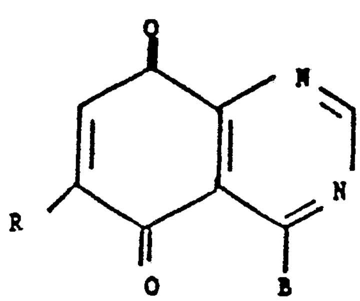

IV

dans laquelle
- R représente un groupe alcoxy de 1 à 5 atomes de carbone, de préférence de 1 à 2 atomes de carbone,

- B représente un groupe de formule
- NH - $R_4$ X

dans lequel

- $R_4$ représente une chaine alkyle, linéaire ou ramifiée, de 1 à 7 atomes de carbone, de préférence de 2 à 3 atomes de carbone,
- X représente
  . un atome d'hydrogène,
  . un groupe hydroxyl OH,
  . un groupe oxycarbonyle de formule
  - O -CO- $R_5$ dans laquelle $R_5$ est un groupe alkyle linéaire ou ramifié de 1 à 5 atomes de carbone, de préférence de 1 à 3 atomes de carbone,
  . un groupe ester de formule - $COOR_8$ dans laquelle $R_8$ est un groupe alkyle linéaire ou ramifié de 1 à 3 atomes de carbone, ou un groupement benzyle,
  . un radical de formule

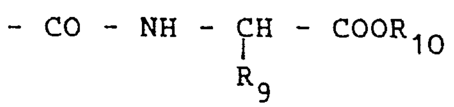

dans laquelle $R_9$ est un atome d'hydrogène ou un groupe alkyle linéaire ou ramifié, de 1 à 4 atomes de carbone, de préférence de 1 à 2 atomes de carbone, et $R_{10}$ représente un groupe alkyle identique ou non à $R_9$ comportant de préférence de 1 à 2 atomes de carbone,

. un groupe amine de formule

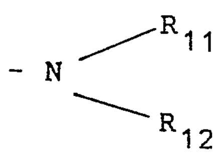

dans laquelle $R_{11}$ et $R_{12}$ sont des groupes alkyle identiques ou différents, linéaires ou ramifiés, de 1 à 3 atomes de carbone, de préférence de 1 à 2 atomes de carbone,

B représentant notamment un groupe de formule

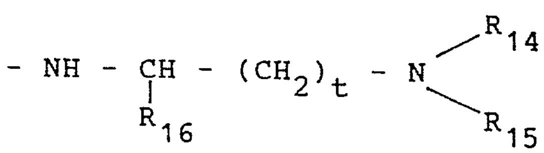

dans laquelle

t vaut de 1 à 3,

$R_{16}$ représente un atome d'hydrogène ou un groupe alkyle de 1 à 3 atomes de carbone, de préférence de 1 atome de carbone,

- $R_{14}$ et $R_{15}$ sont des groupes alkyle identiques ou différents de 1 à 3 atomes de carbone, de préférence de 1 à 2 atomes de carbone,

ou B représentant un peptide comportant de 2 à 5 acides amines et dont la partie C-terminale se présente sous forme d'un groupement COOH ou d'un ester de formule

- $COOR_{13}$ dans laquelle $R_{13}$ est un groupe alkyle de 1 à 2 atomes de carbone ou un groupement benzyle.

Parmi les composés de formule IV, un groupe de composés préférés est constitué par ceux dans lesquels R représente un groupe méthoxy ou éthoxy.

Parmi les composés de formule IV, un groupe de composés préférés est constitué par ceux dans lesquels :

- B représente un groupe de formule NH-$R_4$-X, dans laquelle $R_4$ représente une chaîne alkyle, linéaire ou ramifiée de 2 à 5 atomes de carbone et X représente un groupe amine de formule

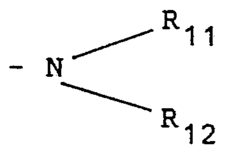 ,

dans laquelle $R_{11}$ et $R_{12}$ sont des groupes alkyle identiques ou différents, linéaires ou ramifiés, de 1 à 3 atomes de carbone, de préférence de 1 à 2 atomes de carbone.

Des composés particulièrement avantageux répondent aux formules suivantes :

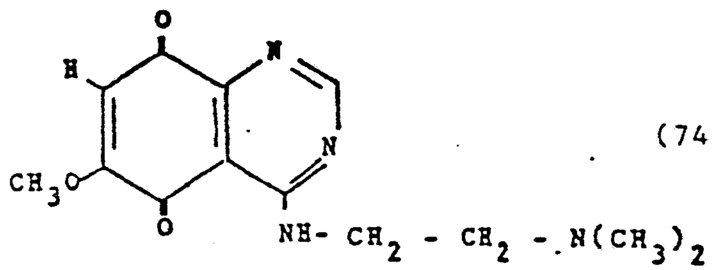

(74)

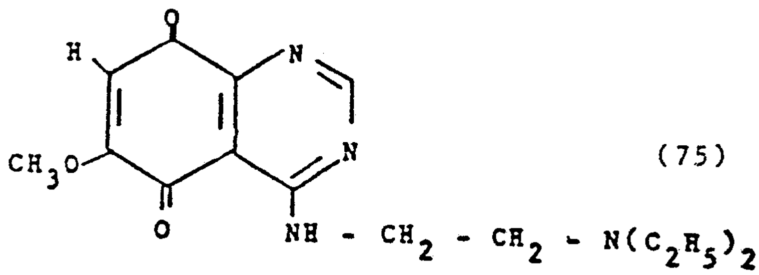

(75)

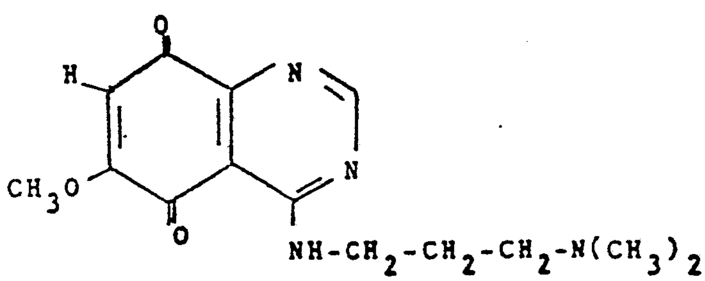

(62)

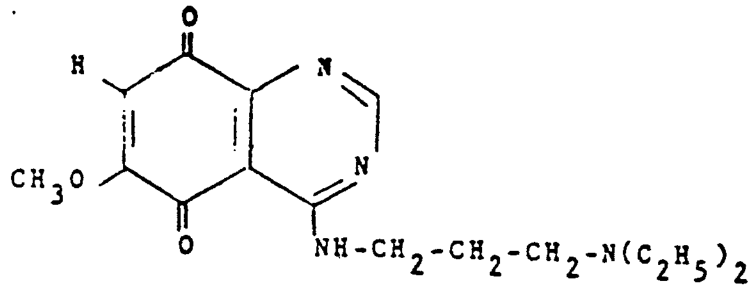

(76)

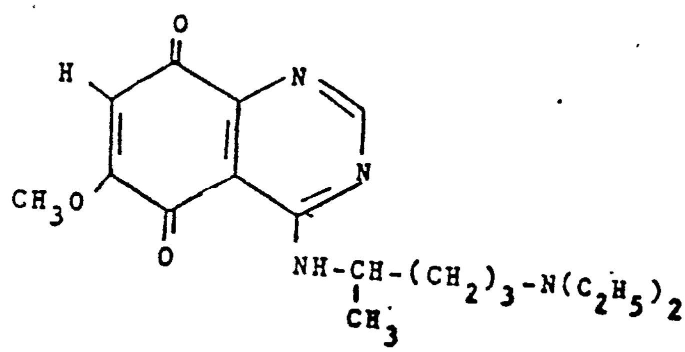

(77)

Les composés de formule IV sont des intermédiaires clé du procédé et représentent un autre aspect de la présente invention.

Les composés de formule IV peuvent également être utilisés pour la préparation des dimères de formule III.

Dans la mise en oeuvre du procédé d'obtention des composés de l'invention de formule III sont nouveaux les composés de formule V :

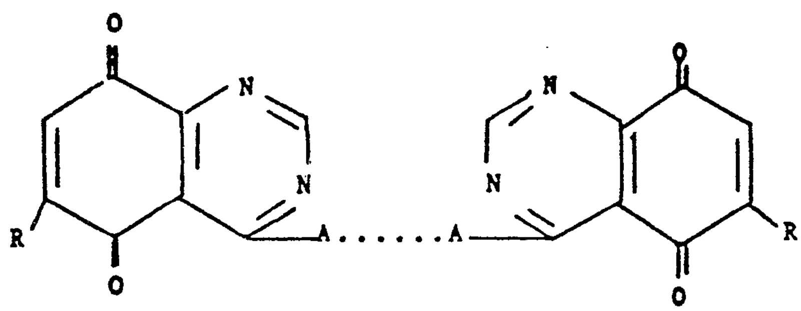

V

dans laquelle :

- R représente un groupe alcoxy de 1 à 5 atomes de carbone, notamment 1 à 2 atomes de carbone et

-A...A représentant soit une chaîne de formule -NH-Y-NH-

. Y représentant

* une chaîne alkyle linéaire de 2 à 10 atomes de carbone de préférence de 2 à 7,

* une chaîne de formule

- $(CH_2)_m$ - W - $(CH_2)_n$ -, dans laquelle m et n sont identiques ou différents et varient de 2 à 5, et W représente soit un groupe amine de formule

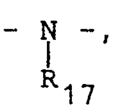 $R_{17}$

représentant un atome d'hydrogène, ou un groupe alkyle de 1 à 3 atomes de carbone, soit un groupe ammonium quaternaire de formule

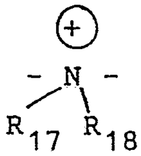

$R_{17}$ et $R_{18}$ représentant des groupes alkyle identiques ou différents de 1 à 3 atomes de carbone, lequel groupe ammonium est associé à un anion minéral ou organique physiologiquement acceptable,
* un radical choisi parmi les formules suivantes

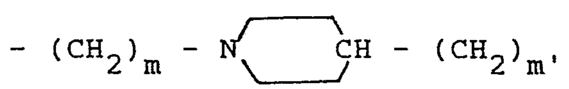
,
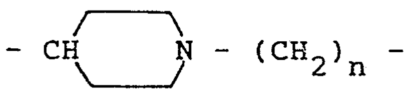

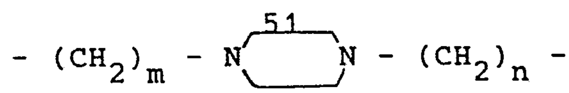

m et n identiques ou différents, variant de 2 à 5, de préférence de 2 à 3,
m' valant de 0 à 3, et de préférence égal à 0 ou 1,

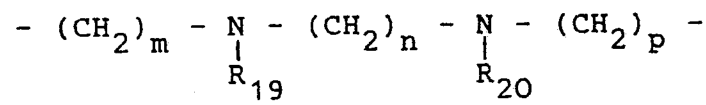

m, n, p identiques ou différents variant de 2 à 5, de préférence de 2 à 4,
$R_{19}$ et $R_{20}$ identiques ou différents représentant un atome d'hydrogène ou une chaîne alkyle de 1 à 3 atomes de carbone,
- $CH_2$ - $(CH_2$ - O - $CH_2)_q$ - $CH_2$ -
- q variant de 1 à 5, et valant de préférence 2,
. ou A...A représentant une chaîne de formule

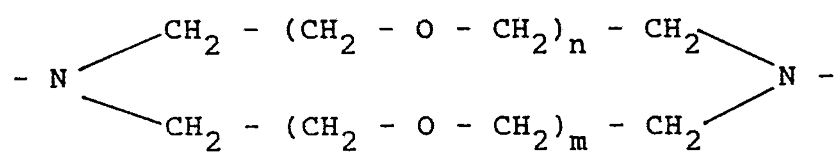

m et n, identiques ou différents, variant de 2 à 5, de préférence de 2 à 3.

Parmi les composés de formule V, un groupe préféré est constitué par ceux dans lesquels A...A représente une chaîne moléculaire de formule
- NH - Y - NH -, dans laquelle Y représente une chaîne alkyle linéaire de 2 à 10 atomes de carbone, de préférence de 2 à 7 atomes de carbone.

Parmi les composés de formule V, un groupe préféré est constitué par ceux dans lesquels A...A représente une chaîne moléculaire de formula
- NH - Y - NH -, dans laquelle Y représente une chaîne moléculaire de formule
- $(CH_2)_m$ - W - $(CH_2)_n$, dans laquelle m et n sont identiques ou différents et varient de 2 à 5, et W représente un groupe amine de formule

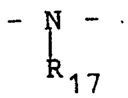

dans laquelle $R_{17}$ représente un atome d'hydrogène, ou un groupe alkyle de 1 à 3 atomes de carbone.

Parmi les composés de formule V, un groupe préféré est constitué par ceux dans lesquels A...A représente une chaîne de formule -NH-Y-NH-, dans laquelle Y représente $-CH_2-(CH_2OCH_2)_qCH_2$, q variant de 1 à 5, et valant, de préférence 2.

Les composés de formule V sont des intermédiaires clé du procédé et représentent un autre aspect de la présente invention.

On a rassemblé dans les tableaux B et B(BIS) ci-après des exemples respectivement de monomères IV et de dimères V, utilisés comme intermédiaires pour la préparation des monomères et dimères de formule II et III de l'invention et qui font également partie de l'invention.

On donne la signification de R, B et A...A ainsi que les numéros des composés correspondants.

TABLEAU B

| Type | B | R | n° du composé |
|---|---|---|---|
| IV | $NH-CH_2-CH_2-OH$ | $CH_3O$ | 66 |
| " | $NH-CH_2-CH_2OCOCH_2CH_2CH_3$ | " | 67 |
| " | $NH-CH_2-COOCH_3$ | " | 65 |
| " | 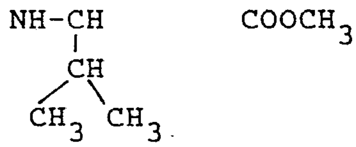 | " | 71 |
| " | 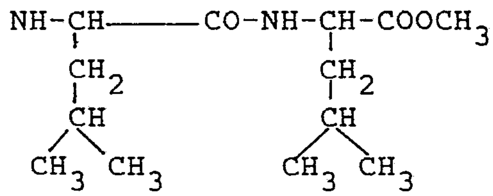 | " | 73 |
| " | $NH-CH_2-CH_2-N(CH_3)_2$ | " | 74 |
| " | $NH-CH_2-CH_2-N(C_2H_5)_2$ | " | 75 |
| " | $NH-CH_2-CH_2-CH_2-N(CH_3)_2$ | " | 62 |
| " | $NH-CH_2-CH_2-CH_2-N(C_2H_5)_2$ | " | 76 |
| " | 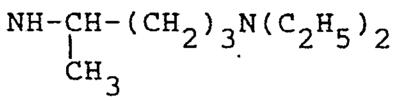 | " | 77 |
| " | 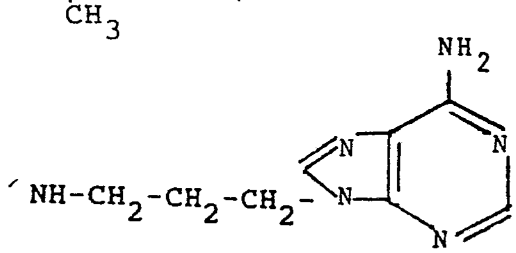 | | 78 |
| " | $NH-CH_2-CH_2-CH_2-N(CH_3)_2$ | 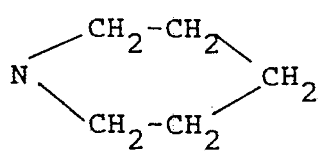 | 72 |

TABLEAU B (BIS)
(suite)

| Type | A....A | | $R_6$ | n° du composé |
|---|---|---|---|---|
| V | $NH-(CH_2)_2-NH$ | | $CH_3O$ | 51 |
| " | $NH-(CH_2)_7-NH$ | | " | 53 |
| " | 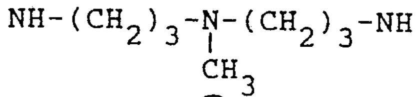 | | " | 55 |
| " | 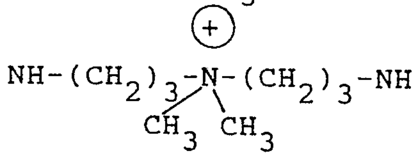 | $I^-$ | " | 56 |
| " | $NH-CH_2-(CH_2-O-CH_2)_2-CH_2-N$ | | " | 58 |
| " | 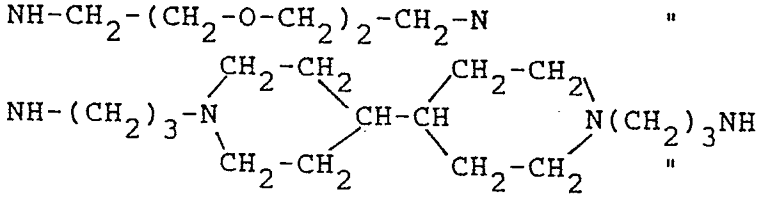 | | " | 70 |
| " | 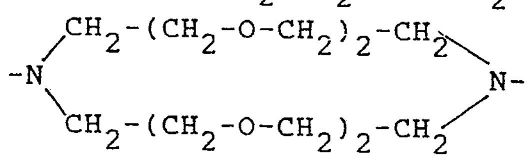 | | " | 68 |

On donne à titre d'exemple non limitatif un mode de réalisation détaillé du procédé de préparation des composés de l'invention.

Préparation de l'acide benzyloxy-2 méthoxy-3 nitro-6 benzoïque :

On ajoute goutte à goutte 700 ml d'une solution à 10 % de permanganate de potassium dans l'eau à 100 g (345 mmoles) de benzyloxy-2 méthoxy-3 nitro-6 benzaldéhyde dans 1,4 l d'acétone en maintenant le milieu réactionnel à 40°C.

Après 7 h d'agitation à 40°C, le précipité est éliminé par filtration, l'acétone est distillée. L'acide benzyloxy-2 méthoxy-3 nitro-6 benzoïque est précipité par addition d'acide chlorhydrique, essoré puis lavé à l'eau. Il est alors purifié par dissolution dans de la lessive de potasse au demi, traitement au charbon, filtration, précipitation par l'acide chlorhydrique et lavage à l'eau.

$C_{15}H_{13}NO_6$.PF = 176°C. Rdt : 71 %.

RMN (DMSO) : $OCH_3$ 3,95(s) ; $OCH_2$ 4,95(s) ; H aromatiques et $H_4$ 7,3(m) ; $H_5$ 7,95(d) , $J_{4-5}$ = 8 Hz ; OH 10(s).

Préparation de l'acide amino-6 benzyloxy-2 méthoxy-3 benzoïque :

A une solution à l'ébullition de 42 g (149 mmoles) d'acide benzyloxy-2 méthoxy-3 nitro-6 benzoïque dans 1 l d'ammoniaque au demi, on ajoute 269 g de $FeSO_4$, $7H_2O$ dans 1,1 l d'eau. Après une demi-heure d'ébuillition, on filtre le précipité, le lave à l'eau. Le filtrat est acidifié par de l'acide chlorhydrique jusqu'à pH 4. L'acide aminé est essoré, puis lavé à l'eau. On obtient l'acide amino-6 benzyloxy-2 méthoxy-3 benzoïque monotache (solvant : acétate d'éthyle-toluène-acide acétique 0,5-0,49-0,01) caractérisé par ses spectres d'IR et de RMN. En raison de son instabilité, il ne peut être purifié, $C_{15}H_{15}NO_4$ PF = 76-78°C. Rdt. : 79 %.

RMN (DMSO) : $OCH_3$ 3,75(s) ; $OCH_2$ 4,95(s), $H_5$ 6,6(d), $J_{4-5}$=8 Hz ; $H_4$ 7,05(d) ; H aromatiques et $NH_3$ 7,4(m).

Préparation de la benzyloxy-5 méthoxy-6 dihydro-3,4 quinazolinone-4 :

On chauffe pendant 30 h à l'ébullition, sous azote, une solution de 30 g (110 mmoles) d'acide amino-6 benzyloxy-2 méthoxy-3 benzoïque et de 8,9 g (110 mmoles) de s-triazine dans 675 ml d'éthanol sec contenant 7,5 ml de pipéridine. Le précipité obtenu après refroidissement est essoré, lavé à l'éthanol puis à l'éther. On obtient un dérivé monotache (solvant : acétate d'éthyl-méthanol 9,5-0,5). Le produit brut peut être utilisé pour la synthèse. $C_{16}H_{14}N_2O_3$. PF = 200° C (chromatographie sur colonne de silice. Solvant : acétate d'éthyle puis acétate d'éthyle méthanol 9,5-0,5). Rdt. : 80 %,

RMN (DMSO) ; $OCH_3$ 3,8(s) ; $OCH_2$ 4,9(s) ; H benzéniques, $H_7$ et $H_8$ 7,1 à 7,6(m) ; $H_2$ 7,8(s) ; NH 11,8(s).

Préparation de la benzyloxy-5 chloro-4 méthoxy-6 quinazoline :

On chauffe 3 h en agitant, à 80° C, sous azote, 16 g (57 mmoles) de benzyloxy-5 méthoxy-6 dihydro-3,4 quinazolinone-4 dans 5,2 ml (57 mmoles) d'oxychlorure de phosphore et 480 ml de benzène sec contenant 20,7 ml (147 mmoles) de triéthylamine. Après refroidissement, le mélange est versé sur de la glace. Le dérivé chloré est extrait au benzène qui est lavé avec de l'eau puis avec une solution à 10 % d'hydrogénocarbonate de sodium et de nouveau avec de l'eau jusqu'à élimination des chlorures. Après distillation du solvant, on obtient un solide jaune instable utilisable directement, contenant une légère impureté (CCM ; solvant : acétate d'éhtyle). $C_{16}H_{13}N_2ClO_2$. PF 137,5° C (flash chromatographie sur colonne de silice. Solvant : dichlorométhane - acétate d'éthyle 9,7-0,3). Rdt. : 80 %.

RMN (DMSO) ; $OCH_3$ 3,85(s) ; $OCH_2$ 4,5(s) ; H benzéniques et $H_7$ 7,3 à 7,6(m) ; $H_8$ 7,7 , $J_{7-8}$ = 8 Hz ; $H_2$ 8,65(s).

Préparation des benzyloxy-5 méthoxy-6 quinazolines substituées en 4 et des dimères de benzyloxy-5 méthoxy-6 quinazolines

On ajoute 11 mmoles de monoamine primaire BH ou 5,5 mmoles d'ω-diamine primaire HA.......AH à une solution de 3 g (10 mmoles) de benzyloxy-5 chloro-4 méthoxy-6 quinazoline dans l'éthanol sec contenant 11 mmoles (ou 22 mmoles si l'amine primaire est utilisée sous forme de sel) de triéthylamine.

On agite 24 h à la température ambiante. Après évaporation du solvent, on essore le précipité, le lave à l'eau ou à l'alcool. Lorsque le produit est liquide, on extrait au dichlorométhane, lave la solution à l'eau jusqu'à neutralité, sèche et évapore sous vide. Les produits sont cristallisés ou purifiés par flash chromatographie.

Les monomères obtenus répondent à la formule 6 indiquée ci-dessus et les dimères répondent à la formule 11 indiquée ci-dessus. Ils sont caractérisés par leur analyse centésimale et RMN.

On a indiqué dans les tableaux I et Ibis ci-après respectivement les composés intermédiaires 6 et 11, obtenus, entrant dans la préparation des composés de l'invention.

TABLEAU I

BENZYLOXY-5 METHOXY-6 QUINAZOLINES SUBSTITUEES EN 4 (COMPOSE 6)

| N° | B | Formule brute | PF (°C) | Solvant cristallisation | Rdt (%) |
|---|---|---|---|---|---|
| 6 | $NH - CH_2 - CH_2OH$ | $C_{18}H_{19}N_3O_3$ | 135.5 | $CH_3CN$ | 73 |
| 6 | $NH - CH_2 - COOCH_3$ | $C_{19}H_{19}N_3O_4$ | 120 | $CH_3OH - H_2O$ | 39 |
| 6 | $NH - CH - COOCH_3$<br>│<br>CH<br>╱ ╲<br>$CH_3$ $CH_3$ | $C_{22}H_{25}N_3O_4$ | huile | | 61 |
| 6 | $NH - CH - CONH - CH - COOCH_3$<br>│ │<br>$CH_2$ $CH_2$<br>│ │<br>CH CH<br>╱ ╲ ╱ ╲<br>$CH_3$ $CH_3$ $CH_3$ $CH_3$ | $C_{29}H_{38}N_4O_5$ | 50 | | 88 |
| 6 | $NH - CH_2 - CH_2 - N(CH_3)_2$ | $C_{20}H_{24}N_4O_2$ | huile | | 93 |
| 6 | $NH - CH_2 - CH_2 - N(C_2H_5)_2$ | $C_{22}H_{28}N_4O_2$ | huile | | 81 |
| 6 | $NH - CH_2 - CH_2 - CH_2 - N(CH_3)_2$ | $C_{21}H_{26}N_4O_2$ | huile | | 95 |
| 6 | $NH - CH_2 - CH_2 - CH_2 - N(C_2H_5)_2$ | $C_{23}H_{30}N_4O_2$ | huile | | 70 |
| 6 | $NH - CH - CH_2 - CH_2 - CH_2 - N(C_2H_5)_2$<br>│<br>$CH_3$ | $C_{25}H_{34}N_4O_2$ | huile | | 83 |
| 6 | $NH - CH_2 - CH_2 - CH_2 - C_5H_4N_5$ (*) | $C_{24}H_{24}N_8O_2$ | 155 | acétate éthyle | 66 |
| 6 | $NH - CH_2 - CH_2 - OCO - CH_2 - CH_2 - CH_3$ | $C_{22}H_{25}N_3O_4$ | huile | | 60 |

(*) $C_5H_4N_5$ = adényl-9

TABLEAU IBIS

DIMERES DE BENZYLOXY-5 METHOXY-6 QUINAZOLINES (COMPOSE 11)

| N° | A..............A | Formule brute | PF (°C) | Solvant cristallisation | Rdt (%) |
|---|---|---|---|---|---|
| 11 | $NH - (CH_2)_2 - NH$ | $C_{34}H_{32}N_6O_4$ | 196 | $C_6H_6$ | 74 |
| 11 | $NH - (CH_2)_7 - NH$ | $C_{39}H_{42}N_6O_4$ | 123.5 | $C_6H_6$ - éther de pétrole | 97 |
| 11 | $NH - (CH_2)_3 - N(CH_3) - (CH_2)_3 - NH$ | $C_{39}H_{43}N_7O_4$ | huile | | 56 |
| 11 | $NH - CH_2 - [CH_2 - O - CH_2]_2 - CH_2 - NH$ | $C_{38}H_{40}N_6O_6 \cdot 0,5\ H_2O$ | 139 | $CH_3OH$ | 60 |
| 11 | $NH - (CH_2)_3 -$ 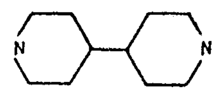 $- (CH_2)_3 - NH$ | $C_{48}H_{58}N_8O_4$ | 162 | $CH_3CN$ | 68 |
| 11 | 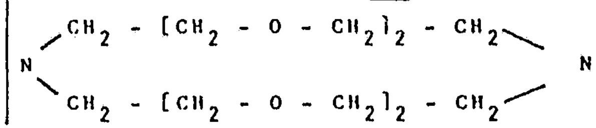 | $C_{44}H_{50}N_6O_8$ | 138 | $CH_3OH$ | 74 |

Préparation des hydroxy-5 méthoxy-6 quinazolines substituées en 4 et des dimères d'hydroxy-5 méthoxy-6 quinazolines

Méthode A : On chauffe à reflux pendant 3 à 5 h, sous azote, 3 mmoles de dérivés benzyloxy de formule 6 ou 11 indiquée dans 30 ml d'acide trifluoroacétique. On dilue avec du méthanol et évapore à sec sous vide. On reprend par de l'eau et amène à pH 9 par addition d'ammoniaque. On essore le précipité et le lave à l'eau.

Méthode B : On hydrogène à la pression atmosphérique et à la température ambiante 3 mmoles des dérivés de formule 6 ou 11 indiquée ci-dessus en solution dans un mélange méthanol-dioxanne (1-1) en présence de 1,3 g de charbon palladié à 10 %. Après filtration et lavage du catalyseur au méthanol, on évapore les solvants sous vide.

Sensibles à l'oxydation, ces hydroxy-5 quinazolines ne sont pas purifiées et sont utilisées brutes.

Les rendements sont compris entre 80 et 100 %. Les dérivés sont identifiés par leurs spectres RMN.

Les monomères obtenus répondent à la formule 7 indiquée ci-dessus et les dimères répondent à la formule 12 indiquée ci-dessus.

Préparation des méthoxy-6 quinazolinediones-5,8 substituées en 4 :

A une solution de 1 mmole d'hydroxyquinazoline de formule 7 ou 12 indiquée ci-dessus et de 1,15 mmoles (pour les monomères de formule 7) ou 2,3 mmoles (pour les dimères de formule 12) de dihyrogénophosphate de potassium dans un mélange de méthanol-eau (6-4) ou d'acétone-eau (6-4), on ajoute un léger excès de nitrosodisulfonate de potassium. On agite à la température ambiante jusqu'à disparition du dérivé de formule 7 ou 12 indiquée ci-dessus (chromatographie sur couche mince de silice). On élimine le solvent organique sous vide, ramène, lorsque cela est nécessaire, le milieu réactionnel à pH 7 par addition d'une solution aqueuse d'hydrogénocarbonate de sodium. On extrait au dichlorométhane. On sèche et élimine le solvant sous vide. On cristallise la quinone ou on la purifie par flash chromatographie. Les rendements sont de 25 à 80 % pour les monomères de formule IV et de 25 à 66 % pour les dimères de formula V.

Leur structure a été établie par RMN et IR et par leur analyse centésimale.

On a rassemblé dans les tableaux II et IIbis ci-après respectivement des exemples de monomères IV et de dimères V.

TABLEAU II

METHOXY-6 QUINAZOLINEDIONE-5,8 SUBSTITUEES EN 4

| N° du composé final | N° | B | Point de fusion | Solvant de cristallisation | Rdt (%) | Formule brute |
|---|---|---|---|---|---|---|
| 66 | IV | $NH - CH_2 - CH_2OH$ | 234 | $CH_3CN$ | 75 | $C_{11}H_{11}N_3O_4$ |
| 67 | IV | $NH - CH_2 - CH_2 - OCOCH_2CH_2CH_3$ | 155 | propanol | 80 | $C_{15}H_{17}N_3O_5$ |
| 65 | IV | $NH - CH_2 - CH_2 - COOCH_3$ | 235 | $CHCl_3$ - Ether de pétrole | 72 | $C_{12}H_{11}N_3O_5$<br>0,25 $H_2O$ |
| 71 | IV | $NH - CH - COOCH_3$<br>$CH$<br>$CH_3 \quad CH_3$ | 150 | (a) | 61 | $C_{15}H_{17}N_3O_5$ |
| 73 | IV | $NH - CH - CONH - CH - COOCH_3$<br>$CH_2 \quad CH_2$<br>$CH \quad CH$<br>$CH_3 \; CH_3 \quad CH_3 \; CH_3$ | 98 | (a) | 45 | $C_{22}H_{30}N_4O_6$ |
| 74 | IV | $NH - CH_2 - CH_2 - N(CH_3)_2$ | 158 | $C_6H_6$ - Ether de pétrole | 31 | $C_{13}H_{16}N_4O_3$ |
| 75 | IV | $NH - CH_2 - CH_2 - N(C_2H_5)_2$ | 174 | (a) | 25 | $C_{15}H_{20}N_4O_3$,<br>0,5 $H_2O$ |
| 62 | IV | $NH - CH_2 - CH_2 - CH_2 - N(CH_3)_2$ | 152 | acétate d'éthyle | 48 | $C_{14}H_{18}N_4O_3$ |
| 76 | IV | $NH - CH_2 - CH_2 - CH_2 - N(C_2H_5)_2$ | 169 | (a) | 30 | $C_{16}H_{22}N_4O_3$ |
| 77 | IV | $NH - CH - (CH_2)_3 - N(C_2H_5)_2$<br>$CH_3$ | 168 | (a) | 27 | $C_{18}H_{26}N_4O_3$ |
| 78 | IV | $NH - CH_2 - CH_2 - CH_2 - C_5H_4N_5$ (b) | 206 | $H_2O - CH_3OH$ | 30 | $C_{17}H_{16}N_8O_3$,<br>1,5 $H_2O$ |

a : voir TABLEAU IIBIS
b :

TABLEAU IIBIS

DIMERES DE METHOXY-6 QUINAZOLINEDIONE-5 8 (COMPOSE 13)

| Code | N° | A...........A | Point de fusion | Solvant cristallisation | Rdt (%) | Formule brute |
|---|---|---|---|---|---|---|
| 51 | V | NH - $(CH_2)_2$ - NH | 250 | DMF | 34 | $C_{20}H_{16}N_6O_6$ |
| 53 | V | NH - $(CH_2)_7$ - NH | 214 | THF | 49 | $C_{25}H_{26}N_6O_6$ |
| 55 | V | NH - $(CH_2)_3$ - $N(CH_3)$ - $(CH_2)_3$ - NH | 215 | $CH_3CN$ | 33 | $C_{25}H_{27}N_7O_6$, $2H_2O$ |
| 58 | V | NH - $CH_2$ - $(CH_2-O-CH_2)_2$ - $CH_2$ - NH | 233 | DMF | 59 | $C_{24}H_{24}N_6O_8$ |
| 70 | V | NH - $(CH_2)_3$ - 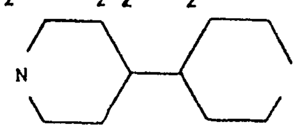 - $(CH_2)_3$ - NH | 247 | $CHCl_3$ - Ether de pétrole | 66 | $C_{34}H_{42}N_8O_6$ |
| 68 | V | 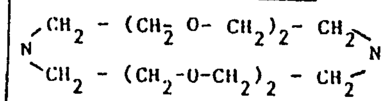 . | 96 | $CH_2Cl_2$ - Ether | 25 | $C_{30}H_{34}N_6O_{10}$, x $H_2O$ |
| 56 | V | NH - $(CH_2)_3$ - $\overset{+}{N}(CH_3)_2$ - $(CH_2)_3$ - NH $I^{\ominus}$ | 235 | $H_2O$ - $CH_3OH$ - acétone | 94 | $C_{26}H_{30}N_7IO_6$, 1,5 $H_2O$ |

a : purification par flash chromatographie

b : $C_5H_4N_5$ : adényl—9

Les monomères IV sont des composés intermédiaires pour la préparation de composés de l'invention dans lesquels $R_6$ représente les groupes aminés définis précédemment, c'est-à-dire un groupe amine primaire, un groupe amine secondaire ou tertiaire de 1 à 3 atomes de carbone, une pipéridine, une pyrrolidine, une diamine cyclique de 4 atomes de carbone de formule

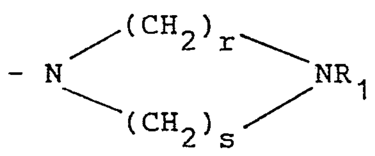

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle,
. une amine cyclique de formule

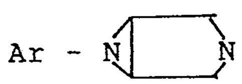

dans laquelle Ar a les significations indiquées ci-dessus.

Les monomères dans lesquels $R_6$ représente un groupe aminé défini ci-dessus sont représentés par 9 sur la figure I.

Les composés IV sont des composés intermédiaires pour la préparation de composés de l'invention dans lesquels $R_6$ et $R_7$ représentent simultanément une aziridine substituée ou non comme défini précédemment. Ces monomères sont représentés par 10 sur la figure I.

Les dimères V sont des composés intermédiaires pour la préparation de composés de l'invention dans lesquels $R_6$ représente les groupes aminés définis précédemment, c'est-à-dire un groupe amine primaire, un groupe amine secondaire ou tertiaire de 1 à 3 atomes de carbone, une pipéridine, une pyrrolidine, une diamine cyclique de 4 atomes de carbone de formule

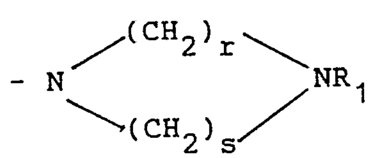

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle,
. une amine cyclique de formule

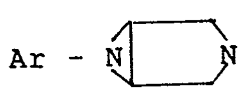

dans laquelle Ar a les significations indiquées ci-dessus.

Les composés V sont également des composés intermédiaires pour la préparation de composés dans lesquels $R_6$ et $R_7$ représentent simultanément une aziridine substituée ou non comme défini précédemment.

Ces dimères sont représentés par 14 sur la figure II.

<u>Préparation des pipéridino-6 quinazolinediones-5,8 et des bis-(aziridinyl-1)-6,7 quinazolinediones-5,8 :</u>

Les pipéridino-6 quinazolinediones-5,8 correspondent au composé 9 de l'invention et sont obtenues de la façon suivante :

On ajoute 1 mmole de quinone IV à une solution maintenue à 0° C de 0,13 ml (1,3 mmole) de pipéridine dans 28 ml de méthanol. Après 5 h d'agitation à 0° C, on évapore à sec, extrait au dichlorométhane. On lave à l'eau, puis évapore à sec. On purifie par flash chromatographie.

Les bis-(aziridinyl-1)-6,7 quinazolinediones-5,8 et les dimères de bis-(aziridinyl-1)-6,7 quinazolinediones-5,8 correspondent respectivement aux composés 10 et 14 de l'invention définis ci-dessus et sont obtenus de la façon suivante :

On ajoute sous azote 0,25 mmole de méthoxyquinone IV ou v dans le minimum d'aziridine maintenue à 0° C. On laisse en contact à cette température jusqu'à disparition du dérivé méthoxylé (5-24 h) (chromatographie sur couche mince de silice). On élimine sous vide l'excès d'aziridine. On extrait au dichlorométhane. On lave à l'eau jusqu'à neutralité. On évapore le solvant et purifie l'aziridinylquinone par flash chromatographie. Les constantes des composés de l'invention 9, 10 et 14 sont indiquées dans le tableau III et IIIbis ci-après.

TABLEAU III

PIPERIDINO-6 QUINAZOLINEDIONE-5 8 (COMPOSE 9) ET

BIS (AZIRIDINYL-1)-6,7 QUINOLINEDIONES-5,8 (COMPOSE 10)

| Code | N° | B | Point de fusion | Solvant de cristallisation | Rendement (%) | Formule brute |
|---|---|---|---|---|---|---|
| 72 | 9 | $NH-(CH_2)_3-N(CH_3)_2$ | 105°C | (a) | 37 | $C_{18}H_{25}N_5O_2$ 0,25$H_2O$ |
| 63 | 10 | $NH-(CH_2)_3-N(CH_3)_2$ | 105°C | (a) | 35 | $C_{17}H_{22}N_6O_2$ 1$CH_3OH$ |

TABLEAU IIIBIS

DIMERES DE BIS (AZIRIDINYL-1)-6,7 QUINOLINEDIONES-5,8
(COMPOSE 14)

| Code | N° | A......A | Point de fusion | Solvant de cristallisation | Rendement (%) | Formule brute |
|---|---|---|---|---|---|---|
| 52 | 14 | $NH-(CH_2)_2-NH$ | 200°C | (a) | 11 | $C_{20}H_{16}N_6O_6 \cdot 1H_2O$ |
| 54 | 14 | $NH-(CH_2)_7-NH$ | 147°C | benzène - éther de pétrole | 7 | $C_{31}H_{34}N_{10}O_4$ |
| 57 | 14 | $NH-(CH_2)_3-N(CH_3)-(CH_2)_3NH$ | 160°C | $CH_2Cl_2$ - éther de pétrole | 7 | $C_{31}H_{35}N_{11}O_4$ |
| 59 | 14 | $NH-CH_2-[CH_2-O-CH_2]_2-CH_2NH$ | 123°C | $CH_2Cl_2$ - éther de pétrole | 20 | $C_{30}H_{32}N_{10}O_6$ $0,5CH_2Cl_2$ |

(a) : purification par flash chromatographie

Synthèse du dérivé 6, dans lequel B = $NH-CH_2-CH_2-OCO-CH_2-CH_2-CH_3$ (tableau I)

On dissout vers 40° C 500 mg (1,5 mmole) de dérivé 6, dans lequel B = $NH-CH_2-CH_2OH$ (cf. tableau I) dans 125 ml de benzène et 3 ml de triéthylamine. Après refroidissement vers 5° C, on ajoute goutte à goutte 245 mg (2,3 mmoles) de chlorure de butyryle. On chauffe 24 h à 50°C. On verse sur de la glace et extrait au benzène. On lave à l'eau et élimine le solvant. On purifie par flash chromatographie.

Synthèse du dérivé de l'invention 56 (cf. tableau IIbis) :

A une suspension de 150 mg (0,3 mmoles) de quinone 55 (cf. tableau IIbis) dans 3 ml d'acétone à 0° C, on ajoute 3 ml d'iodométhane. On agite 30 mm à 0°C puis 27 h à 20°C. On élimine le solvant sous vide.

Etude pharmacologique :

- Détermination de la cytotoxicité

La détermination de la cytotoxicité in vitro sur les cellules leucémiques L 1210 est la méthode utilisée pour sélectionner les molécules potentiellement antitumorales. Le protocole mis en oeuvre est le suivant :

les cellules leucématiques L 1210 adaptées à la culture en suspension stationnaire sont entretenues dans du milieu RPMI 1640 (commercialisé par Seromed) additionné de 20 % de sérum de cheval décomplémenté, L-glutamine (2 mM), pénicilline (200 U/ml), streptomycine (50 µg/ml) et cultivées à 37° C dans une atmosphère à 5 % de $CO_2$. Les cultures testées par la méthode de BARILE (BARILE M.F. Mycoplasmal contamination of cell culture, in Contamination in Tissue Culture, J. Fogh (ed.) Acad. Press, 132-156, 1973) sont exemptes de contaminations bactériennes et mycoplasmiques.

Pour les expérimentations, les cellules sont ensemencées à la concentration de $0{,}8.10^5$ cellules/ml et cultivées par 2 ml dans des flacons de 15 ml. Les substances à étudier sont dissoutes dans du DMSO (1 % final) dont on a préalablement vérifié l'innocuité. Des quantités croissantes de produit à tester sont ajoutées au temps 0 et des numérations sont faites au compteur COULTER après 24 et 48 heures de culture. Des séries de contrôle sont effectuées avec des quantités de DMSO équivalentes à celles des séries traitées.

Le temps de doublement des cellules témoins est approximativement de 12 heures.

La cytotoxicité est déterminée d'après l'inhibition de croissance des cellules L 1210, calculée par rapport aux témoins correspondants selon la formule :

$$\frac{T - t}{T} \times 100$$

T = concentration de cellules non traitées,

t = concentration de cellules traitées.

La concentration de produit qui diminue la croissance cellulaire de 50 % ($CI_{50}$) est calculée d'après la méthode des régressions linéaires en coordonnées log-probit.

Lorsque la $CI_{50}$ est voisine de la micromolarité (égale ou inférieure à 1 µM), il existe une présomption d'activité antitumorale et les essais in vivo indiqués ci-après sont effectués.

A titre d'exemple, la CI50 mesurée 48 heures après traitement des cellules par les composés de l'invention testés est la suivante :

| Numéro composé de l'invention | $CI_{50}$ après 48 h de culture |
|---|---|
| 54 | 0,0037 µM |
| 57 | 0,0048 µM |
| 59 | 0,018 µM |
| 70 | 1,30 µM |
| 63 | 0,0027 µM |

- Etude de l'action antitumorale du composé 63 :

* Effet sur la croissance cellulaire in vitro

Les cellules tumorales en croissance exponentielle sont ensemencées (10.000 cellules/ml) dans des boîtes à Petri contenant au fond de la boîte un couvre-objet (18 x 18 mm) et 3 ml de MEM (MEM = milieu essentiel minimum) + 5 % FCS (FCS = serum de veau fetal) (v/v). Après 24 heures d'incubation, quand les cellules sont bien attachées au fond de la boîte, le milieu est remplacé par MEM expérimental contenant 5 % FCS (v/v) et enrichi, par exemple de :

a) 3 ml du milieu (MEM + 5 % FCS) = témoins

b) 3 ml du milieu contenant une dose élevée du composé à tester : 10 µg/ml : (M)

c) milieu contenant une faible dose du composé à tester : 0.01 µg/ml (f) respectivement aux jours 1, 2, 3 et 4 après addition du milieu expérimental dans les boîtes de Petri. Trois couvre-objets sont fixés pour chaque condition expérimentale : éthanol 95° (75 v), formol neutre à 40 % (20 v), acide acétique (5 v), pendant 10 minutes. Les cellules témoins cultivées dans le milieu expérimental sans le produit à tester sont fixées au même moment. A la fin de la fixation, les couvre-objets sont retournés et montés sur des lames histologiques à l'aide de produit commercialisé sous la marque DPX par la Société British Drug House et conservés à l'abri de la lumière et à 4° en attendant d'être colorés.

Les lames avec les couvre-objets sont colorées par une réaction de Feulgen modifiée. Toutes les lames d'une condition expérimentale sont conditionnées par le réactif de Feulgen en même temps, pendant une heure, après hydrolyse dans un milieu HCl 6N pendant une heure à 20°C. Les préparations cellulaires sont ensuite conservées à l'abri de la lumière et à 4°C en attendant l'analyse.

Grâce à un système d'analyse microscopique à balayage automatique, on détermine l'effet de la drogue sur la croissance cellulaire, au cours du temps, sur deux lignées cellulaires tumorales d'origine humaine : la lignée MCF-7 d'origine mammaire et la lignée HBL (mélanome malin).

On a représenté dans le tableau C les résultats relatifs à l'action du composé 63 sur la croissance des cellules de lignée MCF-7 et dans le tableau C BIS, les résultats relatifs à l'action du composé 63 sur la croissance des cellules de lignée HBL. Ces résultats montrent le nombre de cellules survivant au traitement après 1, 2, 3 et 4 jours d'incubation et à trois doses différentes (10 µg/ml, 1 µg/ml et 0,1 µg/ml).

Chaque chiffre correspond au nombre de cellules observées par champ de microscope.

TABLEAU C

Effet sur la croissance des cellules de lignée MCF-7

| | 1j | 2j | 3j | 4j |
|---|---|---|---|---|
| CONTROLE (Ct) | 133± 13 | 187± 19 | 254± 46 | 683± 63 |
| 10µg/ml (F) | 128± 11<br>ns | 93± 4<br>** | 87± 20<br>** | 88± 18<br>*** |
| 1µg/ml (M) | 133± 7<br>ns | 85± 8<br>** | 58± 7<br>** | 63± 8<br>*** |
| 0.1µg/ml (f) | 174± 38<br>ns | 92± 21<br>** | 38± 6<br>** | 30± 4<br>*** |

ns = non significatif
* p = 0,10
** p = 0.05
*** p = 0,01

TABLEAU C BIS

Effet sur la croissance des cellules de lignée HBL

| | 1j | 2j | 3j | 4j |
|---|---|---|---|---|
| CONTROLE (Ct) | 352± 12 | 466± 20 | 616± 72 | 750± 86 |
| 10µg/ml (F) | 318± 83<br>ns | 166± 28<br>*** | 553±100<br>ns | 210± 48<br>* |
| 1µg/ml (M) | 282± 17<br>ns | 187± 35<br>*** | 199± 71<br>** | 197± 67<br>* |
| 0.1µg/ml (f) | 402±100<br>ns | 233± 36<br>*** | 633± 83<br>ns | 474±265<br>ns |

* p = 0,10
** p = 0,05
*** p = 0,01

On a représenté sur le tableau D les résultats relatifs aux effets du composé 63 au cours du temps sur les différentes phases du cycle cellulaire (lignée MCF-7) où il est possible d'observer le nombre de cellules suivant à chaque phase du cycle cellulaire.

On a représenté sur le tableau D BIS les résultats relatifs aux effets du composé 63 au cours du temps sur les différentes phases du cycle cellulaire (lignée HBL) où il est possible d'observer le nombre de cellules survivant à chaque phase du cycle cellulaire.

Dans les tableaux,

G0-G1, S et G2 + M correspondent à des différentes phases de multiplication des cellules, telles qu'elles sont définies dans "Cell proliferation kinetics of MCF-7 human mammary carcinome cells in culture and effects of tamoxifen on exponentially growing and plateau - phase cells" R.L. Sutherland, R.E. Hall and I.W. Taylor, Cancer Research 43, pp. 3998 à 4006, 1983.

TABLEAU D

Effets sur les paramètres de cinétique cellulaire

| | 1 jour | | | 2 jours | | | 3 jours | | | 4 jours | | |
|---|---|---|---|---|---|---|---|---|---|---|---|---|
| | G0-G1 | S | G2±M | G0-G1 | S | G2±M | G0-G1 | S | G2±M | G1-G0 | S | G2±M |
| | 62.0 ± 2.0 | 19.7 ± 3.2 | 18.3 ± 1.9 | 56.7 ± 2.3 | 27.0 ± 2.7 | 16.3 ± 1.7 | 56.3 ± 2.6 | 39.0 ± 2.3 | 14.7 ± 0.3 | 58.2 ± 1.8 | 27.3 ± 3.1 | 14.5 ± 0.7 |
| F | 42.7 ± 1.7 *** | 48.3 ± 1.2 *** | 9.0 ± 0.6 ** | - ± - | - ± - | - ± - | - ± - | - ± - | - ± - | - ± - | - ± - | - ± - |
| M | 53.0 ± 2.7 * | 32.7 ± 2.4 * | 14.3 ± 0.4 ns | - ± - | - ± - | - ± - | - ± - | - ± - | - ± - | - ± - | - ± - | - ± - |
| f | 46.0 ± 1.5 *** | 34.3 ± 3.8 ** | 19.7 ± 2.3 ns | 47.3 ± 2.3 * | 34.3 ± 3.2 ns | 18.3 ± 1.8 ns | 46.7 ± 1.5 * | 38.0 ± 1.5 * | 15.3 ± 0.3 ns | 59.6 ± 1.9 ns | 24.3 ± 2.6 ns | 17.1 ± 2.7 ns |

* p = 0,10 *** p = 0,01 ns = non significatif
** p = 0,05

TABLEAU D BIS

Effets sur les paramètres de cinétique cellulaire

| | 1 jour | | | 2 jours | | | 3 jours | | | 4 jours | | |
|---|---|---|---|---|---|---|---|---|---|---|---|---|
| | G0-G1 | S | G2+M | G0-G1 | S | G2+M | G0-G1 | S | G2+M | G1-G0 | S | G2+M |
| | 65.0 ± 4.0 | 19.7 ± 3.3 | 15.3 ± 1.2 | 73.3 ± 1.8 | 12.7 ± 0.9 | 14.0 ± 1.2 | 70.7 ± 4.3 | 17.7 ± 2.2 | 11.6 ± 2.2 | 63.0 ± 3.0 | 28.0 ± 2.5 | 9.0 ± 0.6 |
| F | 62.7 ± 2.0 ns | 23.3 ± 2.0 ns | 14.0 ± 0.1 ns | 58.7 ± 0.9 *** | 28.0 ± 2.1 ** | 13.3 ± 2.2 ns | 59.7 ± 0.9 * | 28.0 ± 1.0 ** | 12.3 ± 0.7 ns | 59.0 ± 1.5 ns | 28.7 ± 0.7 ns | 12.3 ± 1.9 ns |
| M | 62.3 ± 1.9 ns | 25.0 ± 3.6 ns | 12.7 ± 1.5 ns | 58.3 ± 1.3 *** | 28.0 ± 1.0 ** | 13.7 ± 1.2 ns | 56.7 ± 1.8 ** | 28.0 ± 3.1 ** | 15.3 ± 1.3 ns | 58.0 ± 0.6 ns | 26.0 ± 1.7 ns | 16.0 ± 2.1 * |
| f | 56.0 ± 1.5 * | 31.3 ± 2.6 * | 12.7 ± 1.2 ns | 53.0 ± 1.5 *** | 36.7 ± 3.7 *** | 10.3 ± 2.2 ns | 53.7 ± 1.3 ** | 35.3 ± 0.9 *** | 11.0 ± 0.6 ns | 53.3 ± 1.2 ** | 32.7 ± 3.5 ns | 14.0 ± 2.3 ns |

* p = 0,10  
** p = 0,05  
*** p = 0,01

Les résultats des tableaux C et D montrent que le composé 63 ralentit fortement la croissance des cellules MCF-7 d'une manière "dose-indépendante" et bloque les cellules en phase S du cycle cellulaire.

Les résultats obtenus avec la lignée provenant d'une métastase de mélanome malin (lignée HBL), et rassemblés dans les tableaux C BIS et D BIS montrent que le composé 63 ralentit faiblement la croissance des cellules HBL à moyenne et forte dose et bloque les cellules en phase S du cycle cellulaire.

- Détermination de la toxicité

Les produits insolubles dans l'eau (tels que par exemple les produits 57 et 59) sont mis en suspension dans une solution aqueuse à 4 o/oo d'hydroxypropylcellulose (commercialisé sous le nom de Klucel J F par la Société Herculès). Ils sont tous administrés, par voie intrapéritonéale (ip), à des souris Swiss de 22 ± 2 g, à doses uniques exprimées en mg/kg de poids corporel. Les animaux sont suivis pendant un mois. La mortalité et les signes de toxicité sont relevés. On détermine ainsi la $DL_0$qui sera la dose maximale utilisée pour la détermination de l'activité antitumorale.

Ainsi à titre d'exemple les $DL_0$ déterminées pour les composés 57, 59 et 63 sont respectivement voisines de 2,5 mg/kg, 10 mg/kg et 3 mg/kg.

D'une manière générale, les $DL_0$ mesurées sont toutes supérieures aux doses auxquelles les produits selon l'invention sont actifs in vivo sur les tumeurs expérimentales qui suivent.

- Détermination de l'activité antitumorale

Afin de tester l'activité antitumorale des composés décrits plus haut, on a utilisé les leucémies murines P388 et L1210, ainsi que le mélanome murin B16. La leucémie P388 est considére comme très sensible et permet une première sélection. D'autre part, une substance ayant une grande activité sur la leucémie L1210 a une grande chance d'être active en clinique humaine (J.M. Venditti; Relevance of transplantable animal tumor systems to the Selection of new agents for clinical trial in pharmacological basis of cancer chemotherapy. The University of Texas ed. Williams and Wilkins, publ. 1975, Baltimore USA).

La leucémie L1210 de la souris est en effet couramment utilisée pour l'évaluation de tous les composés antitumoraux actuellement mis en oeuvre en thérapeutique humaine, ainsi qu'il est décrit, par exemple par C.C. Zubrod dans Proc. Nat. Acad. Sci. USA 69, 1972, p. 1042-1047. Le système tumoral ainsi constitué expérimentalement permet une évaluation quantitative très précise de l'activité du composé testé, et, par conséquent aussi, une comparaison objective entre les activités respectives de différents composés, par

exemple selon les méthodes décrites par R. E. Skipper, F. M. Schabel, Jr. et W. S. Wilcox dans Cancer Chemother. Rep., 35, 1964, p. 1-111 et 45, 1965, p.5-28.

Cette étude a été réalisée sur 1, 2 ou 3 tumeurs murines expérimentales selon le schéma suivant :

| Tumeur expérimentale | Hôte | Taille de l'innoculum tumoral (cellules) | |
|---|---|---|---|
| | | ip | iv |
| Leucémie P388 | souris DBA/2 | $10^6$ | $10^5$ |
| Leucémie L1210 | souris DBA/2 | $10^5$ | - |
| Mélanome B16 | souris BDF1 | 0,5 ml susp. au 1/10° | - |

Les produits en solution dans l'eau ou en suspension dans du Klucel sont administrés par voie intrapéritonéale 24 heures après la greffe des cellules tumorales (10 animaux par série), soit en injection unique ($J_1$), soit pendant 9 jours consécutifs ($J_{1-9}$). Les morts sont relevés chaque jour à la même heure. La comparaison du temps médian de survie des souris témoins (C) et des souris traitées (T) permet d'apprécier l'activité antitumorale du produit. On adopte comme critère d'activité minimale la valeur de T/C supérieure ou égale à 125 % conformément aux critères établis par GERAN et par l'O.M.S. (Réf. GERAN R.I., GREENBERG N.H., MACDONALD M.M., SCHUMACHER A.M. and ABBOTT B.J. Protocols for screening chemical agents and natural products against animal tumors and other biological systems. Cancer Chemother. Rep. Part 3, 3, 1-103 (1972)).

Lorsque le composé est actif sur plusieurs tumeurs, son administration par d'autres voies que la voie intrapéritonéale (i.p.) est envisagée : orale (p.o) et intraveineuse (i.v). Les résultats des essais effectués à l'aide du composé 63 sont rassemblés dans le tableau IV ci-après.

Les résultats des essais effectués à l'aide des composés de l'invention n° 57 et 59 sont rassemblés dans le tableau IV Bis ci-après.

Dans les tableaux IV et IV Bis, P représentent la variation de poids entre J1 et J5 des animaux traités ; lorsque P est supérieure à environ 4 g, cela signifie que la dose à laquelle le composé a été testé est toxique.

TABLEAU IV

ACTIVITE ANTITUMORALE DU COMPOSE 63 EN SOLUTION AQUEUSE SUR LES LEUCEMIES MURINES P388, L1210 ET SUR LE MELANOME B16

| Tumeur | Protocole greffe | Protocole traitement | Nombre d'epxériences | Doses actives** (mg/kg/inj.) | Dose optimale (mg/kg/inj.) | Δ P J1-J5 | T/C* (%) | survivants 30 jours |
|---|---|---|---|---|---|---|---|---|
| P388 | ip($10^6$cel.) | ip J1 | 3 | 0,1-3 | 3 | -2,0 | 188 | 0 |
| | | | | | 1,25 | -1,7 | 174 | 1 |
| | " | ip J1-9 | 1 | 0,017-0,8 | 0,4 | -2,1 | 222 | 0 |
| | " | po J1 | 1 | 20-30 | 30 | -4,7 | 140 | 0 |
| | " | iv J1 | 1 | (2 doses testées) | 1,25 | -0,7 | 136 | 0 |
| | iv($10^5$cel.) | iv J1 | 1 | (1 seule dose testée) | 1,25 | -0,1 | 147 | 0 |
| L1210 | ip($10^5$cel.) | ip J1 | 1 | 0,25-2 | 2 | -2,8 | 160 | 1 |
| | | ip J1-9 | 1 | 0,03-0,8 | 0,4 | -1,6 | 174 | 1 |
| | | | | | | | | survivants 60 jours |
| B16 | ip(susp. au 1/10) | ip J1-9 | 2 | 0,05-0,8 | 0,2 | 0 | 192 | 2 |

* survivants inclus da s calcul T/C  ** doses actives T/C ≥ 125 %

TABLEAU IVBIS

ACTIVITE ANTITUMORALE SUR LA LEUCEMIE P388 DES COMPOSES 57 ET 59 EN SUSPENSION DANS LE KLUCEL 4 °/oo

| N° du composé | Protocole greffe | Protocole traitement | Dose (mg/kg) | Animaux survivants/animaux traités | ΔP (J5-J1) | T/C (%) (*) |
|---|---|---|---|---|---|---|
| 57 | 1p ($10^6$ cel) | 1p J1 | 5 | 10/10 | -4,2 | 168 |
| | " | " | 2,5 | 10/10 | -1,8 | 150 |
| | " | " | 1,25 | 06/06 | -1,0 | 137 |
| 59 | " | " | 15 | 07/10 | -4,1 | 145 |
| | " | " | 10 | 10/10 | -1,8 | 135 |
| | " | " | 5 | 10/10 | -1,1 | 133 |
| | " | " | 2,5 | 10/10 | -0,5 | 115 |

(*) signification si T/C ≥ 125 %

Les premiers essais effectués avec le composé 63 à des doses infratoxique montrent une nette activité sur le réticulosarcome M 5076 sur la souris.

Les produits de l'invention ont tous une activité antitumorale significative.

On constate que les composés de l'invention 57 et 59 sont nettement plus actifs que le composé quinazolinedione décrit dans la publication J. Med. Chem. 1983, 26, 1715-1719, précédemment mentionné (tableau IV).

D'autre part, le composé 63, qui présente l'énorme avantage d'être hydrosoluble, est doté d'une excellente activité antitumorale sur la leucémie P388, lorsqu'il est administré par voie intrapéritonéale ; de plus, il reste actif par voie orale et par voie intraveineuse. Il est également très efficace sur la leucémie L1210 et sur le mélanome B16.

Les produits selon l'invention sont donc particulièrement appropriés pour l'usage thérapeutique chez l'homme, notamment pour le traitement de cancers sensibles à une chimiothérapie.

L'invention concerne aussi les compositions pharmaceutiques comprenant les composés nouveaux susdits en association avec un véhicule pharmaceutique approprié au mode d'administration choisi.

L'invention concerne particulièrement des poudres stériles propres à être utilisées pour la préparation, notamment extemporanée, de solutions injectables aptes à être administrées par injections intraveineuses, intradermiques, intramusculaires, sous-cutanées ou in situ.

Les doses administrées quotidiennement doivent être suffisantes pour qu'une action puisse se manifester au moins dans une proportion relativement importante de patients atteints de l'une ou l'autre des diverses formes de cancer qui sont ou seront accessibles à la chimiothérapie, cependant sans pour autant excéder

celles pour lesquels les composés deviendraient trop toxiques.

A titre d'exemple, on pourrait proposer pour les essais de phase I clinique, la dose de 0.9 $mg/m^2$ de corps humain en traitement unique. En effet, il est généralement admis que les doses à utiliser peuvent être calculées, sur la base de $mg/m^2$, à partir du 10è de la DL10 chez la souris (le passage des doses exprimées en mg/kg aux doses en $mg/m^2$, se fait chez cette espèce animale selon un facteur de 3). Freireich, E.J., Gehan, E.A., Rall, D.P., Schmidt, L.H., and Skipper, H.E. Quantitative comparison of toxicity of anticancer agents in mouse, rat, hamster, dog, monkey and man. Cancer Chemother. Rep. 50, 219-244, 1966 - Rozencweig, M., Von Hoff, D.D., Staquet, J., Schein, P.S., Penta, J.S., Goldin, A., Muggia, F.M., Freireich, E.J., and DeVita, Jr. V.T. Animal toxicology for early clinical trials with anticancer agents. Cancer Clin. Trials 4, 21-28, 1981.

L'invention concerne également les autres formes d'administration, notamment par voie orale (compositions solides ou liquides).

Du fait de leur activité particulièrement importante, ils sont également utiles comme produits de référence dans des études pharmacologiques, notamment aux fins de comparaison de propriétés antitumorales de produits étudiés par rapport à un produit de référence.

FIGURE I

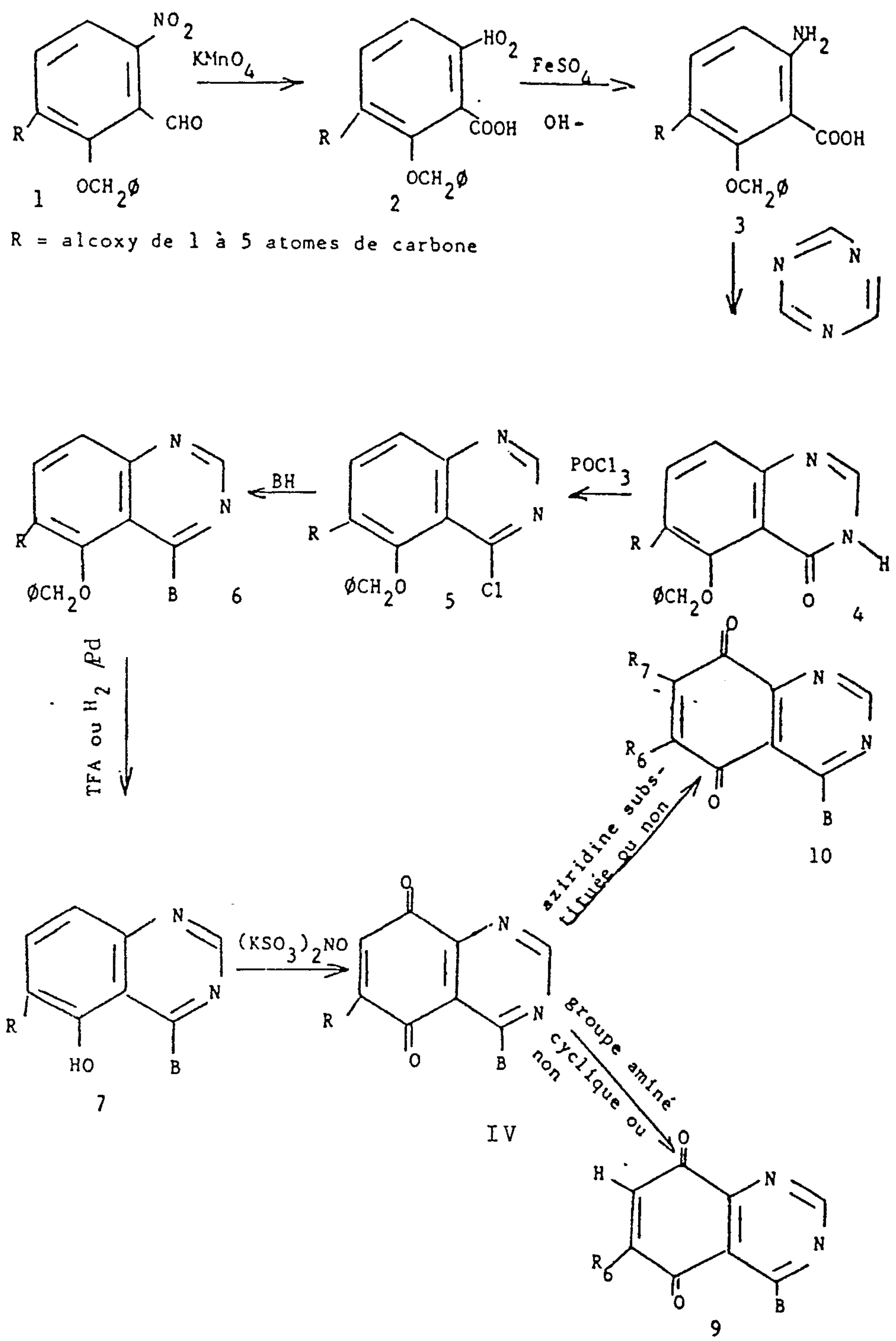
KMnO4
FeSO4
OH-
R = alcoxy de 1 à 5 atomes de carbone
POCl3
BH
TFA ou H2 Pd
(KSO3)2NO
aziridine substituée ou non
groupe aminé cyclique ou non

FIGURE II

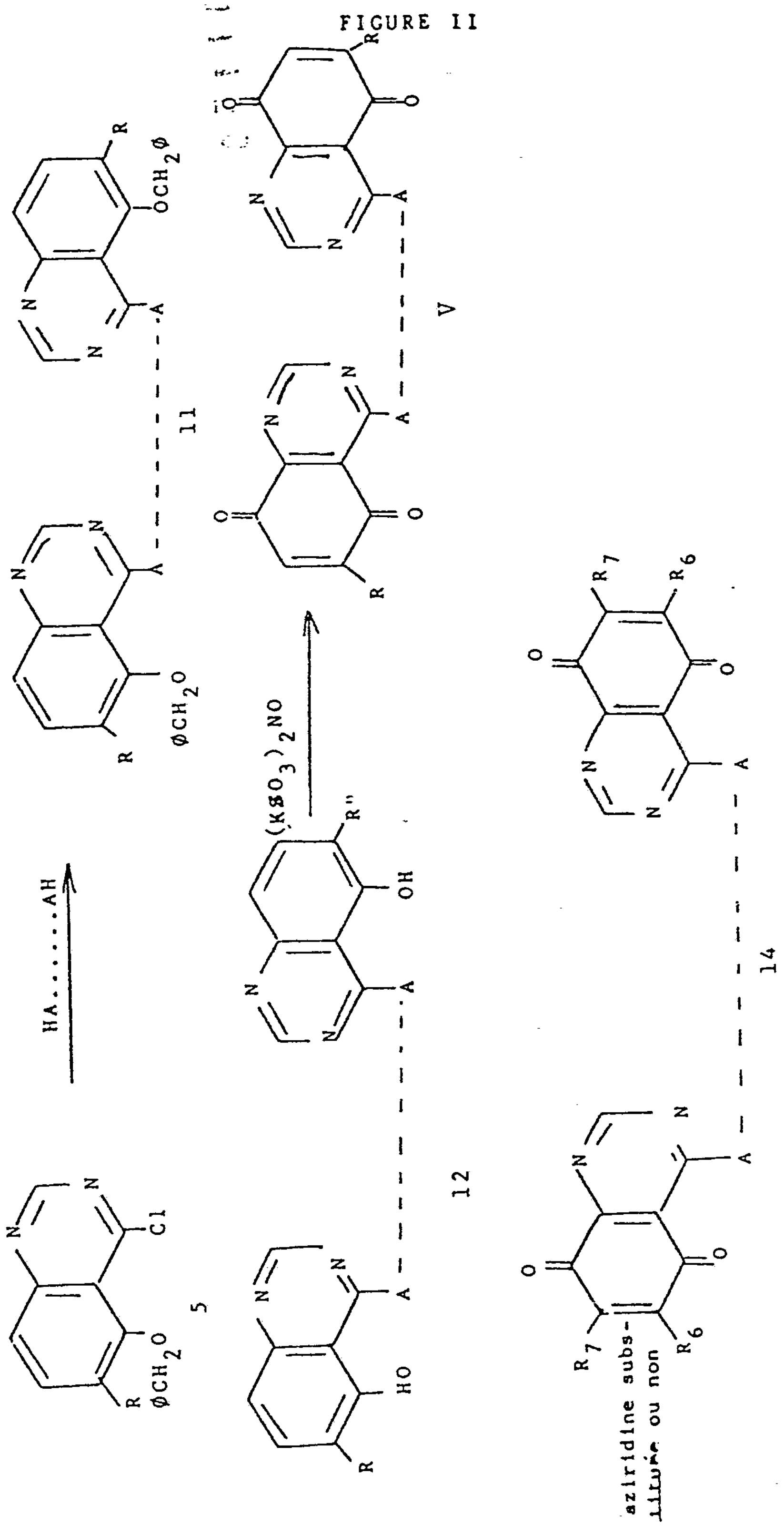


5 11 12 V 14

## Revendications

1. Composés répondant à la formule suivante :

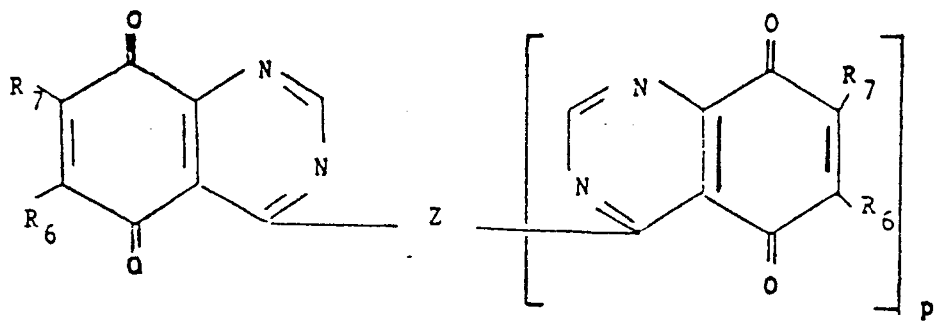

I

dans laquelle :

- p est égal à 0 ou 1,
- $R_7$ représente un atome d'hydrogène
- et $R_6$ représente :
  . un groupe amine primaire,
  . un groupe amine secondaire ou tertiaire de 1 à 3 atomes de carbone,
  . une pipéridine,
  . une pyrrolidine,
  . une diamine cyclique de 4 atomes de carbone de formule

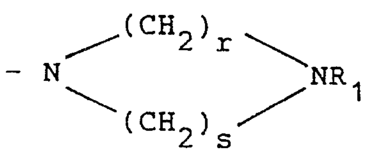

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,
  . une amine cyclique de formule

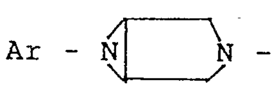

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
ou bien $R_6$ et $R_7$ sont identiques et représentent simultanément un groupe aziridine de formule

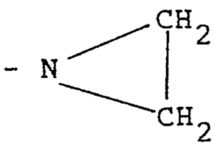 ,

ou un groupe aziridine mono, bi, tri ou tétrasubstitué par des groupes alkyle identiques ou différents, de 1 à 4 atomes de carbone, de préférence de 1 à 2 atomes de carbone,
et lorsque p est égal à 0, Z représente B,
. B représentant un groupe de formule
- NH - $R_4$ X
dans lequel
- $R_4$ représente une chaîne alkyle, linéaire ou ramifiée, de 1 à 7 atomes de carbone, de préférence de 2 à 3 atomes de carbone,
- X représente
. un atome d'hydrogène,
. un groupe hydroxyle OH,
. un groupe oxycarbonyle de formule
- O - CO - $R_5$ dans laquelle $R_5$ est une groupe alkyle linéaire ou ramifié de 1 à 5 atomes de carbone, de préférence de 1 à 3 atomes de carbone,
. un groupe ester de formule - $COOR_8$ dans laquelle $R_8$ est un groupe alkyle linéaire ou ramifié de 1 à 3 atomes de carbone, ou un groupement benzyle,
. un radical de formule

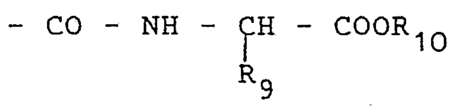

dans laquelle $R_9$ est un atome d'hydrogène ou un groupe alkyle linéaire ou ramifié, de 1 à 4 atomes de carbone, de préférence de 1 à 2 atomes de carbone, et $R_{10}$ représente un groupe alkyle identique ou non à $R_9$ comportant de préférence de 1 à 2 atomes de carbone,
. un groupe amine de formule

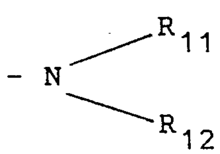

dans laquelle $R_{11}$ et $R_{12}$ sont des groupes alkyle identiques ou différents, linéaires ou ramifiés, de 1 à 3 atomes de carbone, de préférence de 1 à 2 atomes de carbone,
B représentant notamment un groupe de formule

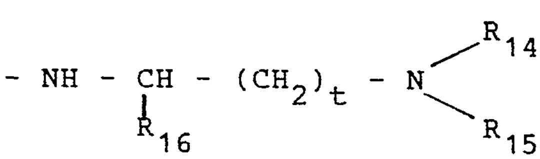

dans laquelle
t vaut de 1 à 3,
$R_{16}$ représente un atome d'hydrogène ou un groupe alkyle de 1 à 3 atomes de carbone, de préférence de 1 atome de carbone,
- $R_{14}$ et $R_{15}$ sont des groupes alkyle identiques ou différents de 1 à 3 atomes de carbone, de préférence de 1 à 2 atomes de carbone,
ou B représentant un peptide comportant de 2 à 5 acides aminés et dont la partie C-terminale se présente

sous forme d'un groupement COOH ou d'un ester de formule - $COOR_{13}$ dans laquelle $R_{13}$ est un groupe alkyle de 1 à 2 atomes de carbone, ou un groupement benzyle, et lorsque p est égal à 1, Z représente A...A,

. A...A représentant soit une chaîne de formule - NH - Y - NH -

. Y représentant

* une chaîne alkyle linéaire de 2 à 10 atomes de carbone de préférence de 2 à 7,

* une chaîne de formule

- $(CH_2)_m$ - W - $(CH_2)_n$ -, dans laquelle m et n sont identiques ou différents et varient de 2 à 5, et W représente soit un groupe amine de formule

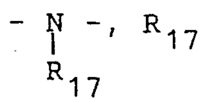

$R_{17}$ représentant un atome d'hydrogène, un groupe alkyle de 1 à 3 atomes de carbone, soit un groupe ammonium quaternaire de formule

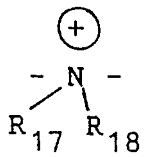

$R_{17}$ et $R_{18}$ représentant des groupes alkyle identiques ou différents de 1 à 3 atomes de carbone, lequel groupe ammonium est associé à un anion minéral ou organique physiologiquement acceptable,

* un radical choisi parmi les formules suivantes

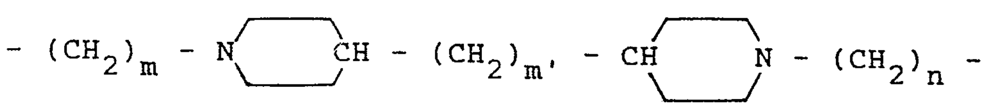

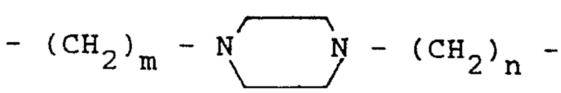

dans lesquelles m et n sont identiques ou différents, et varient de 2 à 5, de préférence de 2 à 3, m' valant de 0 à 3, et de préférence égal à 0 ou 1,

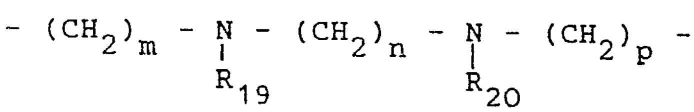

m, n, p identiques ou différents variant de 2 à 5, de préférence de 2 à 4,

$R_{19}$ et $R_{20}$ identiques ou différents représentant un atome d'hydrogène ou une chaîne alkyle de 1 à 3 atomes de carbone,

- $CH_2$ - $(CH_2 - O - CH_2)_q$ - $CH_2$ -

- q variant de 1 à 5, et valant de préférence 2

* ou A...A représentant une chaîne de formule

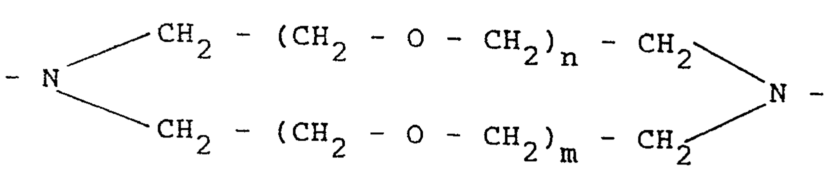

m et n, identiques ou différents, variant de 2 à 5, de préférence de 2 à 3.

2. Composés selon la revendication 1, répondant à formule II suivante :

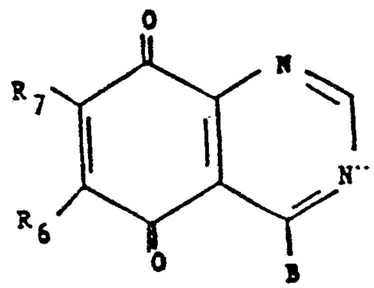

II

dans laquelle $R_6$, $R_7$ et B ont les significations indiquées à la revendication 1.

3. Composés selon les revendications 1 ou 2, caractérisés en ce que B représente un groupe de formule $NH-R_4-X$, dans lequel $R_4$ représente un chaîne alkyle, linéaire ou ramifiée de 2 à 5 atomes de carbone et X représente un groupe amine de formule

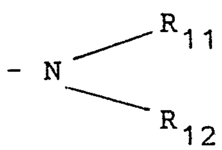

,

dans laquelle $R_{11}$ et $R_{12}$ sont des groupes alkyle identiques ou différents, linéaires ou ramifiés, de 1 à 3 atomes de carbone, de préférence de 1 à 2 atomes de carbone.

4. Composés selon les revendications 1 à 3, caractérisés en ce que
$R_6$ et $R_7$ sont identiques et représentent simultanément
. un groupe aziridine de formule :

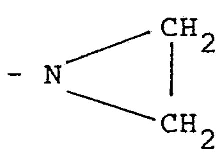

,

. ou un groupe aziridine mono, bi, tri ou tétrasubstitué par des groupes alkyle identiques ou différents, de 1 à 4 atomes de carbone, de préférence de 1 à 2 atomes de carbone.

5. Composé selon la revendication 2, répondant à la formule suivante

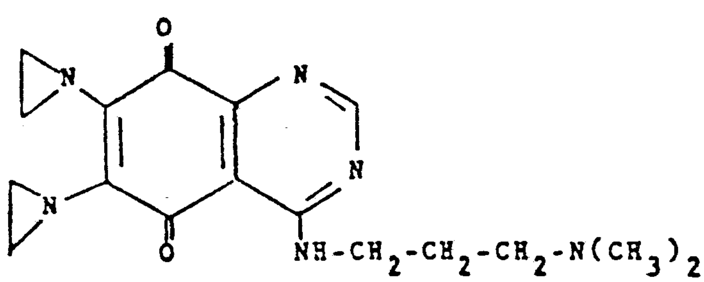

(63)

6. Composés selon la revendication 1 répondant à la formule III suivante :

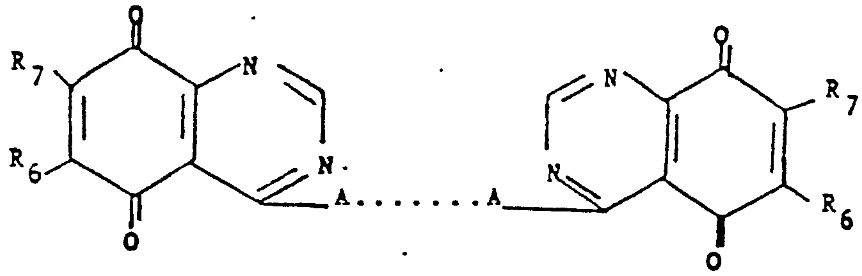 III

dans laquelle A, $R_6$ et $R_7$ ont les significations indiquées à la revendication 1.

7. Composés selon la revendication 6 caractérisés en ce que $R_6$ et $R_7$ sont identiques et représentent simultanément

. un groupe aziridine de formule

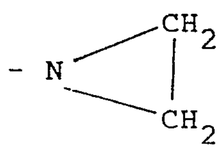 ,

. ou un groupe aziridine mono, bi, tri ou tétrasubstitué par des groupes alkyle, identiques ou différents, de 1 à 4 atomes de carbone, de préférence de 1 à 2 atomes de carbone.

8. Composés selon les revendications 6 et 7 caractérisés en ce que A...A représente un chaîne moléculaire de formule - NH - Y - NH -, dans laquelle Y représente une chaîne alkyle linéaire de 2 à 10 atomes de carbone, de préférence de 2 à 7 atomes de carbone.

9. Composés selon les revendications 6 et 7, caractérisés en ce que A...A représente une chaîne moléculaire de formule - NH - Y - NH -, dans laquelle Y représente une chaîne moléculaire de formule - $(CH_2)_m$ - W - $(CH_2)_n$, dans laquelle m et n sont identiques ou différents et varient de 2 à 5, et W représente un groupe amine de formule

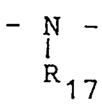

dans laquelle $R_{17}$ représente un atome d'hydrogène, ou un groupe alkyle de 1 à 3 atomes de carbone.

10. Composés selon les revendications 6 et 7, caractérisés en ce que A...A représente une chaîne de formule -NH-Y-NH-, dans laquelle Y représente $-(CH_2)-(CH_2OCH_2)_q-CH_2-$, q variant de 1 à 5, et valant, de préférence 2.

11. Composés selon la revendication 6, répondant aux formules suivantes

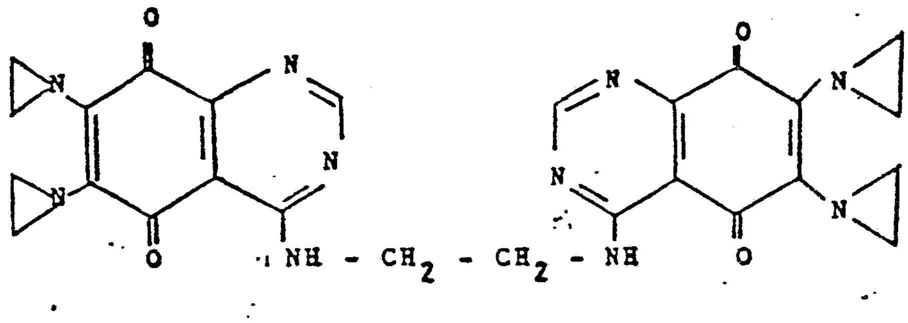

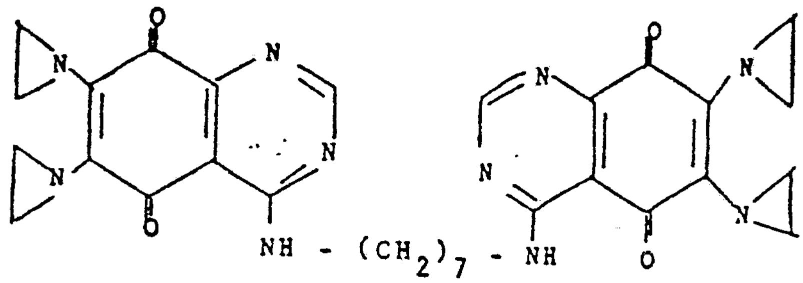

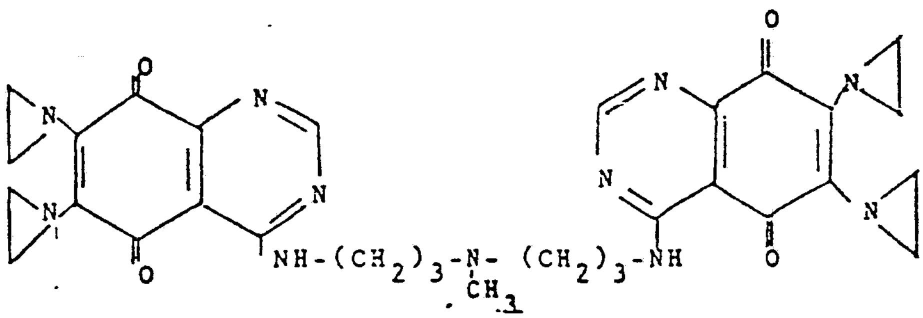

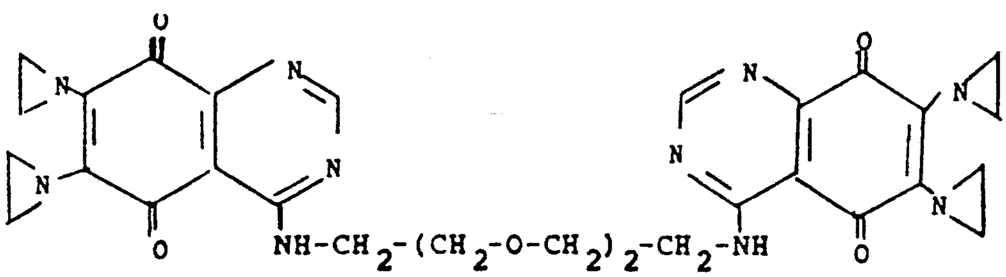

12. Procédé de préparation des composés selon les revendications 1 à 11, caractérisé en ce que on oxyde à l'aide d'un oxydant, notamment $KMnO_4$ le composé de formule 1 :

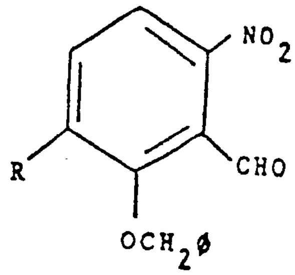

1

dans laquelle
- Ø représente un cycle benzénique,
- R représente un groupe alcoxy de 1 à 5 atomes de carbone,
pour obtenir un composé de formule 2

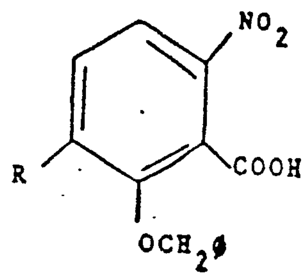

2

- on réduit le groupe $NO_2$ du composé précédent en groupe $NH_2$, notamment à l'aide de $FeSO_4$, pour obtenir un composé de formule 3

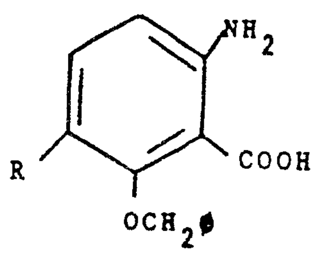

3

- on condense le composé précédent avec la triazine symétrique pour obtenir un composé de formule 4

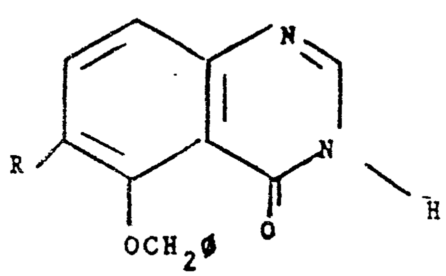

4

- on chlore le composé précédent, notamment à l'aide d'oxychlorure de phosphore, pour obtenir un composé de formule 5

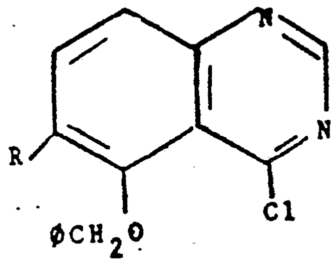

5

et dans le cas où le composé final souhaité est un monomère de formule II, selon la revendication 2,
- on condense le composé de formule 5 sur le composé de formule BH, dans laquelle
. B représente un groupe de formule
- NH - $R_4$ X
dans laquelle
- $R_4$ représente une chaîne alkyle linéaire ou ramifiée de 1 à 7 atomes de carbone, de préférence de 2 à 3,
- X représente
. un atome d'hydrogène,
. un groupe hydroxyle OH,
. un groupe oxycarbonyle de formule
- O - CO - $R_5$ dans laquelle $R_5$ est un groupe alkyl linéaire ou ramifié de 1 à 5 atomes de carbone, de préférence de 1 à 3 atomes de carbone,
. un groupe ester de formule - $COOR_8$ dans laquelle $R_8$ est un groupe alkyle linéaire ou ramifié de 1 à 3 atomes de carbone, ou un groupement benzyle,
. un radical de formule

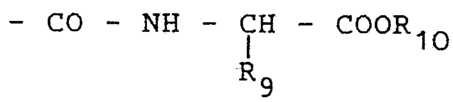

dans laquelle $R_9$ est un atome d'hydrogène ou un groupe alkyle linéaire ou ramifié, de 1 à 4 atomes de carbone, de préférence de 1 à 2 atomes de carbone, et $R_{10}$ représente un groupe alkyle identique ou non à $R_9$ comportant de préférence de 1 à 2 atomes de carbone,
. un groupe amine de formule

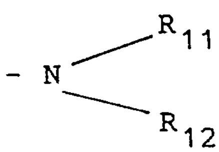

dans laquelle $R_{11}$ et $R_{12}$ sont des groupes alkyle identiques ou différents, linéaires ou ramifiés, de 1 à 3 atomes de carbone, de préférence de 1 à 2 atomes de carbone,
B représentant notamment
. un groupe de formule

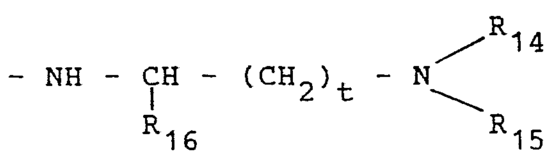

dans laquelle
t vaut de 1 à 3,
$R_{16}$ représente un atome d'hydrogène ou un groupe alkyle de 1 à 3 atomes de carbone, de préférence de 1 atome de carbone,

- $R_{14}$ et $R_{15}$ sont des groupes alkyle identiques ou différents de 1 à 3 atomes de carbone, de préférence de 1 à 2 atomes de carbone,

. ou B représentant un peptide comportant de 2 à 5 acides aminés et dont la partie C-terminale se présente sous forme d'un groupe COOH ou d'un ester de formule - $COOR_{13}$ dans laquelle $R_{13}$ est un groupe alkyle de 1 à 2 atomes de carbone, ou un groupement benzyle, pour obtenir un composé de formule 6

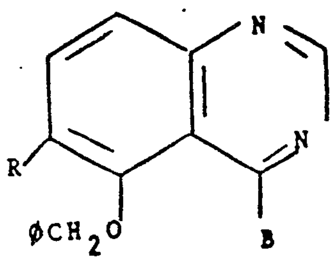

6

- on élimine le groupe benzyle du composé précédent, pour obtenir un composé de formule 7

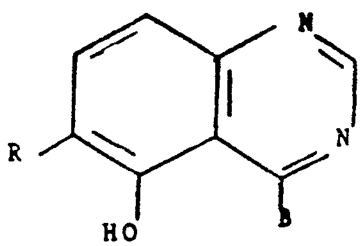

7

- on oxyde le composé précédent pour obtenir un monomère, de formule IV

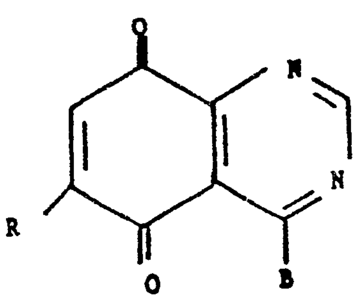

IV

dans laquelle R a la signification indiquée ci-dessus pour R,

- on condense le composé précédent dans lequel R représente un groupe alcoxy de 1 à 5 atomes de carbone, notamment un groupe méthoxy,

* soit sur
  . de l'ammoniaque,
  . une amine primaire ou une amine secondaire de 1 à 3 atomes de carbone,
  . la pipéridine,
  . la pyrrolidine,
  . une diamine cyclique de 4 atomes de carbone de formule

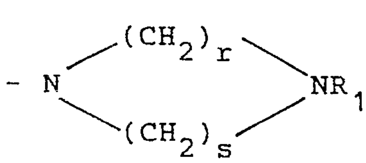

r et s étant des nombres entiers tels que le couple (r,s) représente (2,2) ou bien (r,s) représente (1,3),

(3,1) ou (0,4),
dans laquelle $R_1$ représente une atome d'hydrogène ou un groupe alkyle de 1 à 3 atomes de carbone, notamment la pipérazine,

. une amine cyclique de formule

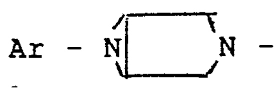

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
pour obtenir un monomère selon la revendication 2, de formule II

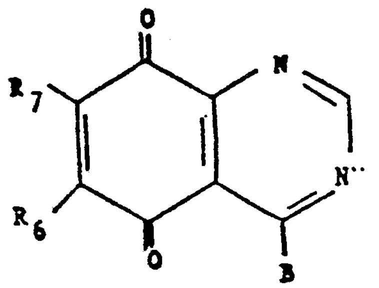

II

dans laquelle $R_6$ représente

. une amine primaire,

. un groupe amine secondaire ou tertiaire de 1 à 3 atomes de carbone,

. une pipéridine,

. une pyrrolidine,

. une diamine cyclique de 4 atomes de carbone de formule

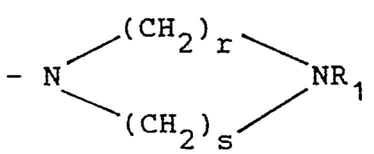

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,

. une amine cyclique de formule

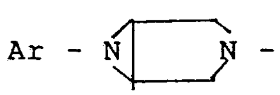

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
* soit sur au moins deux équivalents d'aziridine de formule

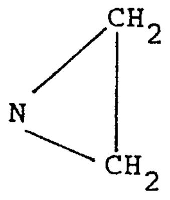

ou sur au moins deux équivalents d'une aziridine mono, bi, tri ou tétrasubstituée par des groupes alkyle, identiques ou différents, de 1 à 4 atomes de carbone, pour obtenir un monomère selon la revendication 2, dans la formule duquel $R_6$ et $R_7$ représentent simultanément une aziridine de formule

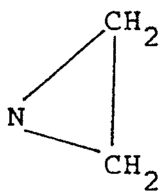

ou respectivement la même aziridine mono, bi, tri ou tétrasubstituées par des groupes alkyle, identiques ou différents, de 1 à 4 atomes de carbone,

- et dans le cas où le composé final souhaité est un dimère de formule III selon la revendication 6, on condense le composé de formule 5 sur le composé de formule HA...AH, dans laquelle soit A...A représente

- NH - Y - NH -

. Y représentant

* une chaîne alkyle linéaire de 2 à 10 atomes de carbone de préférence de 2 à 7,
* une chaîne de formule

- $(CH_2)_m$ - W - $(CH_2)_n$ - dans laquelle m et n sont identiques ou différents et varient de 2 à 5, et W représente soit un groupe amine de formule

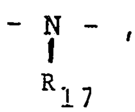

$R_{17}$ représentant un atome d'hydrogène, un groupe alkyle de 1 à 3 atomes de carbone,

. un radical choisi parmi les formules suivantes

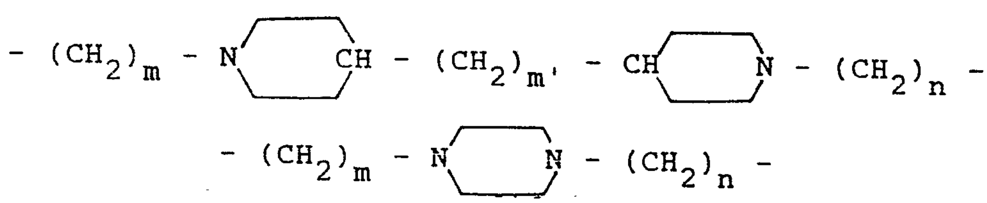

m et n identiques ou différents variant de 2 à 5, de préférence de 2 à 3,
m' valant de 0 à 3, et de préférence égal à 0 ou 1,

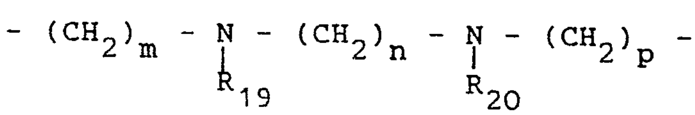

m, n, p identiques ou différents variant de 2 à 5, de préférence de 2 à 4,

$R_{19}$ et $R_{20}$ identiques ou différents représentant un atome d'hydrogène ou une chaîne alkyle de 1 à 3 atomes de carbone,

- $CH_2$ - $(CH_2$ - O - $CH_2)_q$ - $CH_2$ -

- q variant de 1 à 5, et valant de préférence 2,

. soit A...A représente une chaîne de formule

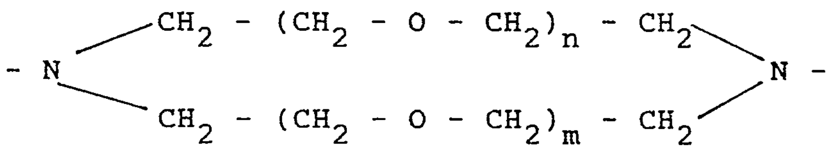

dans laquelle m et n sont identiques ou différents et varient de 2 à 5, de préférence de 2 à 3, pour obtenir le composé de formule 11

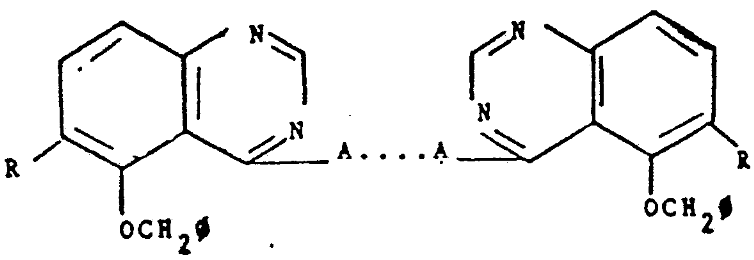

11

- puis on élimine les groupes benzyle du composé précédent pour obtenir le composé de formule 12

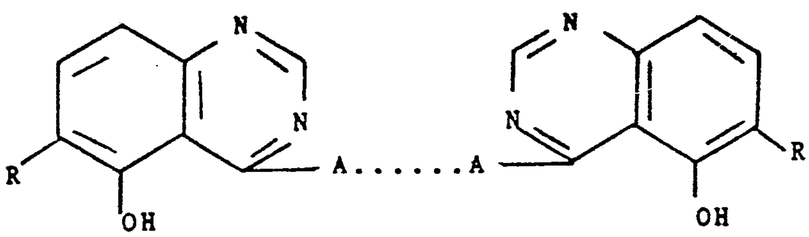

12

- on oxyde le composé précédent pour obtenir un dimère, de formule V :

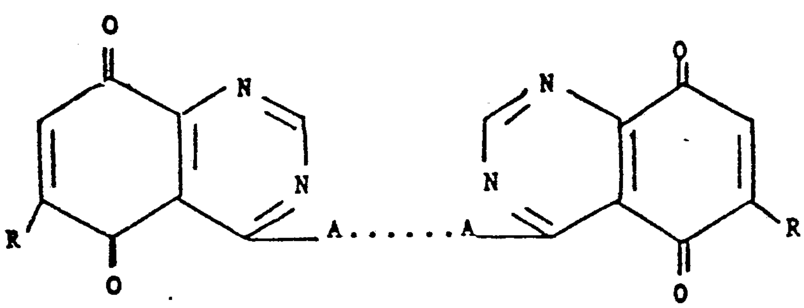

V

dans laquelle R a la signification indiquée ci-dessus,

- on condense le composé précédent dans lequel R représente un groupe alcoxy de 1 à 5 atomes de carbone, notamment un groupe méthoxy

* soit sur

. de l'ammoniaque,

. une amine primaire ou secondaire de 1 à 3 atomes de carbone,

. la pipéridine,

. la pyrrolidine,

. une diamine cyclique de 4 atomes de carbone de formule

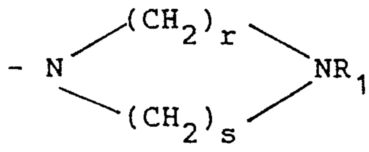

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,

. une amine cyclique de formule

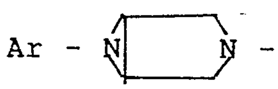

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
pour obtenir un dimère selon la revendication 7, de formule III

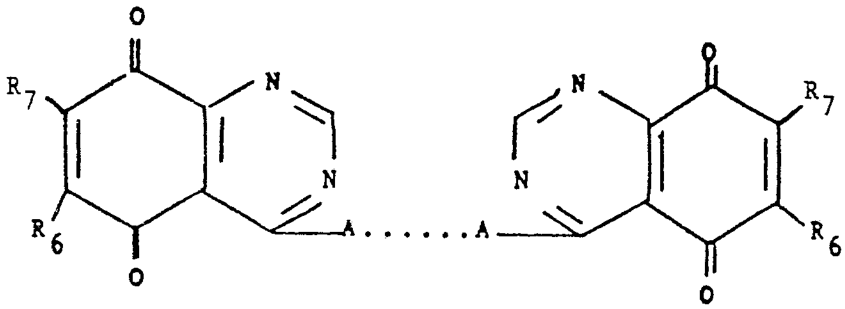

III

dans laquelle $R_7$ représente l'hydrogène
$R_6$ représente

. une amine primaire,
. un groupe amine secondaire ou tertiaire de 1 à 3 atomes de carbone,
. une pipéridine,
. une pyrrolidine,
. une diamine cyclique de 4 atomes de carbone de formule

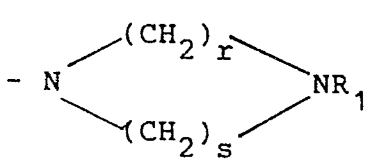

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,

. une amine cyclique de formule

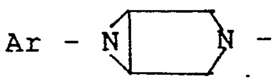

dans laquelle Ar représente un cycle benzénique non substitué ou substitué par un groupe alkyle de 1 à 2 atomes de carbone, avantageusement un méthyle, et/ou substitué par 1 ou 2 atomes d'halogène. notamment le chlore,
* soit sur au moins quatre équivalents d'aziridine de formule

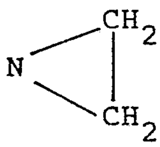

ou sur au moins quatre équivalents d'une aziridine mono, bi, tri ou tétrasubstituée par des groupes alkyle, identiques ou différents, de 1 à 4 atomes de carbone,
pour obtenir un dimère selon la revendication 6 dans lequel $R_6$ et $R_7$ représentent simultanément une aziridine de formule

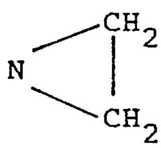

ou respectivement la même aziridine mono, bi, tri ou tétrasubstituées par des groupes alkyle, identiques ou différents, de 1 à 4 atomes de carbone,
- éventuellement on quaternarise l'azote tertiaire

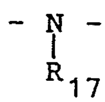

de dimères selon la revendication 6 de formule III, dans laquelle Y représente une chaîne de formule
$-(CH_2)_m-W-(CH_2)_n-$, dans laquelle m et n sont identiques ou différents et varient de 2 à 5, et W représente un groupe amine de formule

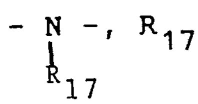

$R_{17}$ représentant un atome d'hydrogène, un groupe alkyle de 1 à 3 atomes de carbone.

13. Procédé, selon la revendication 12, de préparation des composés selon la revendication 2, caractérisé en ce que l'on oxyde à l'aide d'un oxydant, notamment $KMnO_4$ le composé de formule 1

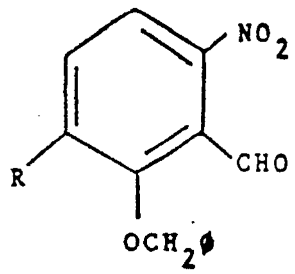

1

dans laquelle
- Ø représente un cycle benzénique,
- R représente un groupe alcoxy de 1 à 5 atomes de carbone,
pour obtenir un composé de formule 2

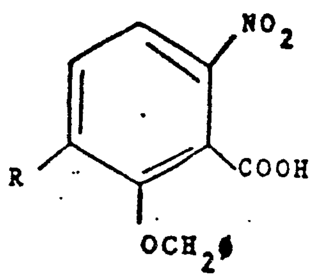

2

- on réduit le groupe $NO_2$ du composé précédent en groupe $NH_2$, notamment à l'aide de $FeSO_4$, pour obtenir un composé de formule 3

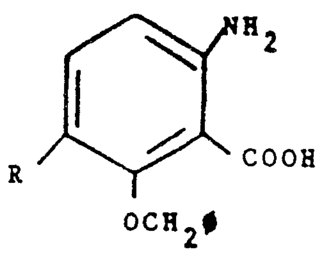

3

- on condense le composé précédent avec la triazine symétrique pour obtenir un composé de formule 4

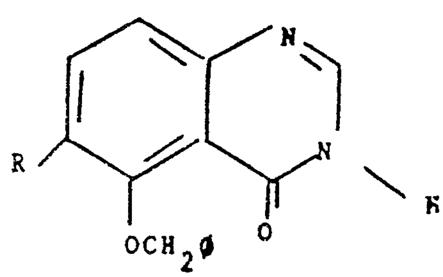

4

- on chlore le composé précédent, notamment à l'aide d'oxychlorure de phosphore, pour obtenir un composé de formule 5

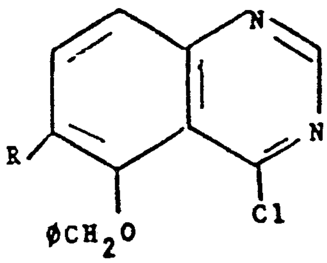

5

- on condense le composé de formule 5 sur le composé de formule BH, dans laquelle
. B représente un groupe de formule
- NH - $R_4$ X
dans laquelle
- $R_4$ représente une chaîne alkyle linéaire ou ramifiée de 1 à 7 atomes de carbone, de préférence de 2 à 3,
- X représente
. un atome d'hydrogène,
. un groupe hydroxyle OH,
. un groupe oxycarbonyle de formule
- O - CO - $R_5$ dans laquelle $R_5$ est un groupe alkyle linéaire ou ramifié de 1 à 5 atomes de carbone, de préférence de 1 à 3 atomes de carbone,
. un groupe ester de formule - $COOR_8$ dans laquelle $R_8$ est un groupe alkyle linéaire ou ramifié de 1 à 3 atomes de carbone, ou un groupement benzyle,
. un radical de formule

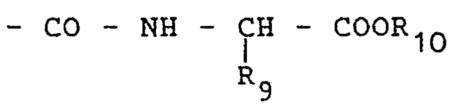

dans laquelle $R_9$ est un atome d'hydrogène ou un groupe alkyle linéaire ou ramifié, de 1 à 4 atomes de carbone, de préférence de 1 à 2 atomes de carbone, et $R_{10}$ représente un groupe alkyle identique ou non à $R_9$ comportant de préférence de 1 à 2 atomes de carbone,
. un groupe amine de formule

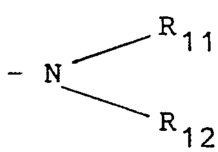

dans laquelle $R_{11}$ et $R_{12}$ sont des groupes alkyle identiques ou différents, linéaires ou ramifiés, de 1 à 3 atomes de carbone, de préférence de 1 à 2 atomes de carbone,
B représentant notamment
. un groupe de formule

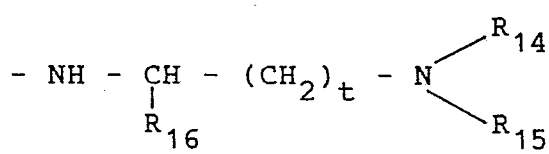

dans laquelle
t vaut de 1 à 3,
$R_{16}$ représente un atome d'hydrogène ou un groupe alkyle de 1 à 3 atomes de carbone, de préférence de 1 atome de carbone,
- $R_{14}$ et $R_{15}$ sont des groupes alkyle identiques ou différents de 1 à 3 atomes de carbone, de préférence

de 1 à 2 atomes de carbone,

. ou B représentant un peptide comportant de 2 à 5 acides aminés et dont la partie C-terminale se présente sous forme d'un ester de formule - $COOR_{13}$ dans laquelle $R_{13}$ est un groupe alkyle de 1 à 2 atomes de carbone, ou un groupement benzyle,

pour obtenir un composé de formule 6

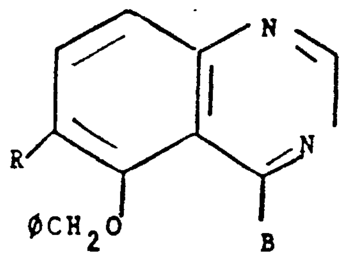

6

- on élimine le groupe benzyle du composé précédent, pour obtenir un composé de formule 7

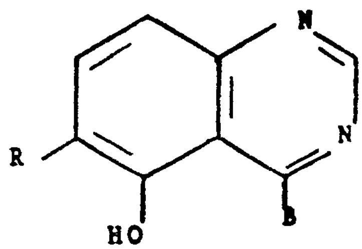

7

- on oxyde le composé précédent pour obtenir un monomère, de formule IV

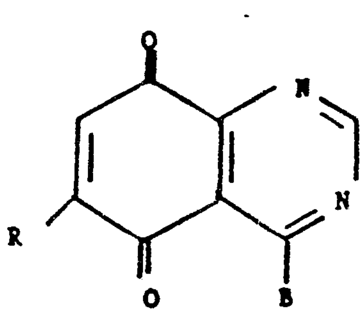

IV

dans laquelle R a la signification indiquée ci-dessus,

- on condense le composé précédent dans lequel R représente un groupe alcoxy de 1 à 5 atomes de carbone, notamment un groupe méthoxy,

* soit sur

. de l'ammoniaque,

. une amine primaire ou une amine secondaire de 1 à 3 atomes de carbone,

. la pipéridine,

. la pyrrolidine,

. une diamine cyclique de 4 atomes de carbone de formule

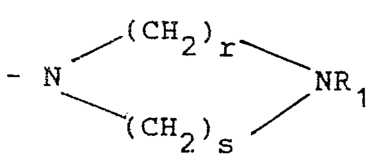

r et s étant des nombres entiers tels que le couple (r,s) représente (2,2) ou bien (r,s) représente (1,3), (3,1) ou (0,4),

dans laquelle $R_1$ représente un atome d'hydrogène ou un groupe alkyle de 1 à 3 atomes de carbone, notamment la pipérazine,

. une amine cyclique de formule

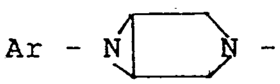

dans laquelle Ar représente un cycle benzénique non substitué ou substitué par un groupe alkyle de 1 à 2 atomes de carbone, avantageusement un méthyle, et/ou substitué par 1 ou 2 atomes d'halogène. notamment le chlore,

pour obtenir un monomère selon la revendication 2, de formule II

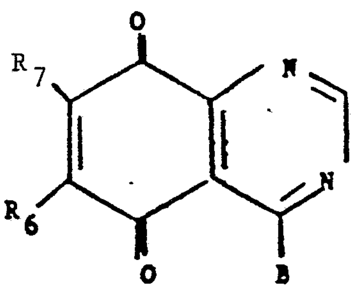

II

dans laquelle $R_7$ représente l'hydrogène

$R_6$ représente

. une amine primaire,

. un groupe amine secondaire ou tertiaire de 1 à 3 atomes de carbone,

. une pipéridine,

. une pyrrolidine,

. une diamine cyclique de 4 atomes de carbone de formule

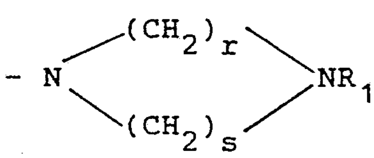

r et s étant des nombres entiers tels que le couple (r,s) représente (2,2) ou bien (r,s) représente (1,3), (3,1) ou (0,4),

dans laquelle $R_1$ représente un atome d'hydrogène ou un groupe alkyle de 1 à 3 atomes de carbone, notamment la pipérazine,

. une amine cyclique de formule

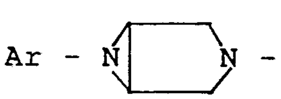

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,

* soit sur au moins deux équivalents d'aziridine de formule

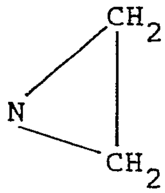

ou sur au moins deux équivalents d'une aziridine mono, bi, tri ou tétrasubstituée par des groupes alkyle, identiques ou différents, de 1 à 4 atomes de carbone,
pour obtenir un monomère selon la revendication 2, dans la formule duquel $R_6$ et $R_7$ représentent simultanément un aziridine de formule

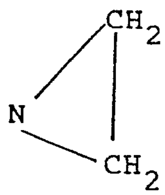

ou respectivement la même aziridine mono, bi, tri ou tétrasubstituées par des groupes alkyle, identiques ou différents, de 1 à 4 atomes de carbone.

14. Procédé, selon la revendication 12, de préparation des composés selon la revendication 3, caractérisé en ce que on oxyde à l'aide d'un oxydant, notamment $KMnO_4$ le composé de formule 1

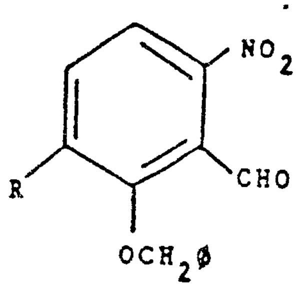

1

dans laquelle
- Ø représente un cycle benzénique,
- R représente un groupe alcoxy de 1 à 5 atomes de carbone,
pour obtenir un composé de formule 2

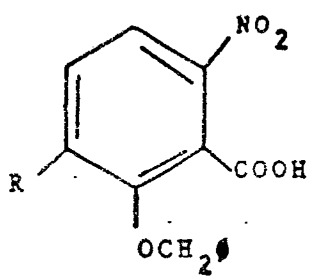

2

- on réduit le groupe $NO_2$ du composé précédent en groupe $NH_2$, notamment à l'aide de $FeSO_4$,
pour obtenir un composé de formule 3

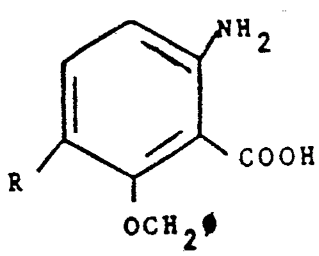

3

- on condense le composé précédent avec la triazine symétrique pour obtenir un composé de formule 4

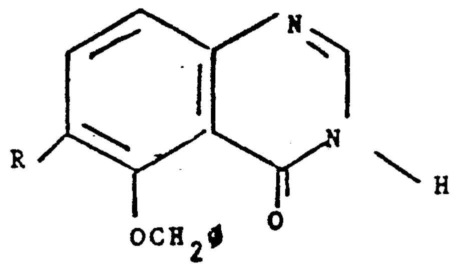

4

- on chlore le composé précédent, notamment à l'aide d'oxychlorure de phosphore, pour obtenir un composé de formule 5

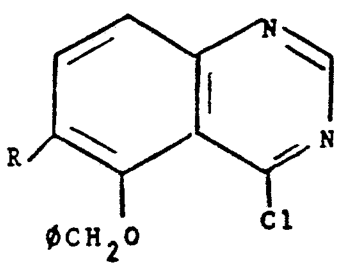

5

- on condense au moins deux molécules de composé de formule 5 sur une molécule de formule HA...AH, dans laquelle

soit A...A représente

- NH - Y - NH -

. Y représentant

* une chaîne alkyle linéaire de 2 à 10 atomes de carbone de préférence de 2 à 7,

* une chaîne de formule

$-(CH_2)_m-W-(CH_2)_n-$ dans laquelle m et n sont identiques ou différents et varient de 2 à 5, et W représente soit un groupe amine de formule

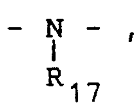

$R_{17}$ représentant un atome d'hydrogène, un groupe alkyle de 1 à 3 atomes de carbone,

. un radical choisi parmi les formules suivantes

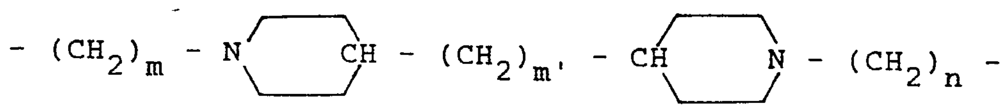

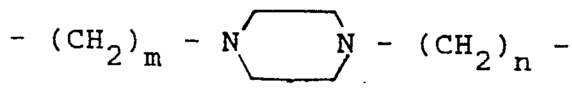

m et n identiques ou différents variant de 2 à 5, de préférence de 2 à 3,
m′ valant de 0 à 3, et de préférence égal à 0 ou 1,

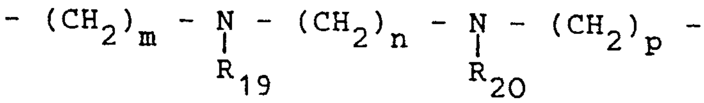

m, n, p identiques ou différents variant de 2 à 5, de préférence de 2 à 4,
$R_{19}$ et $R_{20}$ identiques ou différents représentant un atome d'hydrogène ou une chaîne alkyle de 1 à 3 atomes de carbone,

- $CH_2 - (CH_2 - O - CH_2)_q - CH_2$ -

- q variant de 1 à 5, et valant de préférence 2,

. soit A...A représente une chaîne de formule

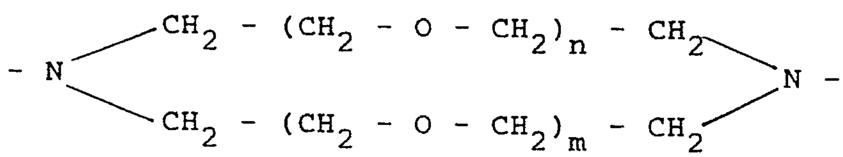

dans laquelle m et n sont identiques ou différents et varient de 2 à 5, de préférence de 2 à 3,
pour obtenir le composé de formule 11

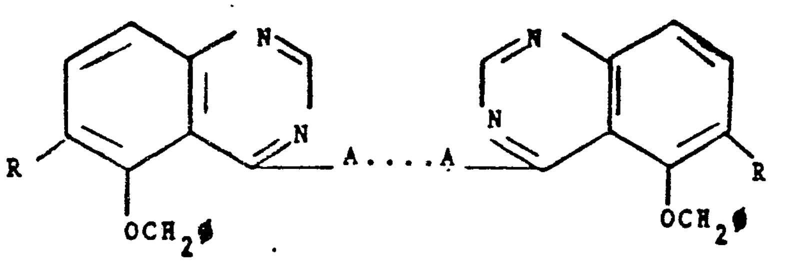

11

- puis on élimine les groupes benzyle du composé précédent pour obtenir le composé de formule 12

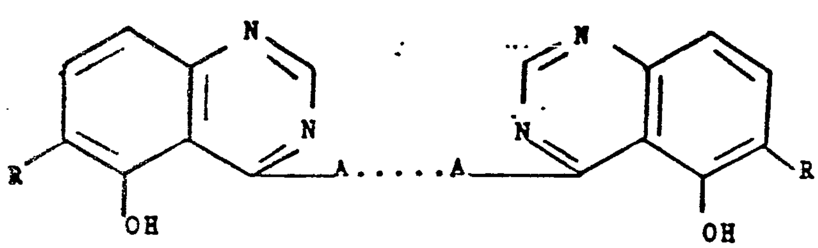

12

- on oxyde le composé précédent pour obtenir un dimère, de formule V :

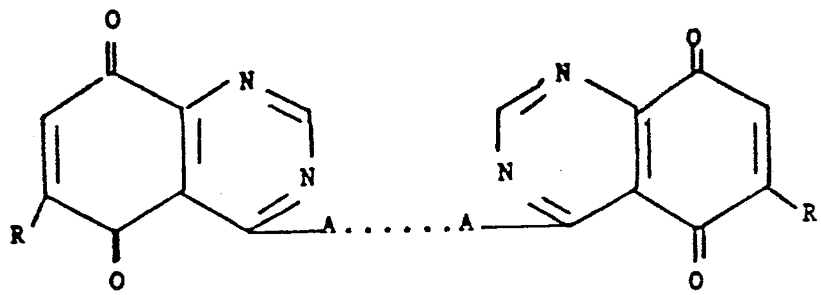

V

dans laquelle R a la signification indiquée ci-dessus,

- on condense le composé précédent dans lequel R représente un groupe alcoxy de 1 à 5 atomes de carbone, notamment un groupe méthoxy

* soit sur
  . de l'ammoniaque,
  . une amine primaire ou secondaire de 1 à 3 atomes de carbone,
  . la pipéridine,
  . la pyrrolidine,
  . une diamine cyclique de 4 atomes de carbone de formule

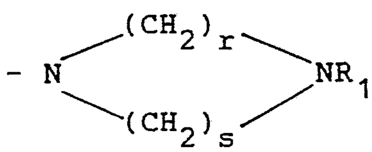

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,

  . un amine cyclique de formule

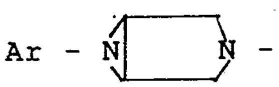

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
pour obtenir un dimère selon la revendication 6, de formule III

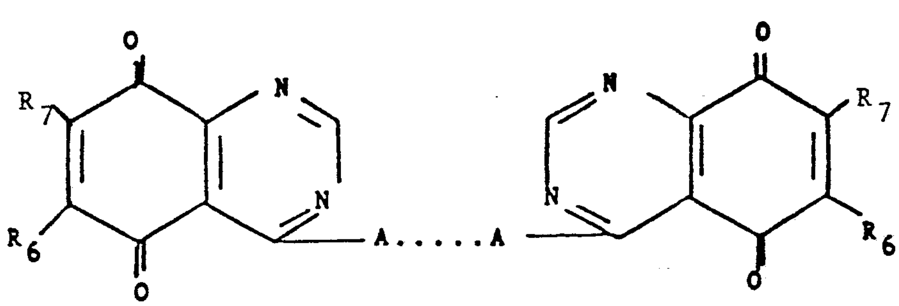

III

dans laquelle $R_7$ représente l'hydrogène
$R_6$ représente
  . une amine primaire,
  . un groupe amine secondaire ou tertiaire de 1 à 3 atomes de carbone,

. une pipéridine,
. une pyrrolidine,
. une diamine cyclique de 4 atomes de carbone de formule

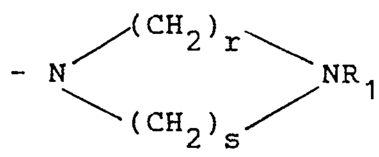

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,
. une amine cyclique de formule

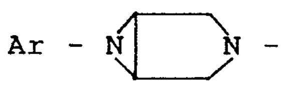

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
. soit sur au moins quatre équivalents d'une aziridine de formule

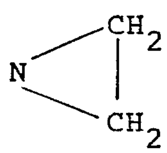

ou sur au moins quatre équivalents d'aziridine mono, bi, tri ou tétrasubstituée par des groupes alkyle, identiques ou différents, de 1 à 4 atomes de carbone,
pour obtenir un dimère selon la revendication 7, dans lequel $R_6$ et $R_7$ représentent simultanément une aziridine de formule

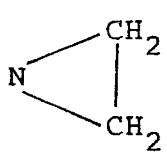

ou respectivement la même aziridine mono, bi, tri ou tétrasubstituées par des groupes alkyle, identiques ou différents, de 1 à 4 atomes de carbone,
- éventuellement on quaternarise l'azote tertiaire

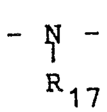

de dimères selon la revendication 7 de formule III, dans laquelle Y représente une chaîne de formule
$-(CH_2)_m-W-(CH_2)_n-$, dans laquelle m et n sont identiques ou différents et varient de 2 à 5, et W représente un groupe amine de formule

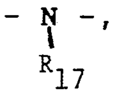, $R_{17}$

$R_{17}$ représentant un atome d'hydrogène, un groupe alkyle de 1 à 3 atomes de carbone.

15. Composés intermédiaires pour la préparation des composés de formule I, répondant aux formules suivantes :

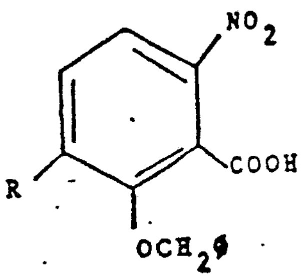

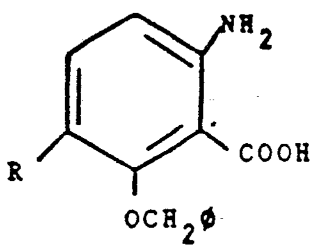

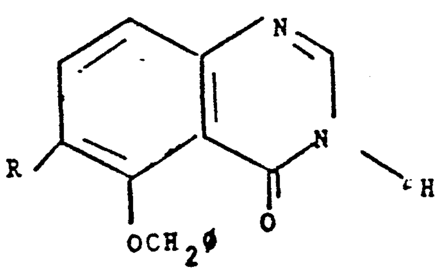

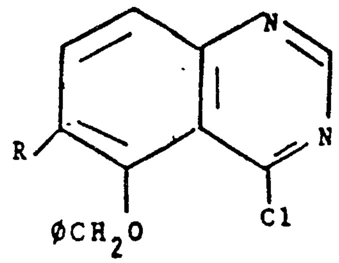

5

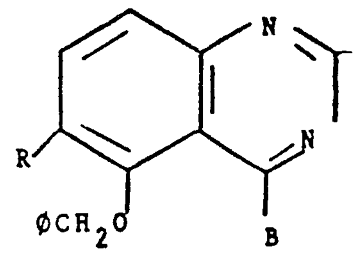

6

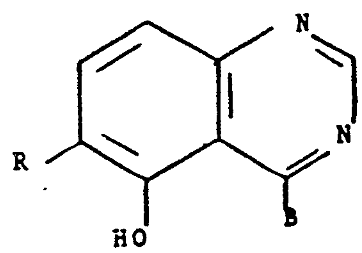

7

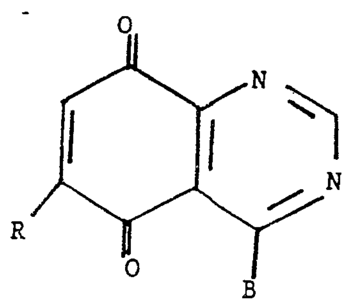

IV

dans lesquels

. R représente un groupe alcoxy de 1 à 5 atomes de carbone, de préférence $CH_3O$ ou $C_2H_5O$

. Ø représente un groupe benzénique et B a la signification indiquée à la revendication 1.

16. Composés intermédiaires pour la préparation des composés de formule I et répondant aux formules suivantes :

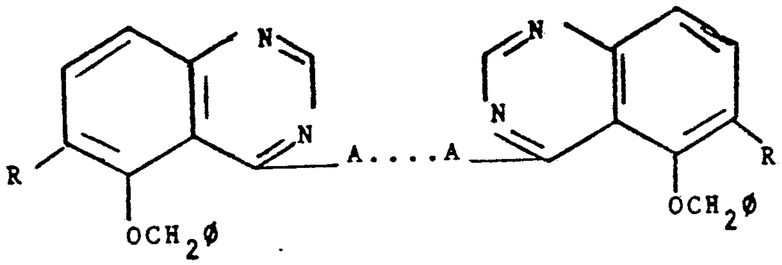

11

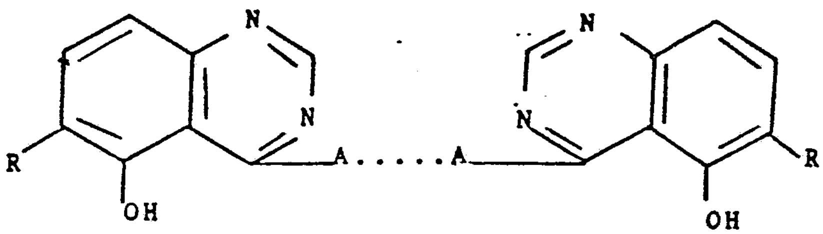

12

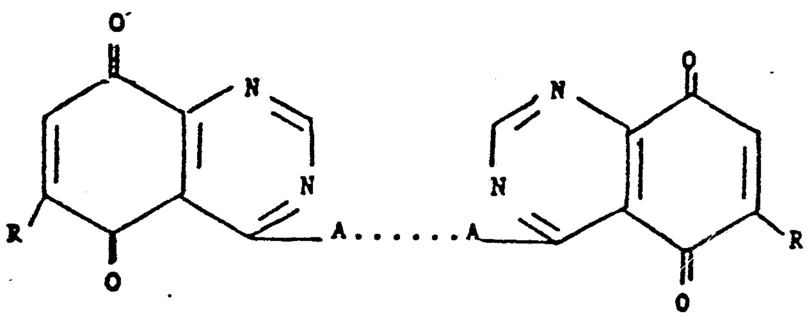

V

dans lesquels

. R représente un groupe alcoxy de 1 à 5 atomes de carbone, de préférence $CH_3O$ ou $C_2H_5O$

. Ø représente un groupe benzénique et

. A...A a la signification indiquée à la revendication 1.

17. Composés intermédiaires répondant aux formules suivantes :

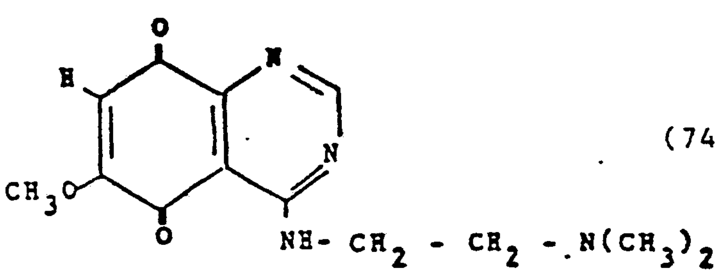

(74)

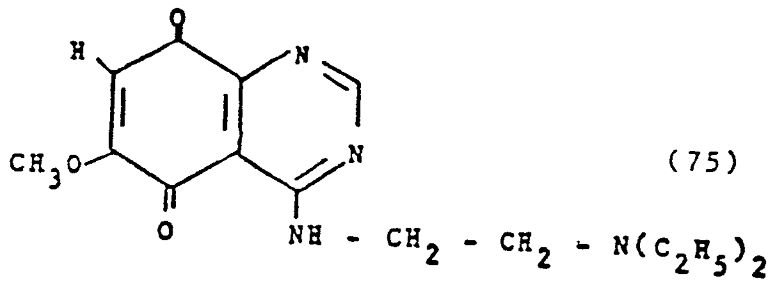

(75)

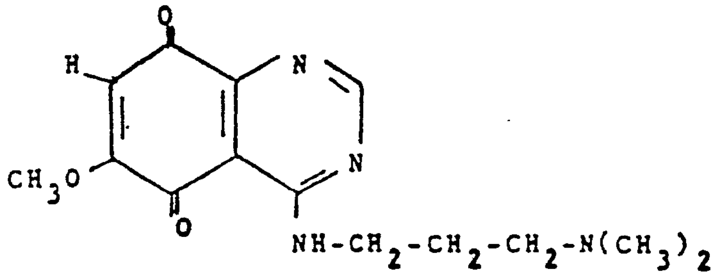

(62)

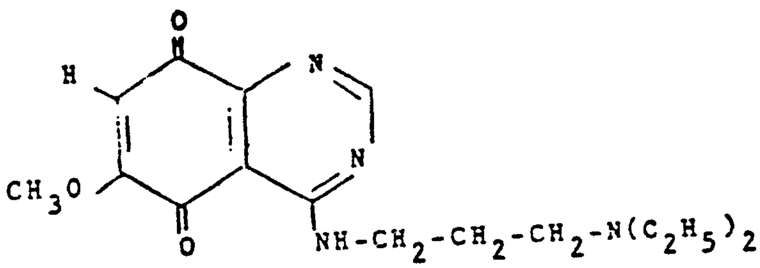

(76)

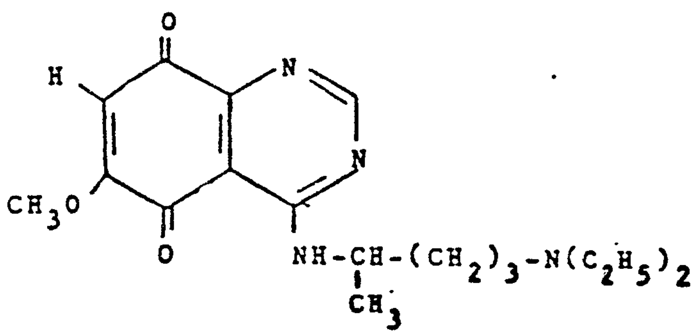

(77)

18. Les composés selon l'une quelconque des revendications 1 à 11, en tant que substance thérapeutiquement active.

19. Composition pharmaceutique à action antitumorale caractérisée en ce qu'elle comprend comme substance active l'un des composés selon l'une des revendications 1 à 11.

20. Composition pharmaceutique comprenant à titre de substance active l'un au moins des composés selon l'une des revendications 1 à 11, associé à un véhicule pharmaceutiquement acceptable, sous forme de préparations injectables comprenant d'environ 2 à environ 10 mg de substance active par dose unitaire.

21. Composition pharmaceutique comprenant à titre de substance active l'une au moins des composés selon l'une des revendications 1 à 11, associé à un véhicule pharmaceutiquement acceptable sous forme de présentations destinées à l'administration orale, contenant d'environ 10 à environ 20 mg par dose unitaire.

**Revendications pour l'Etat contractant suivant: GR**

1. Composés répondant à la formule suivante :

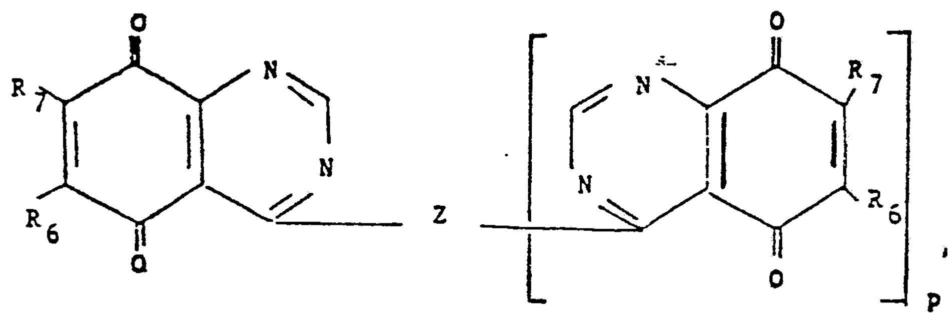

I

dans laquelle :

- p est égal à 0 ou 1,
- $R_7$ représente un atome d'hydrogène
- et $R_6$ représente :
  . un groupe amine primaire,
  . un groupe amine secondaire ou tertiaire de 1 à 3 atomes de carbone,
  . une pipéridine,
  . une pyrrolidine,
  . une diamine cyclique de 4 atomes de carbone de formule

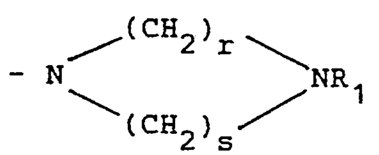

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,
. une amine cyclique de formule

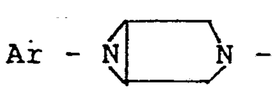

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
ou bien $R_6$ et $R_7$ sont identiques et représentent simultanément un groupe aziridine de formule

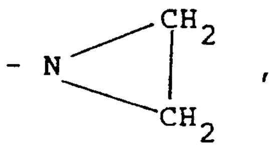

ou un groupe aziridine mono, bi, tri ou tétrasubstitué par des groupes alkyle identiques ou différents, de 1 à 4 atomes de carbone, de préférence de 1 à 2 atomes de carbone,
et lorsque p est égal à 0, Z représente B,
. B représentant un groupe de formule
- NH - $R_4$ X
dans lequel
- $R_4$ représente une chaîne alkyle, linéaire ou ramifiée, de 1 à 7 atomes de carbone, de préférence de 2 à 3 atomes de carbone,
- X représente
. un atome d'hydrogène,
. un groupe hydroxyle OH,
. un groupe oxycarbonyle de formule
- O - CO - $R_5$ dans laquelle $R_5$ est un groupe alkyle linéaire ou ramifié de 1 à 5 atomes de carbone, de préférence de 1 à 3 atomes de carbone,
. un groupe ester de formule - $COOR_8$ dans laquelle $R_8$ est un groupe alkyle linéaire ou ramifié de 1 à 3 atomes de carbone, ou un groupement benzyle,
. un radical de formule

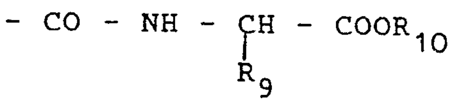

dans laquelle $R_9$ est un atome d'hydrogène ou un groupe alkyle linéaire ou ramifié, de 1 à 4 atomes de carbone, de préférence de 1 à 2 atomes de carbone, et $R_{10}$ représente un groupe alkyle identique ou non à $R_9$ comportant de préférence de 1 à 2 atomes de carbone,
. un groupe amine de formule

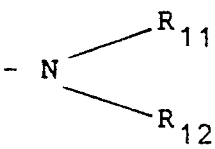

dans laquelle $R_{11}$ et $R_{12}$ sont des groupes alkyle identiques ou différents, linéaires ou ramifiés, de 1 à 3 atomes de carbone, de préférence de 1 à 2 atomes de carbone,
B représentant notamment un groupe de formule

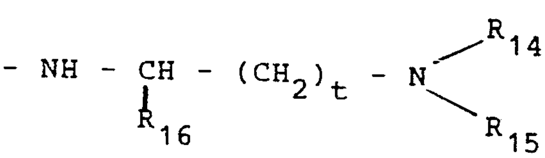

dans laquelle
t vaut de 1 à 3,
$R_{16}$ représente un atome d'hydrogène ou un groupe alkyle de 1 à 3 atomes de carbone, de préférence de 1 atome de carbone,
- $R_{14}$ et $R_{15}$ sont des groupes alkyle identiques ou différents de 1 à 3 atomes de carbone, de préférence de 1 à 2 atomes de carbone,
ou B représentant un peptide comportant de 2 à 5 acides aminés et dont la partie C-terminale se présente

sous forme d'un groupement COOH ou d'un ester de formule - COOR $_{13}$ dans laquelle $R_{13}$ est un groupe alkyle de 1 à 2 atomes de carbone, ou un groupement benzyle, et lorsque p est égal à 1, Z représente A...A,

. A...A représentant soit une chaîne de formule - NH - Y - NH -

. Y représentant

* une chaîne alkyle linéaire de 2 à 10 atomes de carbone de préférence de 2 à 7,

* une chaîne de formule

$-(CH_2)_m - W - (CH_2)_n -$, dans laquelle m et n sont identiques ou différents et varient de 2 à 5, et W représente soit un groupe amine de formule

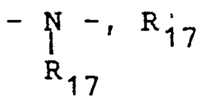

$R_{17}$ représentant un atome d'hydrogène, un groupe alkyle de 1 à 3 atomes de carbone, soit un groupe ammonium quaternaire de formule

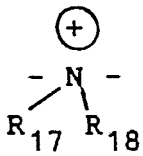

$R_{17}$ et $R_{18}$ représentant des groupes alkyle identiques ou différents de 1 à 3 atomes de carbone, lequel groupe ammonium est associé à un anion minéral ou organique physiologiquement acceptable,

* un radical choisi parmi les formules suivantes

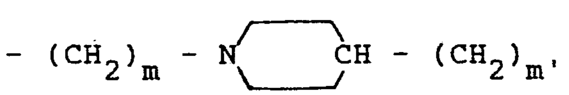

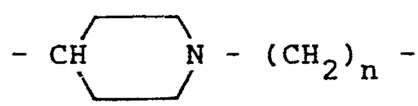

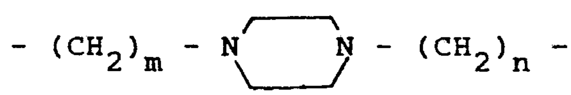

dans lesquelles m et n sont identiques ou différents, et varient de 2 à 5, de préférence de 2 à 3, m' valant de 0 à 3, et de préférence égal à 0 ou 1,

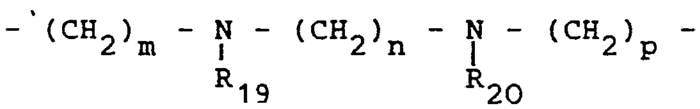

m, n, p identiques ou différents variant de 2 à 5, de préférence de 2 à 4,

$R_{19}$ et $R_{20}$ identiques ou différents représentant un atome d'hydrogène ou une chaîne alkyle de 1 à 3 atomes de carbone,

$- CH_2 - (CH_2 - O - CH_2)_q - CH_2 -$

- q variant de 1 à 5, et valant de préférence 2

* ou A...A représentant une chaîne de formule

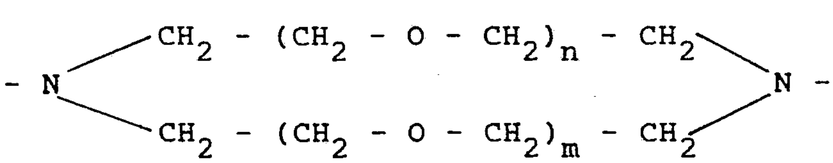

m et n, identiques ou différents, variant de 2 à 5, de préférence de 2 à 3.

2. Composés selon la revendication 1, répondant à formule II suivante :

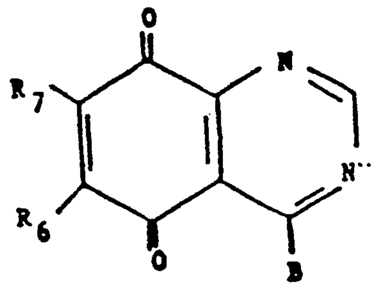

II

dans laquelle $R_6$, $R_7$ et B ont les significations indiquées à la revendication 1.

3. Composés selon les revendications 1 ou 2, caractérisés en ce que B représente un groupe de formule $NH-R_4-X$, dans lequel $R_4$ représente un chaîne alkyle, linéaire ou ramifiée de 2 à 5 atomes de carbone et X représente un groupe amine de formule

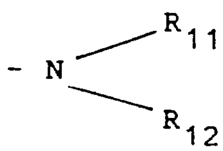

,

dans laquelle $R_{11}$ et $R_{12}$ sont des groupes alkyle identiques ou différents, linéaires ou ramifiés, de 1 à 3 atomes de carbone, de préférence de 1 à 2 atomes de carbone.

4. Composés selon les revendications 1 à 3, caractérisés en ce que
$R_6$ et $R_7$ sont identiques et représentent simultanément

. un groupe aziridine de formule :

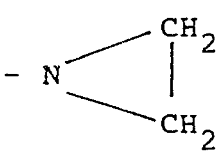

,

. ou un groupe aziridine mono, bi, tri ou tétrasubstitué par des groupes alkyle identiques ou différents, de 1 à 4 atomes de carbone, de préférence de 1 à 2 atomes de carbone.

5. Composé selon la revendication 2, répondant à la formule suivante

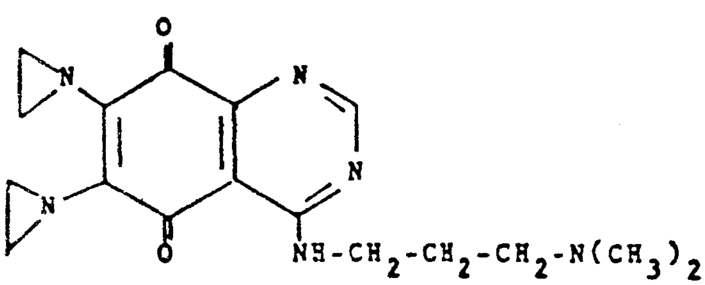

(63)

6. Composés selon la revendication 1 répondant à la formule III suivante :

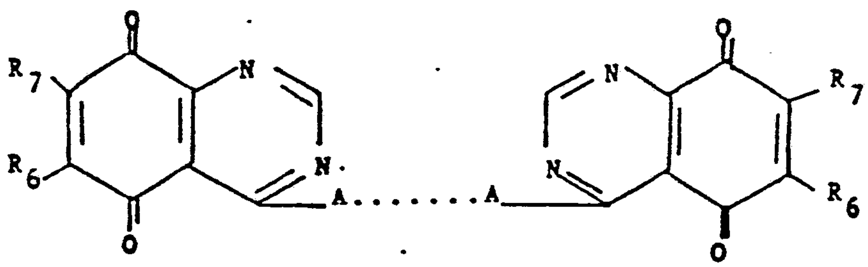

III

dans laquelle A, $R_6$ et $R_7$ ont les significations indiquées à la revendication 1.

7. Composés selon la revendication 6 caractérisés en ce que $R_6$ et $R_7$ sont identiques et représentent simultanément

. un groupe aziridine de formule

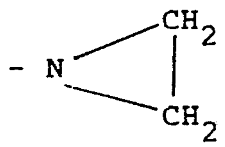

,

. ou un groupe aziridine mono, bi, tri ou tétrasubstitué par des groupes alkyle, identiques ou différents, de 1 à 4 atomes de carbone, de préférence de 1 à 2 atomes de carbone.

8. Composés selon les revendications 6 et 7 caractérisés en ce que A...A représente une chaîne moléculaire de formule - NH - Y - NH -, dans laquelle Y représente une chaîne alkyle linéaire de 2 à 10 atomes de carbone, de préférence de 2 à 7 atomes de carbone.

9. Composés selon les revendications 6 et 7, caractérisés en ce que A...A représente une chaîne moléculaire de formule - NH - Y - NH -, dans laquelle Y représente une chaîne moléculaire de formule - $(CH_2)_m$ - W - $(CH_2)_n$, dans laquelle m et n sont identiques ou différents et varient de 2 à 5, et W représente un groupe amine de formule

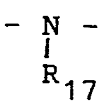

dans laquelle $R_{17}$ représente un atome d'hydrogène, ou un groupe alkyle de 1 à 3 atomes de carbone.

10. Composés selon les revendications 6 et 7, caractérisés en ce que A...A représente une chaîne de formule -NH-Y-NH-, dans laquelle Y représente $-(CH_2)-(CH_2OCH_2)_q-CH_2-$, q variant de 1 à 5, et valant, de préférence 2.

11. Composés selon la revendication 6, répondant aux formules suivantes

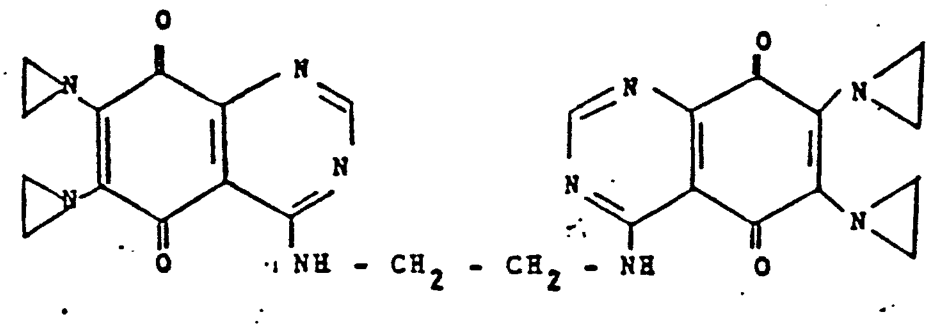

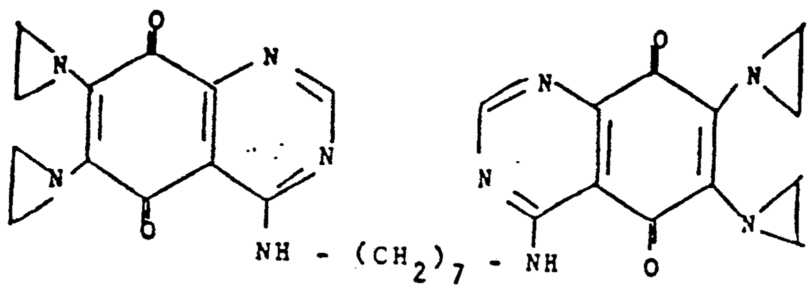

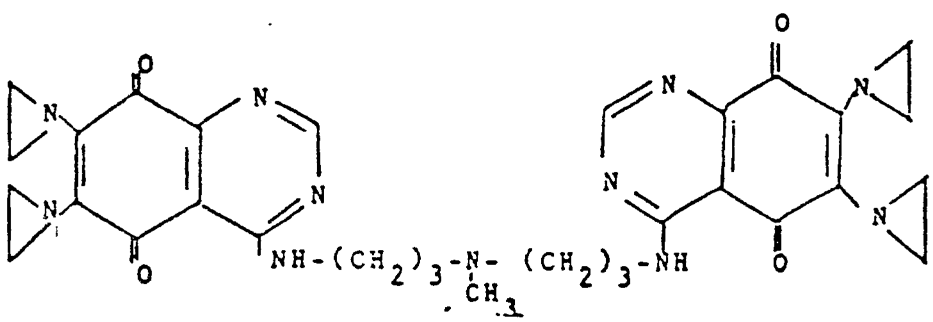

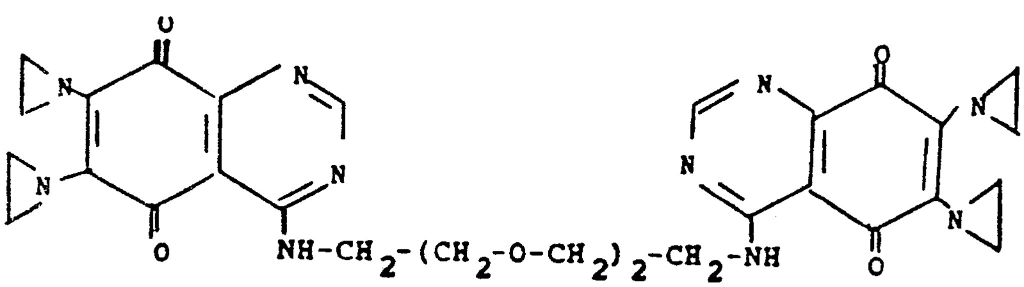

12. Procédé de préparation des composés selon les revendications 1 à 11, caractérisé en ce que on oxyde à l'aide d'un oxydant, notamment $KMnO_4$ le composé de formule 1 :

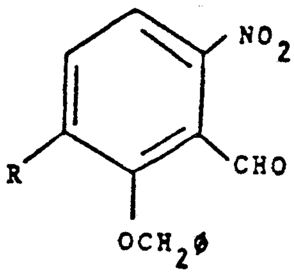

1

dans laquelle
- Ø représente un cycle benzénique,
- R représente un groupe alcoxy de 1 à 5 atomes de carbone,
pour obtenir un composé de formule 2

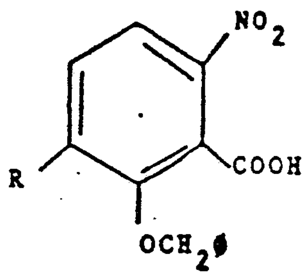

2

- on réduit le groupe $NO_2$ du composé précédent en groupe $NH_2$, notamment à l'aide de $FeSO_4$, pour obtenir un composé de formule 3

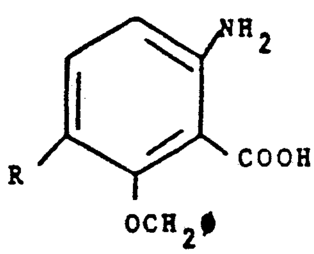

3

- on condense le composé précédent avec la triazine symétrique pour obtenir un composé de formule 4

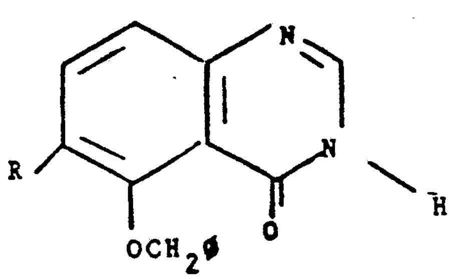

4

- on chlore le composé précédent, notamment à l'aide d'oxychlorure de phosphore, pour obtenir un composé de formule 5

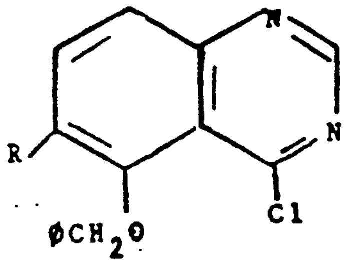

5

et dans le cas où le composé final souhaité est un monomère de formule II, selon la revendication 2,
- on condense le composé de formule 5 sur le composé de formule BH, dans laquelle
. B représente un groupe de formule
- NH - $R_4$ X
dans laquelle
- $R_4$ représente une chaîne alkyle linéaire ou ramifiée de 1 à 7 atomes de carbone, de préférence de 2 à 3,
- X représente
. un atome d'hydrogène,
. un groupe hydroxyle OH,
. un groupe oxycarbonyle de formule
- O - CO - $R_5$ dans laquelle $R_5$ est un groupe alkyle linéaire ou ramifié de 1 à 5 atomes de carbone, de préférence de 1 à 3 atomes de carbone,
. un groupe ester de formule - $COOR_8$ dans laquelle $R_8$ est un groupe alkyle linéaire ou ramifié de 1 à 3 atomes de carbone, ou un groupement benzyle,
. un radical de formule

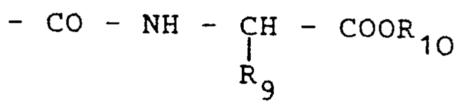

dans laquelle $R_9$ est un atome d'hydrogène ou un groupe alkyle linéaire ou ramifié, de 1 à 4 atomes de carbone, de préférence de 1 à 2 atomes de carbone, et $R_{10}$ représente un groupe alkyle identique ou non à $R_9$ comportant de préférence de 1 à 2 atomes de carbone,
. un groupe amine de formule

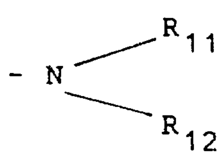

dans laquelle $R_{11}$ et $R_{12}$ sont des groupes alkyle identiques ou différents, linéaires ou ramifiés, de 1 à 3 atomes de carbone, de préférence de 1 à 2 atomes de carbone,
B représentant notamment
. un groupe de formule

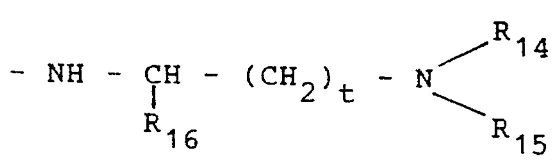

dans laquelle
t vaut de 1 à 3,
$R_{16}$ représente un atome d'hydrogène ou un groupe alkyle de 1 à 3 atomes de carbone, de préférence de 1 atome de carbone,

- $R_{14}$ et $R_{15}$ sont des groupes alkyle identiques ou différents de 1 à 3 atomes de carbone, de préférence de 1 à 2 atomes de carbone,

. ou B représentant un peptide comportant de 2 à 5 acides aminés et dont la partie C-terminale se présente sous forme d'un groupe COOH ou d'un ester de formule - $COOR_{13}$ dans laquelle $R_{13}$ est un groupe alkyle de 1 à 2 atoms de carbone, ou un groupement benzyle, pour obtenir un composé de formule 6

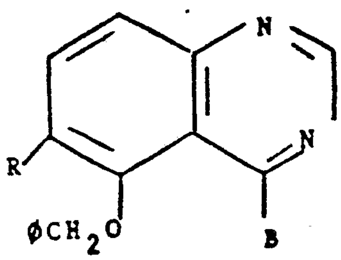

6

- on élimine le groupe benzyle du composé précédent, pour obtenir un composé de formule 7

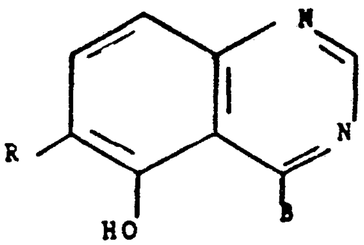

7

- on oxyde le composé précédent pour obtenir un monomère, de formule IV

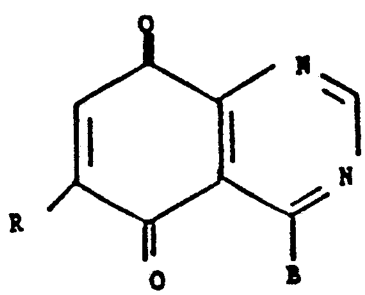

IV

dans laquelle R a la signification indiquée ci-dessus pour R,

- on condense le composé précédent dans lequel R représente un groupe alcoxy de 1 à 5 atomes de carbone, notamment un groupe méthoxy,

* soit sur

. de l'ammoniaque,

. une amine primaire ou une amine secondaire de 1 à 3 atomes de carbone,

. la pipéridine,

. la pyrrolidine,

. une diamine cyclique de 4 atomes de carbone de formule

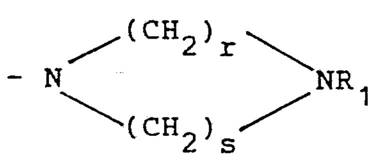

r et s étant des nombres entiers tels que le couple (r,s) représente (2,2) ou bien (r,s) représente (1,3),

(3,1) ou (0,4),
dans laquelle $R_1$ représente un atome d'hydrogène ou un groupe alkyle de 1 à 3 atomes de carbone, notamment la pipérazine,

. une amine cyclique de formule

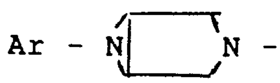

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
pour obtenir un monomère selon la revendication 2, de formule II

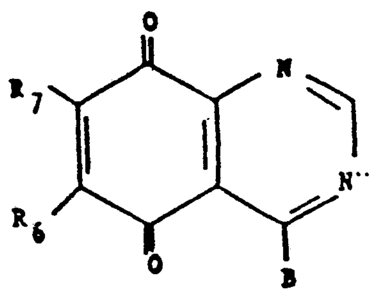

II

dans laquelle $R_6$ représente

. une amine primaire,
. un groupe amine secondaire ou tertiaire de 1 à 3 atomes de carbone,
. une pipéridine,
. une pyrrolidine,
. une diamine cyclique de 4 atomes de carbone de formule

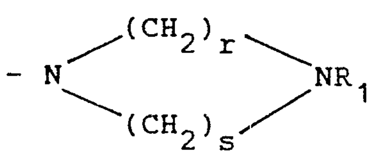

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,

. une amine cyclique de formule

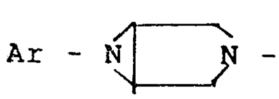

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
* soit sur au moins deux équivalents d'aziridine de formule

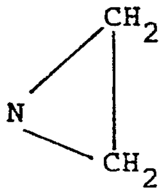

ou sur au moins deux équivalents d'une aziridine mono, bi, tri ou tétrasubstituée par des groupes alkyle, identiques ou différents, de 1 à 4 atomes de carbone, pour obtenir un monomère selon la revendication 2, dans la formule duquel $R_6$ et $R_7$ représentent simultanément une aziridine de formule

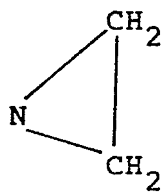

ou respectivement la même aziridine mono, bi, tri ou tétrasubstituées par des groupes alkyle, identiques ou différents, de 1 à 4 atomes de carbone,

- et dans le cas où le composé final souhaité est un dimère de formule III selon la revendication 6, on condense le composé de formule 5 sur le composé de formule HA...AH, dans laquelle soit A...A représente

- NH - Y - NH -

. Y représentant

* une chaîne alkyle linéaire de 2 à 10 atomes de carbone de préférence de 2 à 7,
* une chaîne de formule

$-(CH_2)_m - W - (CH_2)_n -$ dans laquelle m et n sont identiques ou différents et varient de 2 à 5, et W représente soit un groupe amine de formule

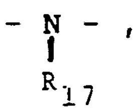

$R_{17}$ représentant un atome d'hydrogène, un groupe alkyle de 1 à 3 atomes de carbone,

. un radical choisi parmi les formules suivantes

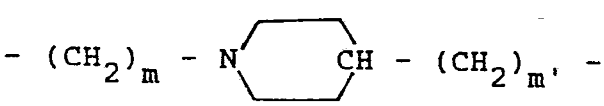
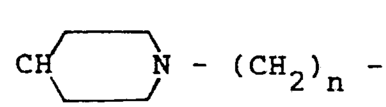
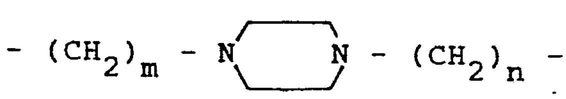

m et n identiques ou différents variant de 2 à 5, de préférence de 2 à 3,
m' valant de 0 à 3, et de préférence égal à 0 ou 1,

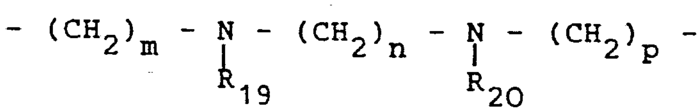

m, n, p identiques ou différents variant de 2 à 5, de préférence de 2 à 4,

$R_{19}$ et $R_{20}$ identiques ou différents représentant un atome d'hydrogène ou une chaîne alkyle de 1 à 3 atomes de carbone,

- $CH_2$ - $(CH_2$ - O - $CH_2)_q$ - $CH_2$ -

- q variant de 1 à 5, et valant de préférence 2,

. soit A...A représente une chaîne de formule

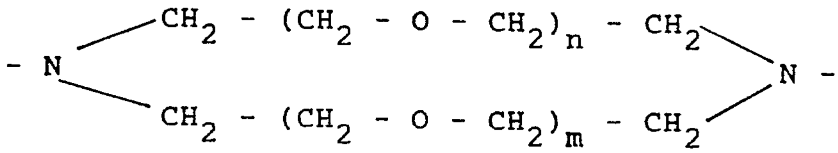

dans laquelle m et n sont identiques ou différents et varient de 2 à 5, de préférence de 2 à 3, pour obtenir le composé de formule 11

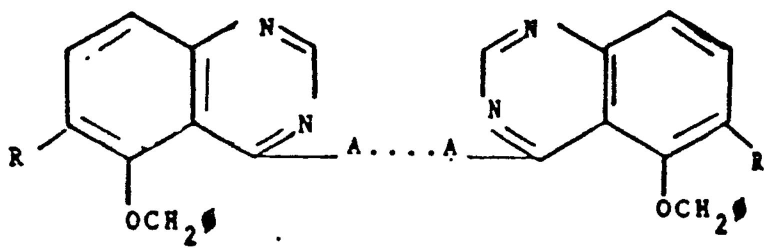

11

- puis on élimine les groupes benzyle du composé précédent pour obtenir le composé de formule 12

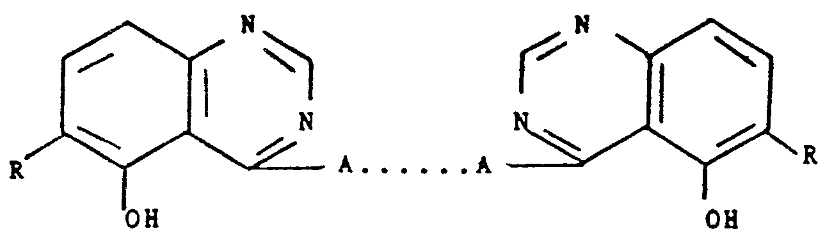

12

- on oxyde le composé précédent pour obtenir un dimère, de formule V :

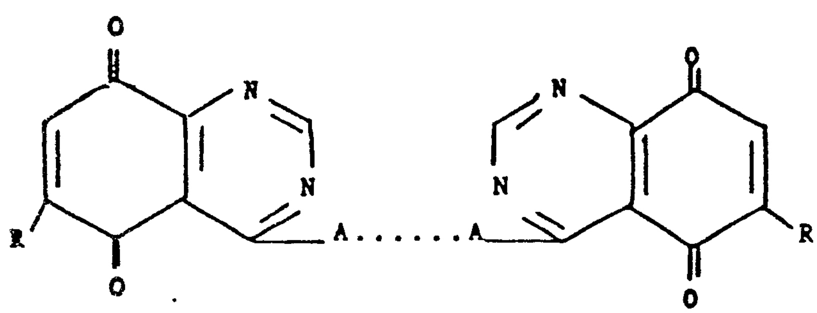

V

dans laquelle R a la signification indiquée ci-dessus,

- on condense le composé précédent dans lequel R représente un groupe alcoxy de 1 à 5 atomes de carbone, notamment un groupe méthoxy

* soit sur

. de l'ammoniaque,

. une amine primaire ou secondaire de 1 à 3 atomes de carbone,

. la pipéridine,

. la pyrrolidine,

. une diamine cyclique de 4 atomes de carbone de formule

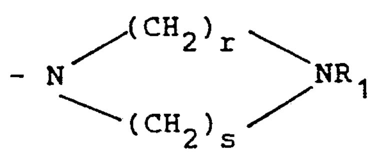

r et s étant des nombres entiers tels que le couple (r,s) représente (2,2) ou bien (r,s) représente (1.3), (3,1) ou (0,4),
dans laquelle $R_1$ représente un atome d'hydrogène ou un groupe alkyle de 1 à 3 atomes de carbone, notamment la pipérazine,

. une amine cyclique de formule

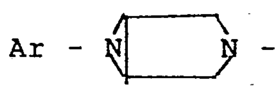

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
pour obtenir un dimère selon la revendication 7, de formule III

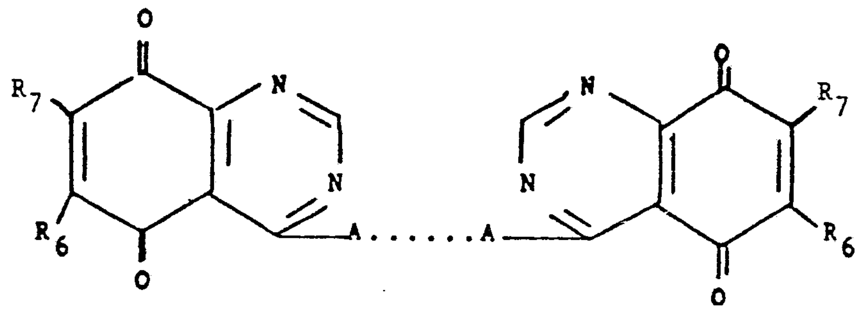

III

dans laquelle $R_7$ représente l'hydrogène
$R_6$ représente

. une amine primaire,
. un groupe amine secondaire ou tertiaire de 1 à 3 atomes de carbone,
. une pipéridine,
. une pyrrolidine,
. une diamine cyclique de 4 atomes de carbone de formule

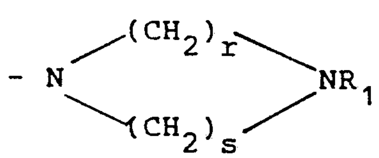

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,

. une amine cyclique de formule

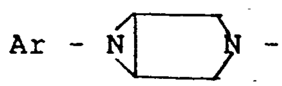

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,

* soit sur au moins quatre équivalents d'aziridine de formule

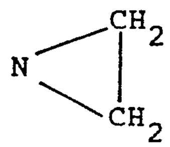

ou sur au moins quatre équivalents d'une aziridine mono, bi, tri ou tétrasubstituée par des groupes alkyle, identiques ou différents, de 1 à 4 atomes de carbone, pour obtenir un dimère selon la revendication 6, dans lequel $R_6$ et $R_7$ représentent simultanément une aziridine de formule

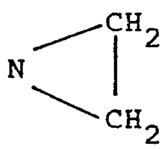

ou respectivement la même aziridine mono, bi, tri ou tétrasubstituées par des groupes alkyle, identiques ou différents, de 1 à 4 atomes de carbone,

- éventuellement on quaternarise l'azote tertiaire

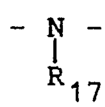

de dimères selon la revendication 6 de formule III, dans laquelle Y représente une chaîne de formule $-(CH_2)_m - W - (CH_2)_n -$, dans laquelle m et n sont identiques ou différents et varient de 2 à 5, et W représente un groupe amine de formule

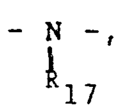

$R_{17}$ représentant un atome d'hydrogène, un groupe alkyle de 1 à 3 atomes de carbone.

13. Procédé, selon la revendication 12, de préparation des composés selon la revendication 2, caractérisé en ce que l'on oxyde à l'aide d'un oxydant, notamment $KMnO_4$ le composé de formule 1

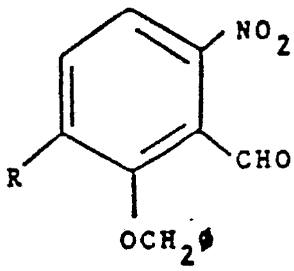

1

dans laquelle
- Ø représente un cycle benzénique,
- R représente un groupe alcoxy de 1 à 5 atomes de carbone,
pour obtenir un composé de formule 2

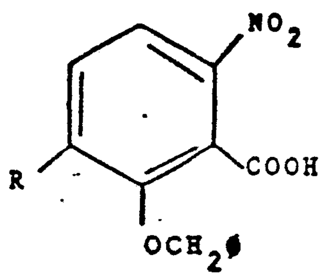

2

- on réduit le groupe $NO_2$ du composé précédent en groupe $NH_2$, notamment à l'aide de $FeSO_4$, pour obtenir un composé de formule 3

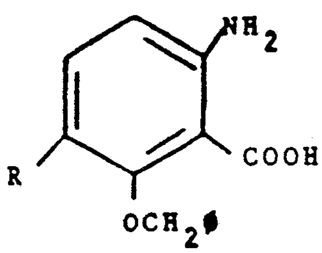

3

- on condense le composé précédent avec la triazine symétrique pour obtenir un composé de formule 4

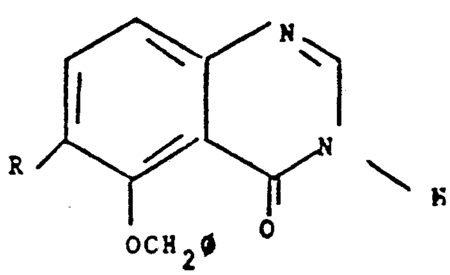

4

- on chlore le composé précédent, notamment à l'aide d'oxychlorure de phosphore, pour obtenir un composé de formule 5

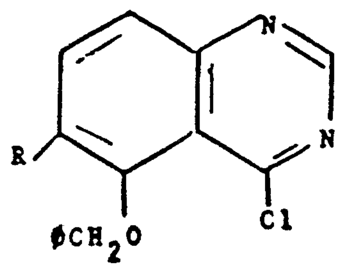

5

- on condense le composé de formule 5 sur le composé de formule BH, dans laquelle
. B représente un groupe de formule
- NH - $R_4$ X
dans laquelle
- $R_4$ représente une chaîne alkyle linéaire ou ramifiée de 1 à 7 atomes de carbone, de préférence de 2 à 3,
- X représente
. un atome d'hydrogène,
. un groupe hydroxyle OH,
. un groupe oxycarbonyle de formule
- O - CO - $R_5$ dans laquelle $R_5$ est un groupe alkyle linéaire ou ramifié de 1 à 5 atomes de carbone, de préférence de 1 à 3 atomes de carbone,
. un groupe ester de formule - $COOR_8$ dans laquelle $R_8$ est un groupe alkyle linéaire ou ramifié de 1 à 3 atomes de carbone, ou un groupement benzyle,
. un radical de formule

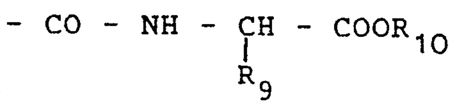

dans laquelle $R_9$ est un atome d'hydrogène ou un groupe alkyle linéaire ou ramifié, de 1 à 4 atomes de carbone, de préférence de 1 à 2 atomes de carbone, et $R_{10}$ représente un groupe alkyle identique ou non à $R_9$ comportant de préférence de 1 à 2 atomes de carbone,
. un groupe amine de formule

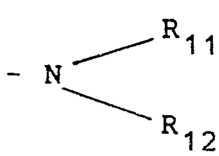

dans laquelle $R_{11}$ et $R_{12}$ sont des groupes alkyle identiques ou différents, linéaires ou ramifiés, de 1 à 3 atomes de carbone, de préférence de 1 à 2 atomes de carbone,
B représentant notamment
. un groupe de formule

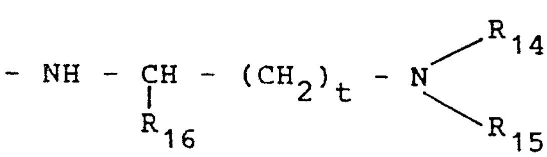

dans laquelle
t vaut de 1 à 3,
$R_{16}$ représente un atome d'hydrogène ou un groupe alkyle de 1 à 3 atomes de carbone, de préférence de 1 atome de carbone,
- $R_{14}$ et $R_{15}$ sont des groupes alkyle identiques ou différents de 1 à 3 atomes de carbone, de préférence

de 1 à 2 atomes de carbone,

. ou B représentant un peptide comportant de 2 à 5 acides aminés et dont la partie C-terminale se présente sous forme d'un ester de formule - $COOR_{13}$ dans laquelle $R_{13}$ est un groupe alkyle de 1 à 2 atomes de carbone, ou un groupement benzyle,

pour obtenir un composé de formule 6

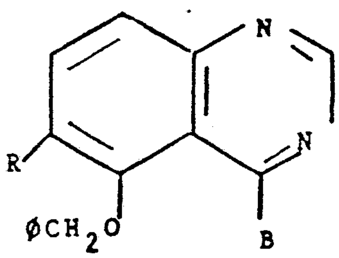

6

- on élimine le groupe benzyle du composé précédent, pour obtenir un composé de formule 7

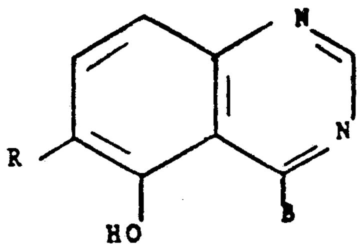

7

- on oxyde le composé précédent pour obtenir un monomère, de formule IV

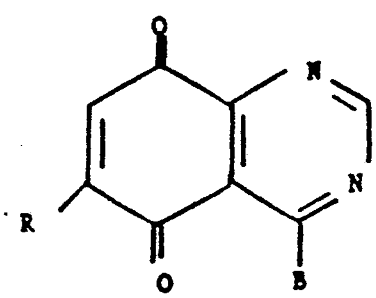

IV

dans laquelle R a la signification indiquée ci-dessus,

- on condense le composé précédent dans lequel R représente un groupe alcoxy de 1 à 5 atomes de carbone, notamment un groupe méthoxy,

* soit sur

. de l'ammoniaque,

. une amine primaire ou une amine secondaire de 1 à 3 atomes de carbone,

. la pipéridine,

. la pyrrolidine,

. une diamine cyclique de 4 atomes de carbone de formule

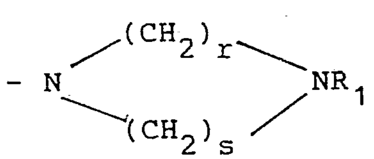

r et s étant des nombres entiers tels que le couple (r,s) représente (2,2) ou bien (r,s) représente (1,3), (3,1) ou (0,4),

dans laquelle $R_1$ représente un atome d'hydrogène ou un groupe alkyle de 1 à 3 atomes de carbone, notamment la pipérazine,

. une amine cyclique de formule

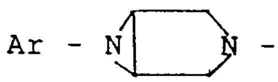

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,

pour obtenir un monomère selon la revendication 2, de formule II

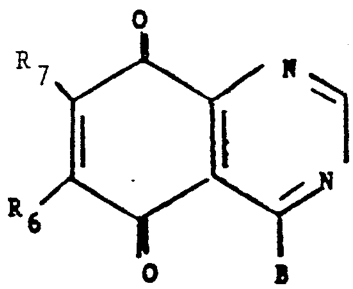

II

dans laquelle $R_7$ représente l'hydrogène

$R_6$ représente

. une amine primaire,

. un groupe amine secondaire ou tertiaire de 1 à 3 atomes de carbone,

. une pipéridine,

. une pyrrolidine,

. une diamine cyclique de 4 atomes de carbone de formule

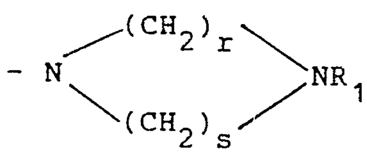

r et s étant des nombres entiers tels que le couple (r,s) représente (2,2) ou bien (r,s) représente (1,3), (3,1) ou (0,4),

dans laquelle $R_1$ représente un atome d'hydrogène ou un groupe alkyle de 1 à 3 atomes de carbone, notamment la pipérazine,

. une amine cyclique de formule

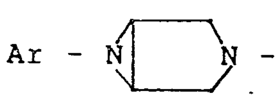

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,

* soit sur au moins deux équivalents d'aziridine de formule

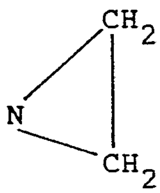

ou sur au moins deux équivalents d'une aziridine mono, bi, tri ou tétrasubstituée par des groupes alkyle, identiques ou différents, de 1 à 4 atomes de carbone,
pour obtenir un monomère selon la revendication 2, dans la formule duquel $R_6$ et $R_7$ représentent simultanément une aziridine de formule

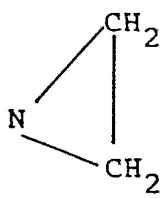

ou respectivement la même aziridine mono, bi, tri ou tétrasubstituées par des groupes alkyle, identiques ou différents, de 1 à 4 atomes de carbone.

14. Procédé, selon la revendication 12, de préparation des composés selon la revendication 3, caractérisé en ce que on oxyde à l'aide d'un oxydant, notamment $KMnO_4$ le composé de formule 1

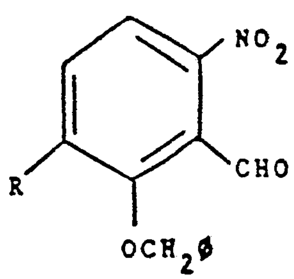

1

dans laquelle
- Ø représente un cycle benzénique,
- R représente un groupe alcoxy de 1 à 5 atomes de carbone,
pour obtenir un composé de formule 2

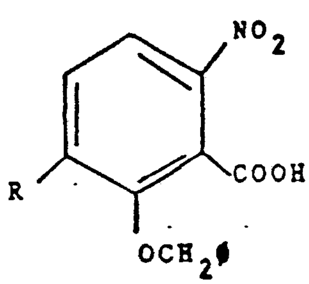

2

- on réduit le groupe $NO_2$ du composé précédent en groupe $NH_2$, notamment à l'aide de $FeSO_4$ pour obtenir un composé de formule 3

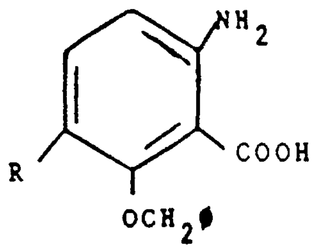

3

- on condense le composé précédent avec la triazine symétrique pour obtenir un composé de formule 4

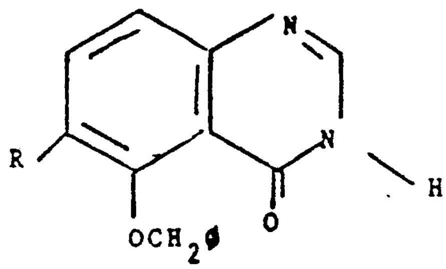

4

- on chlore le composé précédent, notamment à l'aide d'oxychlorure de phosphore, pour obtenir un composé de formule 5

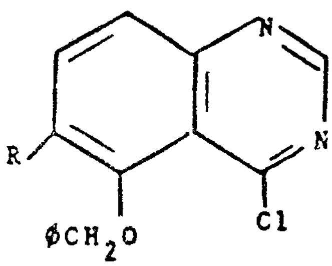

5

- on condense au moins deux molécules de composé de formule 5 sur une molécule de formule HA...AH, dans laquelle

soit A...A représente

- NH - Y - NH -

. Y représentant

* une chaîne alkyle linéaire de 2 à 10 atomes de carbone de préférence de 2 à 7,

* une chaîne de formule

$-(CH_2)_m - W - (CH_{2n} -$ dans laquelle m et n sont identiques ou différents et varient de 2 à 5, et W représente soit un groupe amine de formule

$$-\underset{R_{17}}{\underset{|}{N}}-\ ,$$

$R_{17}$ représentant un atome d'hydrogène, un groupe alkyle de 1 à 3 atomes de carbone,

. un radical choisi parmi les formules suivantes

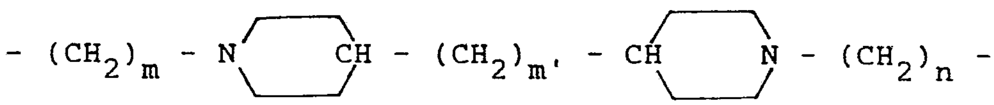

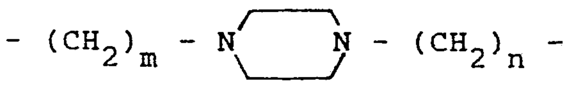

m et n identiques ou différents variant de 2 à 5, de préférence de 2 à 3, m' valant de 0 à 3, et de préférence égal à 0 ou 1,

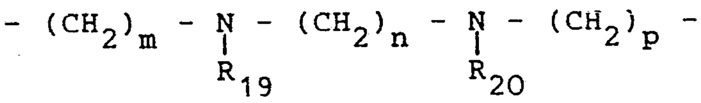

m, n, p identiques ou différents variant de 2 à 5, de préférence de 2 à 4, $R_{19}$ et $R_{20}$ identiques ou différents représentant un atome d'hydrogène ou une chaîne alkyle de 1 à 3 atomes de carbone,

$- CH_2 - (CH_2 - O - CH_2)_q - CH_2 -$

- q variant de 1 à 5, et valant de préférence 2,

. soit A...A représente une chaîne de formule

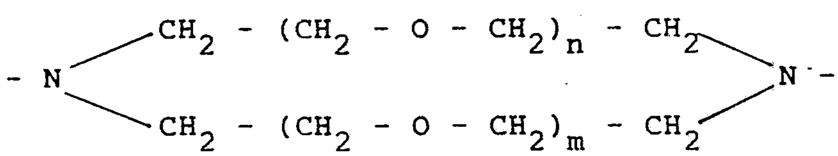

dans laquelle m et n sont identiques ou différents et varient de 2 à 5, de préférence de 2 à 3, pour obtenir le composé de formule 11

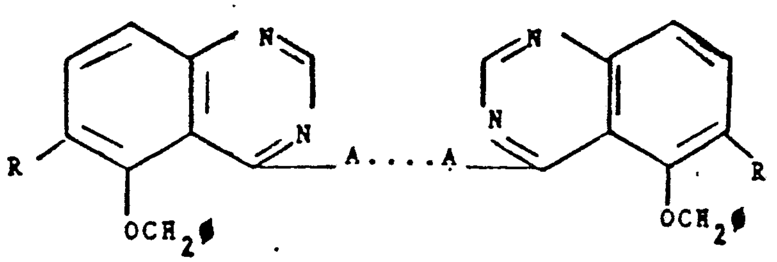

11

- puis on élimine les groupes benzyle du composé précédent pour obtenir le composé de formule 12

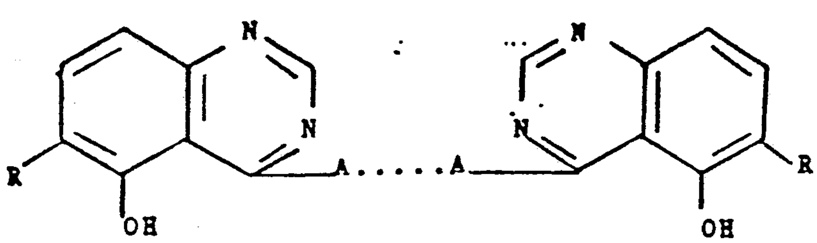

12

- on oxyde le composé précédent pour obtenir un dimère, de formule V :

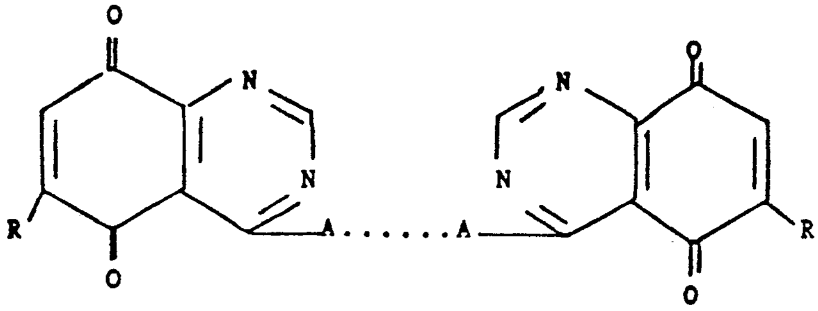

V

dans laquelle R a la signification indiquée ci-dessus,
- on condense le composé précédent dans lequel R représente un groupe alcoxy de 1 à 5 atomes de carbone, notamment un groupe méthoxy
* soit sur
  . de l'ammoniaque,
  . une amine primaire ou secondaire de 1 à 3 atomes de carbone,
  . la pipéridine,
  . la pyrrolidine,
  . une diamine cyclique de 4 atomes de carbone de formule

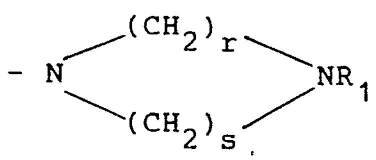

r et s étant des nombres entiers tels que le couple (r,s) représente (2,2) ou bien (r,s) représente (1,3), (3,1) ou (0,4),
dans laquelle R représente un atome d'hydrogène ou un groupe alkyle de 1 à 3 atomes de carbone, notamment la pipérazine,
  . une amine cyclique de formule

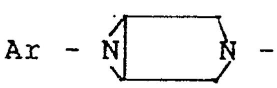

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
pour obtenir un dimère selon la revendication 6, de formule III

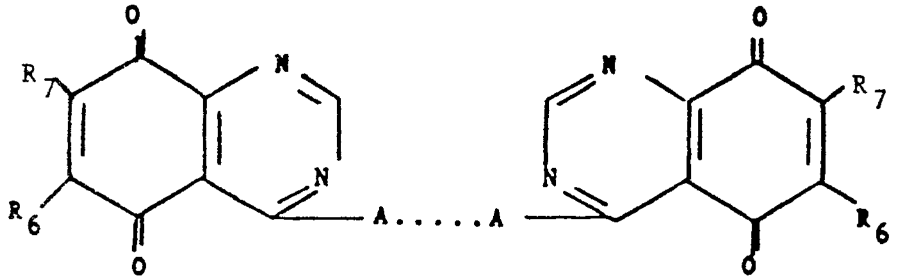

III

dans laquelle $R_7$ représente l'hydrogène
$R_6$ représente
  . une amine primaire,
  . un groupe amine secondaire ou tertiaire de 1 à 3 atomes de carbone,

. une pipéridine,
. une pyrrolidine,
. une diamine cyclique de 4 atomes de carbone de formule

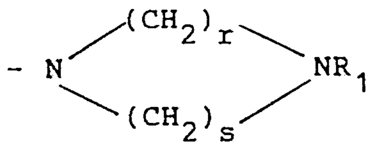

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,
. une amine cyclique de formule

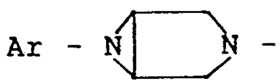

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
. soit sur au moins quatre équivalents d'une aziridine de formule

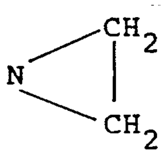

ou sur au moins quatre équivalents d'aziridine mono, bi, tri ou tétrasubstituée par des groupes alkyle, identiques ou différents, de 1 à 4 atomes de carbone, pour obtenir un dimère selon la revendication 7, dans lequel $R_6$ et $R_7$ représentent simultanément une aziridine de formule

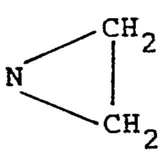

- éventuellement on quaternarise l'azote tertiaire

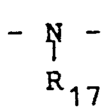

de dimères selon la revendication 7 de formule III, dans laquelle Y représente une chaîne de formule $-(CH_2)_m-W-(CH_2)_n-$, dans laquelle m et n sont identiques ou différents et varient de 2 à 5, et W représente un groupe amine de formule

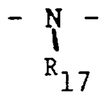, $R_{17}$ représentant un atome d'hydrogène, un groupe alkyle de 1 à 3 atomes de carbone.

15. Composés intermédiaires pour la préparation des composés de formule I, répondant aux formules suivantes :

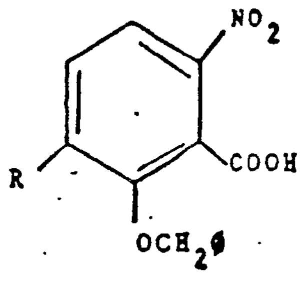

2

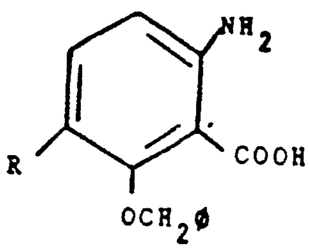

3

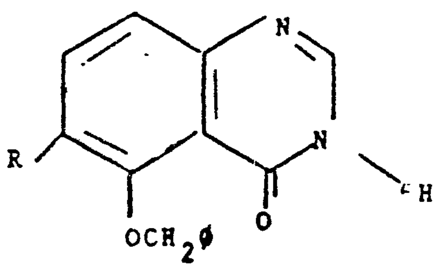

4

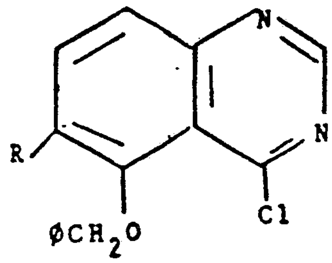

5

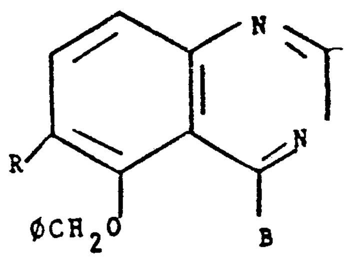

6

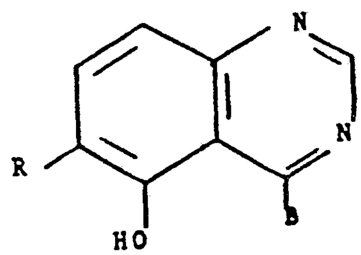

7

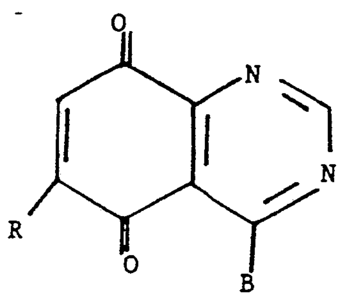

IV

dans lesquels

. R représente un groupe alcoxy de 1 à 5 atomes de carbone, de préférence $CH_3O$ ou $C_2H_5O$

. Ø représente un groupe benzénique et B a la signification indiquée à la revendication 1.

16. Composés intermédiaires pour la préparation des composés de formule I et répondant aux formules suivantes :

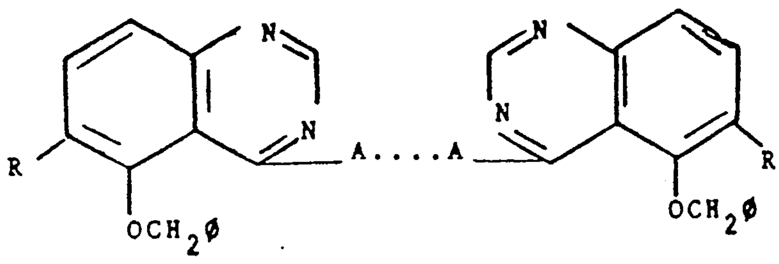

11

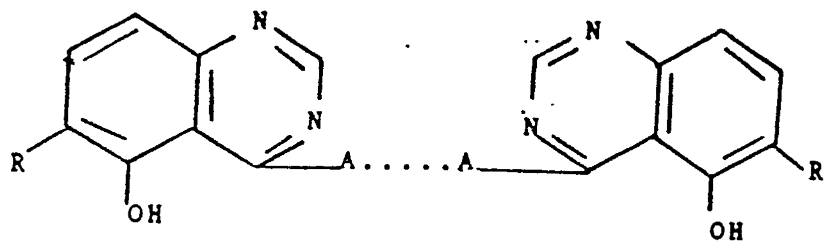

12

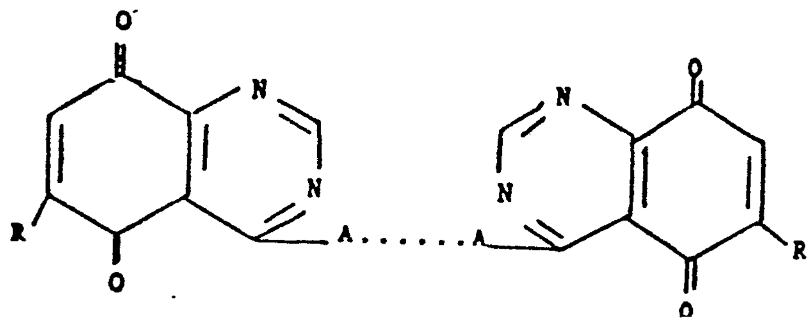

V

dans lesquels

. R représente un groupe alcoxy de 1 à 5 atomes de carbone, de préférence $CH_3O$ ou $C_2H_5O$

. Ø représente un groupe benzénique et

. A...A a la signification indiquée à la revendication 1.

17. Composés intermédiaires répondant aux formules suivantes :

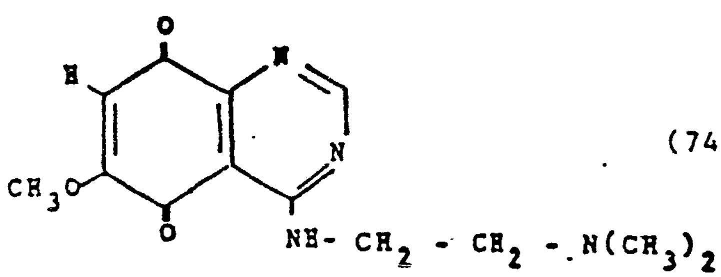

(74)

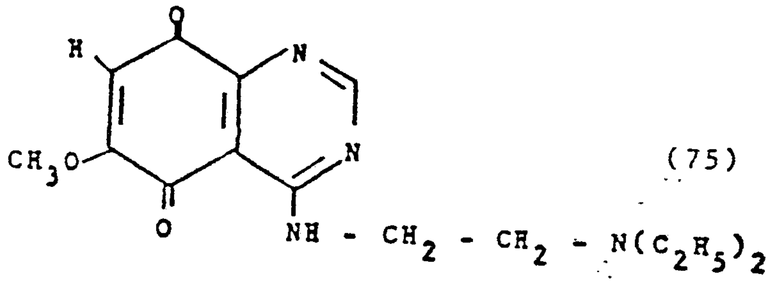

(75)

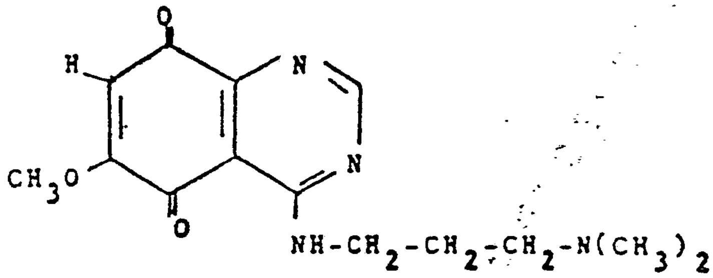

(62)

(76)

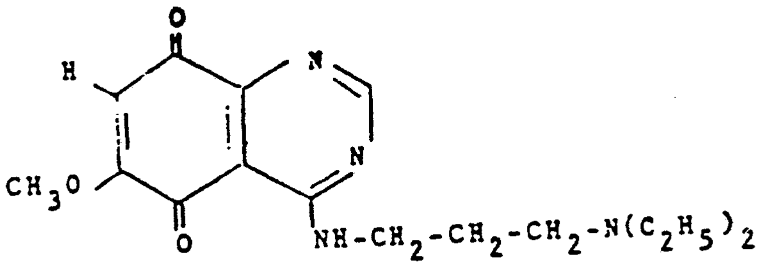

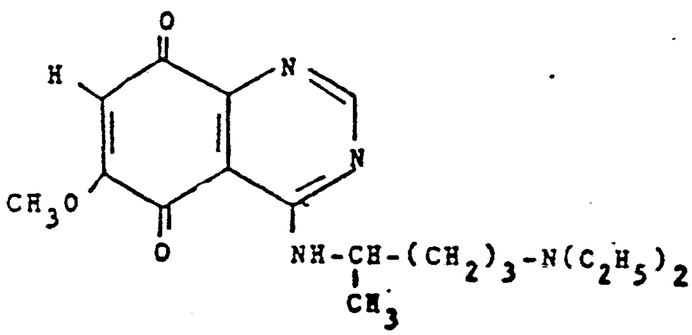

(77)

**Revendications pour l'Etat contractant suivant: ES**

1. Procédé de préparation des composés de formule I :

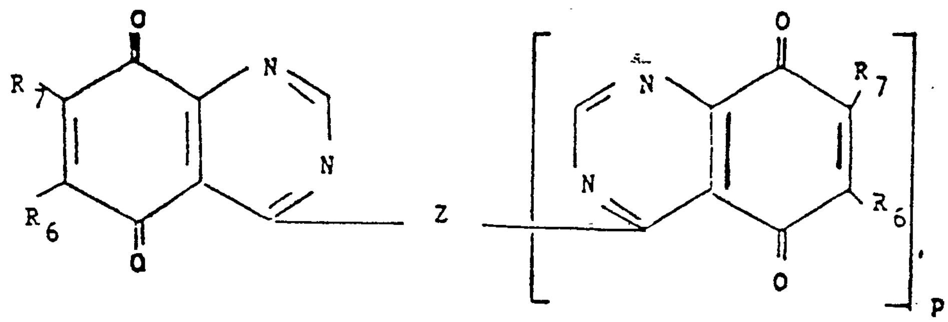

I

dans laquelle :

- p est égal à 0 ou 1,
- $R_7$ représente un atome d'hydrogène
- et $R_6$ représente :
  . un groupe amine primaire,
  . un groupe amine secondaire ou tertiaire de 1 à 3 atomes de carbone,
  . une pipéridine,
  . une pyrrolidine,
  . une diamine cyclique de 4 atomes de carbone de formule

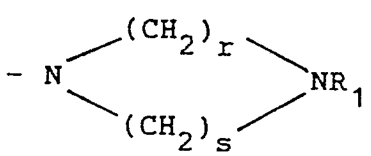

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,

. une amine cyclique de formule

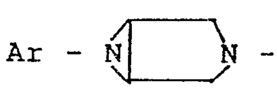

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
ou bien $R_6$ et $R_7$ sont identiques et représentent simultanément un groupe aziridine de formule

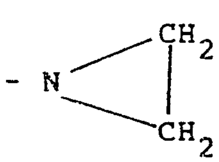

,

ou un groupe aziridine mono, bi, tri ou tétrasubstitué par des groupes alkyle identiques ou différents, de 1 à 4 atomes de carbone, de préférence de 1 à 2 atomes de carbone,
et lorsque p est égal à 0, Z représente B,
. B représentant un groupe de formule

- NH - $R_4$ X

dans lequel

- $R_4$ représente une chaîne alkyle, linéaire ou ramifiée, de 1 à 7 atomes de carbone, de préférence de 2 à 3 atomes de carbone,

- X représente

. un atome d'hydrogène,

. un groupe hydroxyle OH,

. un groupe oxycarbonyle de formule

- O- CO - $R_5$ dans laquelle $R_5$ est un groupe alkyle linéaire ou ramifié de 1 à 5 atomes de carbone, de préférence de 1 à 3 atomes de carbone,

. un groupe ester de formule - $COOR_8$ dans laquelle $R_8$ est un groupe alkyle linéaire ou ramifié de 1 à 3 atomes de carbone, ou un groupement benzyle,

. un radical de formule

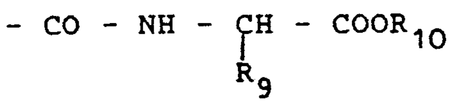

dans laquelle $R_9$ est un atome d'hydrogène ou un groupe alkyle linéaire ou ramifié, de 1 à 4 atomes de carbone, de préférence de 1 à 2 atomes de carbone, et $R_{10}$ représente un groupe alkyle identique ou non à $R_9$ comportant de préférence de 1 à 2 atomes de carbone,

. un groupe amine de formule

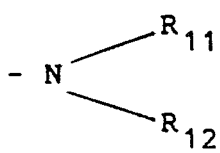

dans laquelle $R_{11}$ et $R_{12}$ sont des groupes alkyle identiques ou différents, linéaires ou ramifiés, de 1 à 3 atomes de carbone, de préférence de 1 à 2 atomes de carbone,

B représentant notamment un groupe de formule

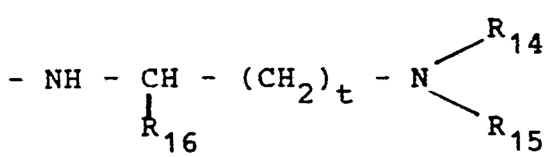

dans laquelle

t vaut de 1 à 3,

$R_{16}$ représente un atome d'hydrogène ou un groupe alkyle de 1 à 3 atomes de carbone, de préférence de 1 atome de carbone,

- $R_{14}$ et $R_{15}$ sont des groupes alkyle identiques ou différents de 1 à 3 atomes de carbone, de préférence de 1 à 2 atomes de carbone,

ou B représentant un peptide comportant de 2 à 5 acides aminés et dont la partie C-terminale se présente sous forme d'un groupement COOH ou d'un ester de formule - $COOR_{13}$ dans laquelle $R_{13}$ est un groupe alkyle de 1 à 2 atomes de carbone, ou un groupement benzyle, et lorsque p est égal à 1, Z représente A...A,

. A...A représentant soit une chaîne de formule - NH - Y- NH -

. Y représentant

* une chaîne alkyle linéaire de 2 à 10 atomes de carbone de préférence de 2 à 7,

* une chaîne de formule

- $(CH_2)_m$ - W - $(CH_2)_n$ -, dans laquelle m et n sont identiques ou différents et varient de 2 à 5, et W représente soit un groupe amine de formule

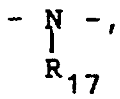

$R_{17}$ représentant un atome d'hydrogène, un groupe alkyle de 1 à 3 atomes de carbone, soit un groupe ammonium quaternaire de formule

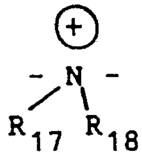

$R_{17}$ et $R_{18}$ représentant des groupes alkyle identiques ou différents de 1 à 3 atomes de carbone, lequel groupe ammonium est associé à un anion minéral ou organique physiologiquement acceptable,
* un radical choisi parmi les formules suivantes

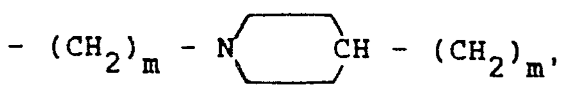
,
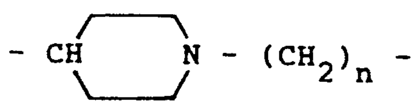

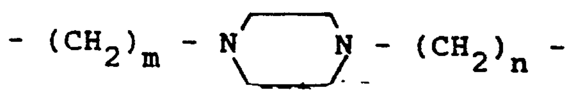

dans lesquelles m et n sont identiques ou différents, et varient de 2 à 5, de préférence de 2 à 3,
m' valant de 0 à 3, et de préférence égal à 0 ou 1,

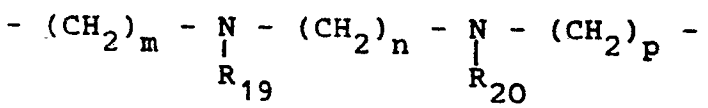

m, n, p identiques ou différents variant de 2 à 5, de préférence de 2 à 4,
$R_{19}$ et $R_{20}$ identiques ou différents représentant un atome d'hydrogène ou une chaîne alkyle de 1 à 3 atomes de carbone,

$- CH_2 - (CH_2 - O - CH_2)_q - CH_2 -$

- q variant de 1 à 5, et valant de préférence 2
* ou A...A représentant une chaîne de formule

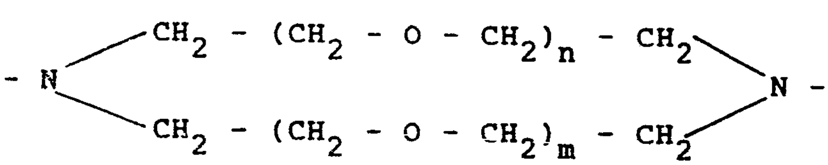

m et n, identiques ou différents, variant de 2 à 5, de préférence de 2 à 3,
caractérisé en ce qu'on oxyde à l'aide d'un oxydant notamment $KMnO_4$ le composé de formule 1 :

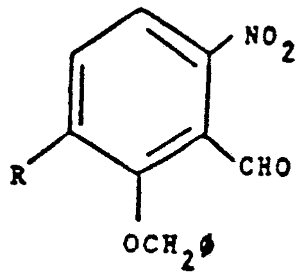

1

dans laquelle
- Ø représente un cycle benzénique,
- R représente un groupe alcoxy de 1 à 5 atomes de carbone,
pour obtenir un composé de formule 2

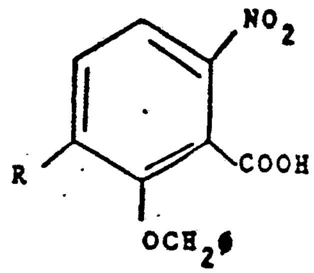

2

- on réduit le groupe $NO_2$ du composé précédent en groupe $NH_2$, notamment à l'aide de $FeSO_4$, pour obtenir un composé de formule 3

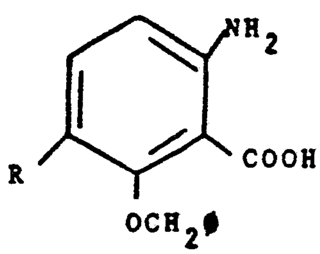

3

- on condense le composé précédent avec la triazine symétrique pour obtenir un composé de formule 4

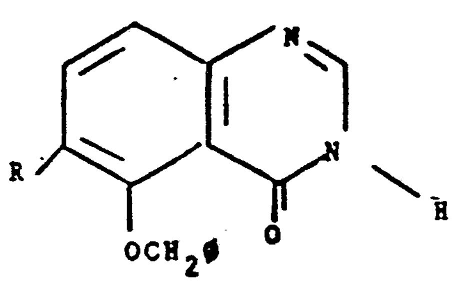

4

- on chlore le composé précédent, notamment à l'aide d'oxychlorure de phosphore, pour obtenir un composé de formule 5

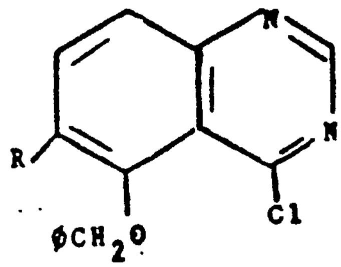

5

et dans le cas où le composé final souhaité est un monomère de formule II

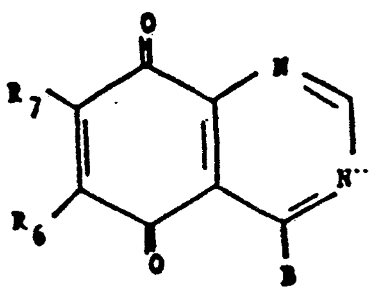

II

dans laquelle $R_6$, $R_7$ et B ont les significations indiquées ci-dessus,
- on condense le composé de formule 5 sur le composé de formule BH, dans laquelle
. B a la signification indiquée ci-dessus,
pour obtenir un composé de formule 6

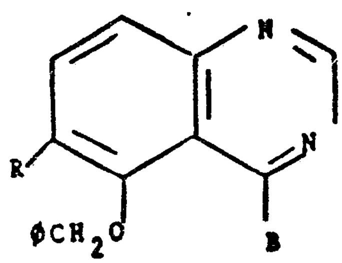

6

- on élimine le groupe benzyle du composé précédent, pour obtenir un composé de formule 7

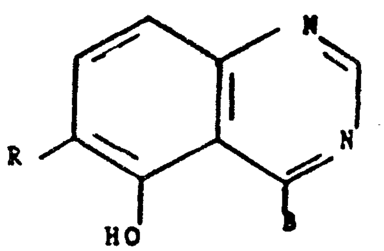

7

- on oxyde le composé précédent pour obtenir un monomère, de formule IV

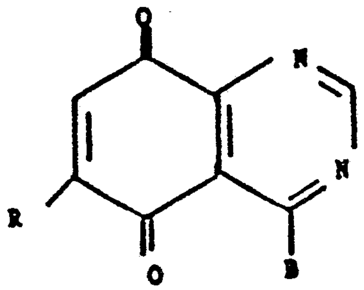

IV

dans laquelle R a la signification indiquée ci-dessus,

- on condense le composé précédent dans lequel R représente un groupe alcoxy de 1 à 5 atomes de carbone, notamment un groupe méthoxy,

* soit sur
  . de l'ammoniaque,
  . une amine primaire ou une amine secondaire de 1 à 3 atomes de carbone,
  . la pipéridine,
  . la pyrrolidine,
  . une diamine cyclique de 4 atomes de carbone de formule

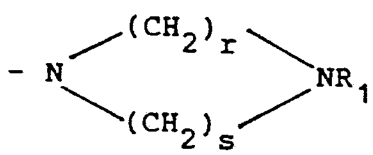

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,
  . une amine cyclique de formule

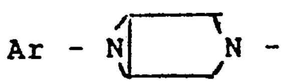

dans laquelle Ar représente un cycle benzénique non substitué ou substitué par un groupe alkyle de 1 à 2 atomes de carbone, avantaeusement un méthyle, et/ou substitué par 1 ou 2 atomes d'halogène, notamment le chlore,
pour obtenir un monomère de formule II

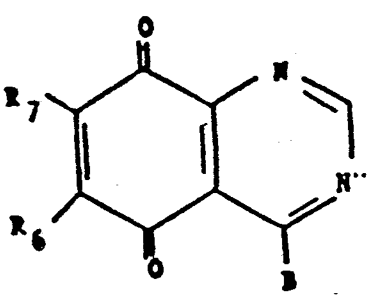

II

dans laquelle $R_7$ représente l'hydrogène
$R_6$ représente
  . une amine primaire,
  . un groupe amine secondaire ou tertiaire de 1 à 3 atomes de carbone,
  . une pipéridine,

. une pyrrolidine,

. une diamine cyclique de 4 atomes de carbone de formule

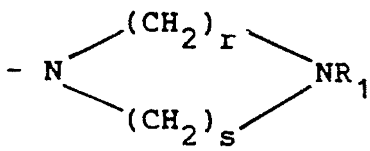

r et s étant des nombres entiers tels que le couple (r,s) représente (2,2) ou bien (r,s) représente (1,3), (3,1) ou (0,4),

dans laquelle $R_1$ représente un atome d'hydrogène ou un groupe alkyle de 1 à 3 atomes de carbone, notamment la pipérazine,

. une amine cyclique de formule

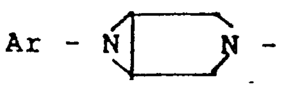

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,

* soit sur au moins deux équivalents d'aziridine de formule

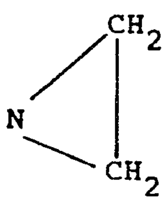

ou sur au moins deux équivalents d'une aziridine mono, bi, tri ou tétrasubstituée par des groupes alkyle, identiques ou différents, de 1 à 4 atomes de carbone, pour obtenir un monomère selon la revendication 2, dans la formule duquel $R_6$ et $R_7$ représentent simultanément une aziridine de formule

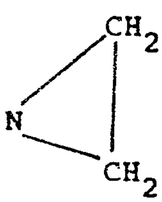

ou respectivement la même aziridine mono, bi, tri ou tétrasubstituées par des groupes alkyle, identiques ou différents, de 1 à 4 atomes de carbone,

- et dans le cas où le composé final souhaité est un dimère de formule III

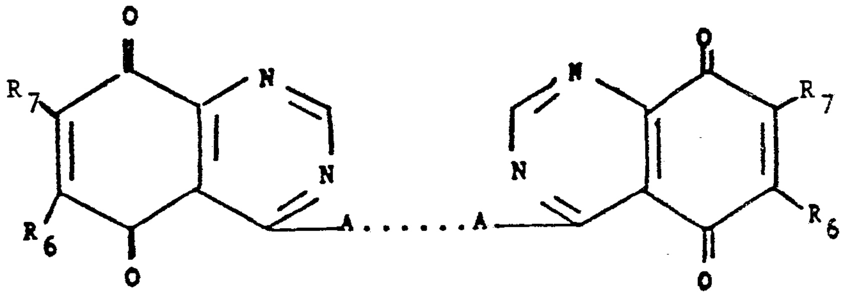

III

dans laquelle A,$R_6$ et $R_7$ ont les significations indiquées ci-dessus,

- on condense le composé de formule 5 sur le composé de formule HA...AH, dans laquelle soit A...A a la signification indiquée ci-dessus,

pour obtenir le composé de formule II

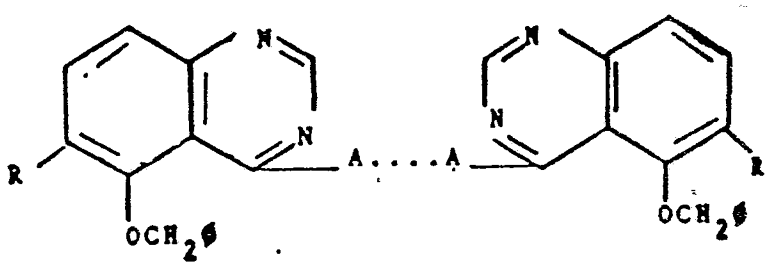

11

- puis on élimine les groupes benzyle du composé précédent pour obtenir le composé de formule 12

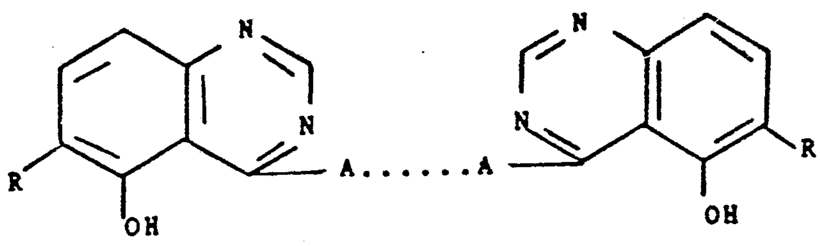

12

- on oxyde le composé précédent pour obtenir un dimère, de formule V :

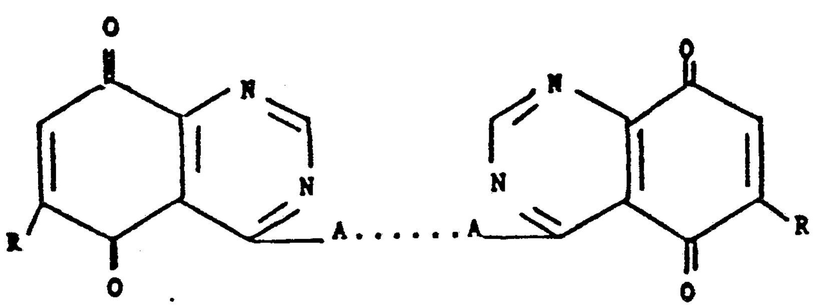

V

dans laquelle R a la signification indiquée ci-dessus,

- on condense le composé précédent dans lequel R représente un groupe alcoxy de 1 à 5 atomes de carbone, notamment un groupe méthoxy

* soit sur

. de l'ammoniaque,

. une amine primaire ou secondaire de 1 à 3 atomes de carbone,

. la pipéridine,
. la pyrrolidine,
. une diamine cyclique de 4 atomes de carbone de formule

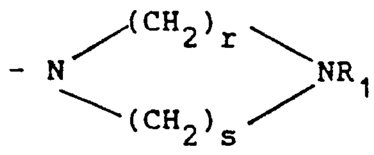

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,
. une amine cyclique de formule

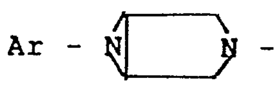

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
pour obtenir un dimère de formule III

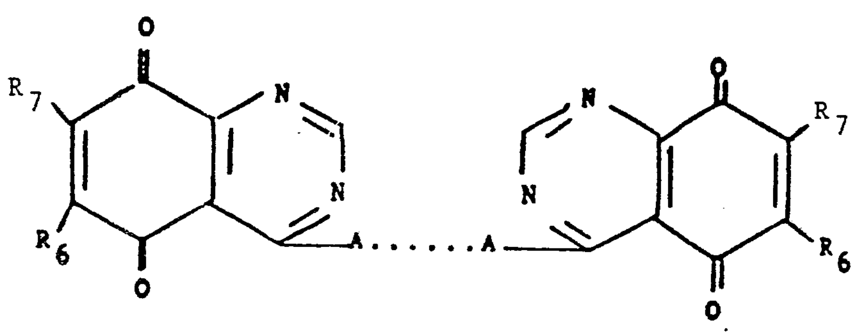
III

dans laquelle $R_7$ représente l'hydrogène
$R_6$ représente
. une amine primaire,
. un groupe amine secondaire ou tertiaire de 1 à 3 atomes de carbone,
. une pipéridine,
. une pyrrolidine,
. une diamine cyclique de 4 atomes de carbone de formule

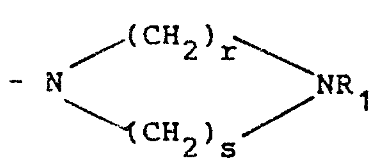

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,
. une amine cyclique de formule

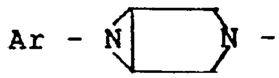

dans laquelle Ar représente un cycle benzénique non substitué ou substitué par un groupe alkyle de 1 à 2 atomes de carbone, avantageusement um méthyle, et/ou substitué par 1 ou 2 atomes d'halogène notamment le chlore,
* soit sur au moins quatre équivalents d'aziridine de formule

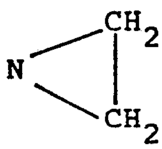

ou sur au moins quatre équivalents d'une aziridine mono, bi, tri ou tétrasubstituée par des groupes alkyle, identiques ou différents, de 1 à 4 atomes de carbone, pour obtenir un dimère de formule III, dans lequel $R_6$ et $R_7$ représentent simultanément une aziridine de formule

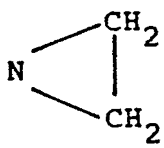

ou respectivement la même aziridine mono, bi, tri ou tétrasubstituées par des groupes alkyle, identiques ou différents, de 1 à 4 atomes de carbone, - éventuellement on quaternarise l'azote tertiaire

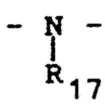

de dimères selon la revendication 6 de formule III, dans laquelle Y représente une chaîne de formule $-(CH_2)_m-W-(CH_2)_n-$, dans laquelle m et n sont identiques ou différents et varient de 2 à 5, et W représente un groupe amine de formule

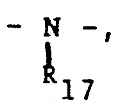

$R_{17}$ représentant un atome d'hydrogène, un groupe alkyle de 1 à 3 atomes de carbone.

2. Procédé, selon la revendication 1, de préparation des composés de formule II

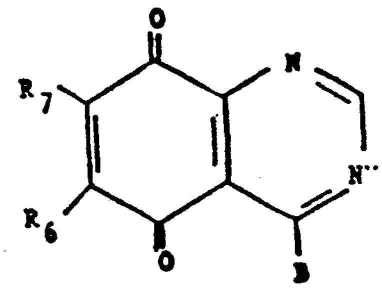

II

dans laquelle B, $R_6$ et $R_7$ ont les significations indiquées à la revendication 1, caractérisé en ce que l'on oxyde à l'aide d'un oxydant, notamment $KMnO_4$ le composé de formule 1

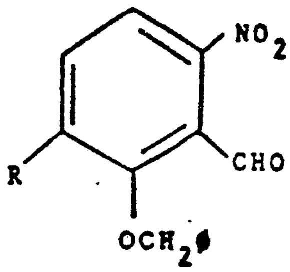

1

dans laquelle
- Ø représente un cycle benzénique,
- R représente un groupe alcoxy de 1 à 5 atomes de carbone,

pour obtenir un composé de formule 2

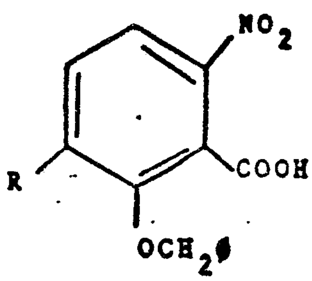

2

- on réduit le groupe $NO_2$ du composé précédent en groupe $NH_2$, notamment à l'aide de $FeSO_4$, pour obtenir un composé de formule 3

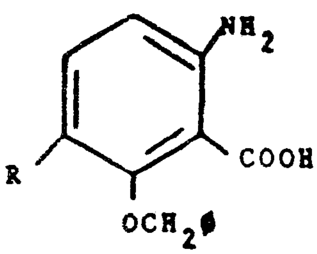

3

- on condense le composé précédent avec la triazine symétrique pour obtenir un composé de formule 4

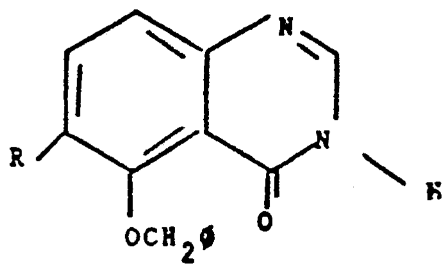

4

- on chlore le composé précédent, notamment à l'aide d'oxychlorure de phosphore, pour obtenir un composé de formule 5

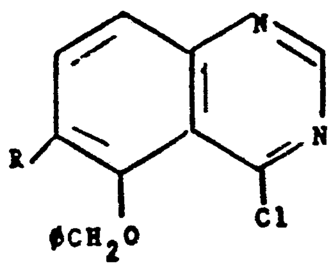

5

- on condense le composé de formule 5 sur le composé de formule BH, dans laquelle
. B représente un groupe de formule
- NH - $R_4$ X
dans laquelle
- $R_4$ représente une chaîne alkyle linéaire ou ramifiée de 1 à 7 atomes de carbone, de préférence de 2 à 3,
- X représente
. un atome d'hydrogène,
. un groupe hydroxyle OH,
. un groupe oxycarbonyle de formule
- O - CO - $R_5$ dans laquelle $R_5$ est un groupe alkyle linéaire ou ramifié de 1 à 5 atomes de carbone, de préférence de 1 à 3 atomes de carbone,
. un groupe ester de formule - $COOR_8$ dans laquelle $R_8$ est un groupe alkyle linéaire ou ramifié de 1 à 3 atomes de carbone, ou un groupement benzyle,
. un radical de formule

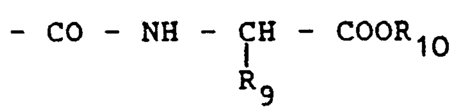

dans laquelle $R_9$ est un atome d'hydrogène ou un groupe alkyle linéaire ou ramifié, de 1 à 4 atomes de carbone, de préférence de 1 à 2 atomes de carbone, et $R_{10}$ représente un groupe alkyle identique ou non à $R_9$ comportant de préférence de 1 à 2 atomes de carbone,
. un groupe amine de formule

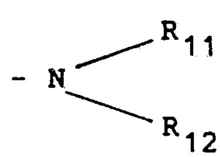

dans laquelle $R_{11}$ et $R_{12}$ sont des groupes alkyle identiques ou différents, linéaires ou ramifiés, de 1 à 3 atomes de carbone, de préférence de 1 à 2 atomes de carbone,
B représentant notamment

. un groupe de formule

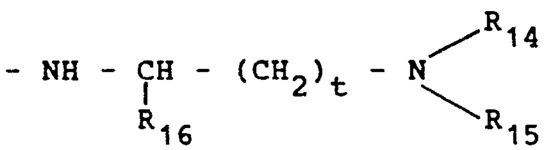

dans laquelle
t vaut de 1 à 3,
$R_{16}$ représente un atome d'hydrogène ou un groupe alkyle de 1 à 3 atomes de carbone, de préférence de 1 atome de carbone,
- $R_{14}$ et $R_{15}$ sont des groupes alkyle identiques ou différents de 1 à 3 atomes de carbone, de préférence de 1 à 2 atomes de carbone,
. ou B représentant un peptide comportant de 2 à 5 acides aminés et dont la partie C-terminale se présente sous forme d'un ester de formule - $COOR_{13}$ dans laquelle $R_{13}$ est un groupe alkyle de 1 à 2 atomes de carbone, ou un groupement benzyle,
pour obtenir un composé de formule 6

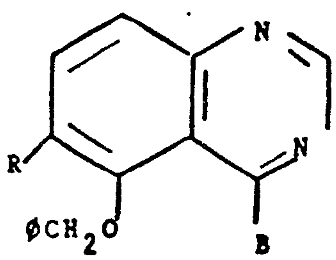

6

- on élimine le groupe benzyle du composé précédent, pour obtenir un composé de formule 7

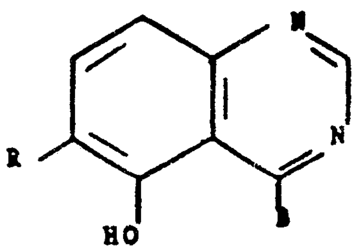

7

- on oxyde le composé précédent pour obtenir un monomère, de formule IV

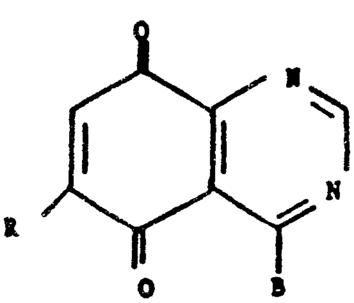

IV

dans laquelle R a la signification indiquée ci-dessus,
- on condense le composé précédent dans lequel R représente un groupe alcoxy de 1 à 5 atomes de carbone, notamment un groupe méthoxy,
* soit sur
. de l'ammoniaque,

. une amine primaire ou une amine secondaire de 1 à 3 atomes de carbone,
. la pipéridine,
. la pyrrolidine,
. une diamine cyclique de 4 atomes de carbone de formule

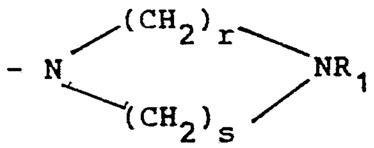

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,
. une amine cyclique de formule

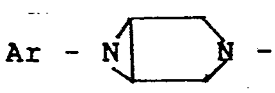

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
pour obtenir un monomère de formule II

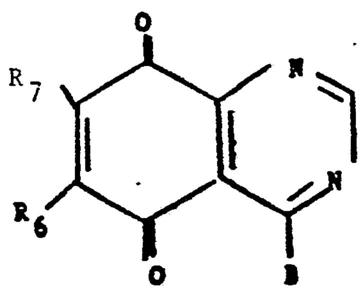

II

dans laquelle $R_7$ représente l'hydrogène
$R_6$ représente
. une amine primaire.
. un groupe amine secondaire ou tertiaire de 1 à 3 atomes de carbone,
. une pipéridine,
. une pyrrolidine,
. une diamine cyclique de 4 atomes de carbone de formule

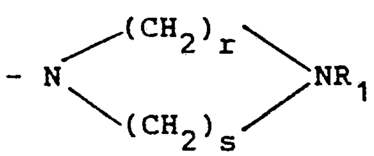

r et s étant des nombres entiers tels que le couple (r,s) représente (2,2) ou bien (r,s) représente (1,3), (3,1) ou (0,4),
dans laquelle $R_1$ représente un atome d'hydrogène ou un groupe alkyle de 1 à 3 atomes de carbone, notamment la pipérazine,
. une amine cyclique de formule

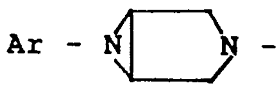

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,
* soit sur au moins deux équivalents d'aziridine de formule

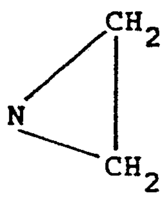

ou sur au moins deux équivalents d'une aziridine mono, bi, tri ou tétrasubstituée par des groupes alkyle, identiques ou différents, de 1 à 4 atomes de carbone,
pour obtenir un monomère de formule II, dans la formule duquel $R_6$ et $R_7$ représentent simultanément une aziridine de formule

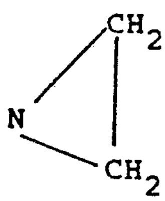

ou respectivement la même aziridine mono, bi, tri ou tétrasubstituées par des groupes alkyle, identiques ou différents, de 1 à 4 atomes de carbone.

3. Procédé, selon la revendication 1 , de préparation des composés de formule III

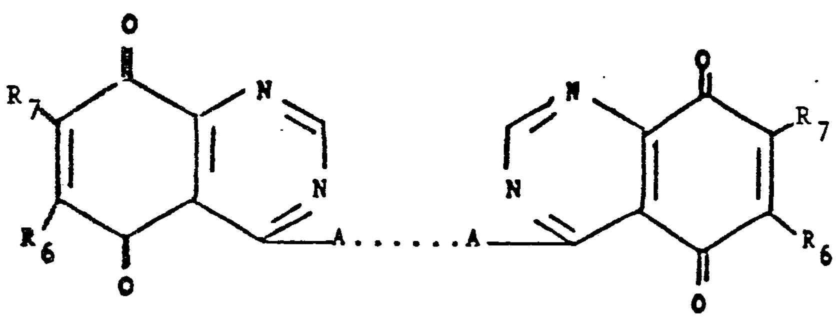

III

dans laquelle $R_6$, $R_7$ et A...A ont les significations indiquées à la revendication 1,
caractérisé en ce que on oxyde à l'aide d'un oxydant, notamment $KMnO_4$ le composé de formule 1

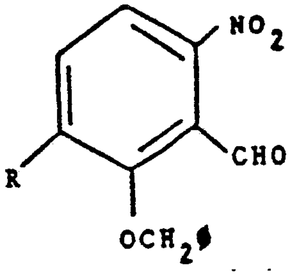

1

dans laquelle
- Ø représente un cycle benzénique,
- R représente un groupe alcoxy de 1 à 5 atomes de carbone,
pour obtenir un composé de formule 2

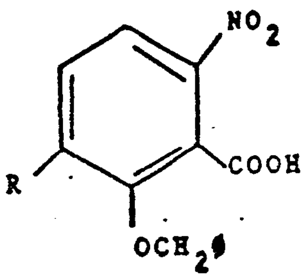

- on réduit le groupe $NO_2$ du composé précédent en groupe $NH_2$, notamment à l'aide de $FeSO_4$, pour obtenir un composé de formule 3

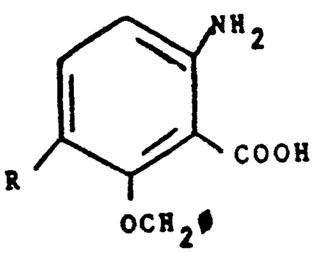

3

- on condense le composé précédent avec la triazine symétrique pour obtenir un composé de formule 4

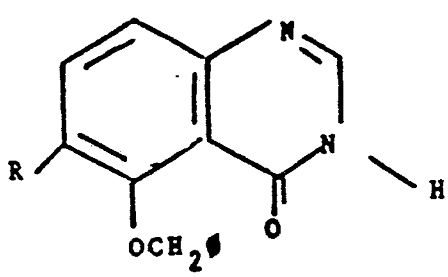

4

- on chlore le composé précédent, notamment à l'aide d'oxychlorure de phosphore, pour obtenir un composé de formule 5

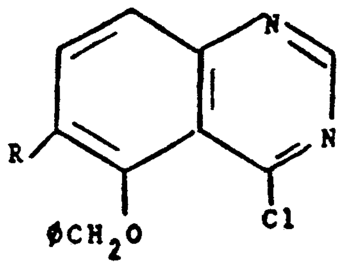

5

- on condense au moins deux molécules de composé de formule 5 sur une molécule de formule HA...AH, dans laquelle

soit A...A représente

- NH - Y - NH -

. Y représentant

* une chaîne alkyle linéaire de 2 à 10 atomes de carbone de préférence de 2 à 7,

* une chaîne de formule

$-(CH_2)_m - W - (CH_2)_n -$ dans laquelle m et n sont identiques ou différents et varient de 2 à 5, et W représente soit un groupe amine de formule

$-\underset{R_{17}}{N}-$ ,

$R_{17}$ représentant un atome d'hydrogène, un groupe alkyle de 1 à 3 atomes de carbone,

. un radical choisi parmi les formules suivantes

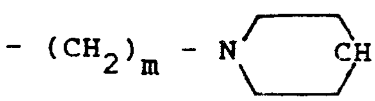

,

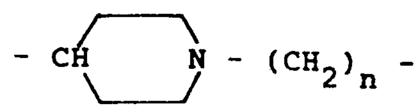

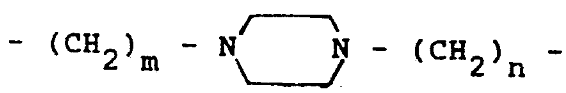

m et n identiques ou différents variant de 2 à 5, de préférence de 2 à 3,

m' valant de 0 à 3, et de préférence égal à 0 ou 1,

$$-(CH_2)_m - \underset{R_{19}}{N} - (CH_2)_n - \underset{R_{20}}{N} - (CH_2)_p -$$

m, n, p identiques ou différents variant de 2 à 5, de préférence de 2 à 4,

$R_{19}$ et $R_{20}$ identiques ou différents représentant un atome d'hydrogène ou une chaîne alkyle de 1 à 3 atomes de carbone,

$-CH_2 - (CH_2 - O - CH_2)_q - CH_2 -$

- q variant de 1 à 5, et valant de préférence 2,

. soit A...A représente une chaîne de formule

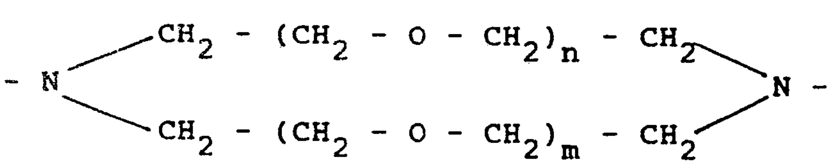

dans laquelle m et n sont identiques ou différents et varient de 2 à 5, de préférence de 2 à 3, pour obtenir le composé de formule 11

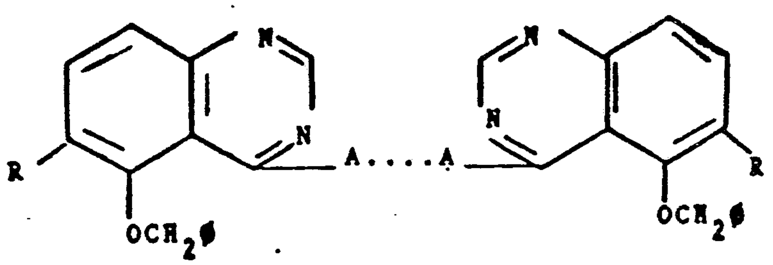

11

- puis on élimine les groupes benzyle du composé précédent pour obtenir le composé de formule 12

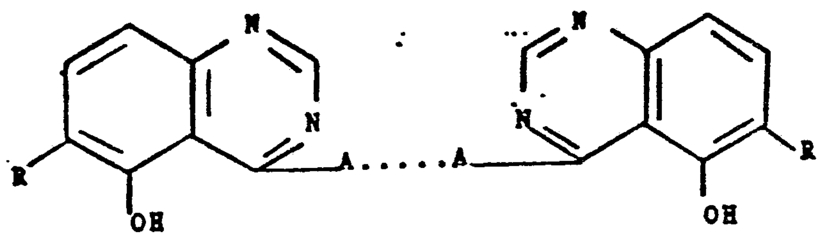

12

- on oxyde le composé précédent pour obtenir un dimère, de formule V :

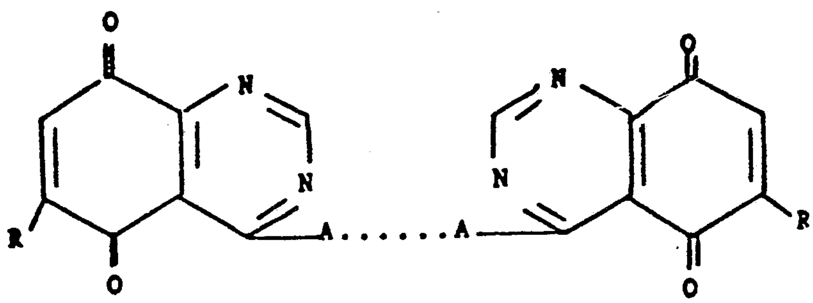

V

dans laquelle R a la signification indiquée ci-dessus,

- on condense le composé précédent dans lequel R représente un groupe alcoxy de 1 à 5 atomes de carbone, notamment un groupe méthoxy

* soit sur

. de l'ammoniaque,

. une amine primaire ou secondaire de 1 à 3 atomes de carbone,

. la pipéridine,

. la pyrrolidine,

. une diamine cyclique de 4 atomes de carbone de formule

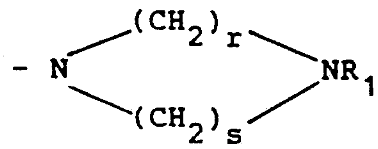

r et s étant des nombres entiers tels que le couple (r,s) représente (2,2) ou bien (r,s) représente (1,3), (3,1) ou (0,4),

dans laquelle $R_1$ représente un atome d'hydrogène ou un groupe alkyle de 1 à 3 atomes de carbone, notamment la pipérazine,

. une amine cyclique de formule

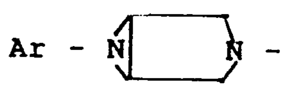

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,

pour obtenir un dimère de formule III

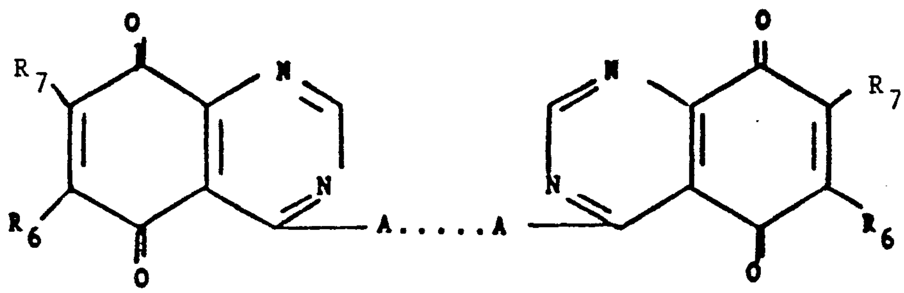
III

dans laquelle $R_7$ eprésente l'hydrogène

$R_6$ représente

. une amine primaire,
. un groupe amine secondaire ou tertiaire de 1 à 3 atomes de carbone,
. une pipéridine,
. une pyrrolidine,
. une diamine cyclique de 4 atomes de carbone de formule

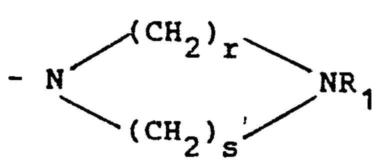

r et s étant des nombres entiers tels que le couple (r,s) représente (2,2) ou bien (r,s) représente (1,3), (3,1) ou (0.4),

dans laquelle $R_1$ représente un atome d'hydrogène ou un groupe alkyle de 1 à 3 atomes de carbone, notamment la pipérazine,

. une amine cyclique de formule

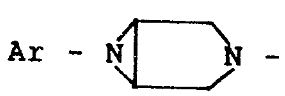

dans laquelle Ar représente un cycle benzénique non substitué ou substitué par un groupe alkyle de 1 à 2 atomes de carbone, avantageusement un méthyle, et/ou substitué par 1 ou 2 atomes d'halogène, notamment le chlore,

. soit sur au moins quatre équivalents d'une aziridine de formule

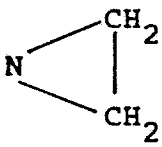

ou sur au moins quatre équivalents d'aziridine mono, bi, tri ou tétrasubstituée par des groupes alkyle, identiques ou différents, de 1 à 4 atomes de carbone, pour obtenir un dimère selon la revendication 7, dans lequel $R_6$ et $R_7$ représentent simultanément une aziridine de formule

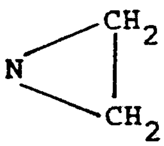

ou respectivement la même aziridine mono, bi, tri ou tétrasubstituées par des groupes alkyle, identiques ou différents, de 1 à 4 atomes de carbone,
- éventuellement on quaternarise l'azote tertiaire

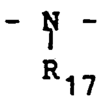

de dimères selon la revendication 7 de formule III, dans laquelle Y représente une chaîne de formule $-(CH_2)_m-W-(CH_2)_n-$, dans laquelle m et n sont identiques ou différents et varient de 2 à 5, et W représente un groupe amine de formule

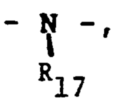

$R_{17}$ représentant un atome d'hydrogène, un groupe alkyle de 1 à 3 atomes de carbone.

4. Procédé selon la revendication 2, de préparation de composés de formule II, caractérisés en ce que B représente un groupe de formule $NH-R_4-X$, dans lequel $R_4$ représente une chaîne alkyle, linéaire ou ramifiée de 2 à 5 atomes de carbone et X représente un groupe amine de formule

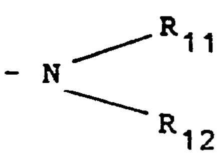 ,

dans laquelle $R_{11}$ et $R_{12}$ sont des groupes alkyle identiques ou différents, linéaires ou ramifiés, de 1 à 3 atomes de carbone, de préférence de 1 à 2 atomes de carbone.

5. Procédé selon la revendication 2, de préparation de composés de formule II, caractérisés en ce que $R_6$ et $R_7$ sont identiques et représentent simultanément un groupe aziridine de formule :

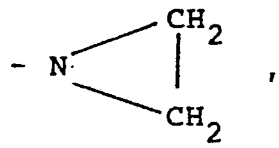

. ou un groupe aziridine mono, bi, tri ou tétrasubstitué par des groupes alkyle identiques ou différents, de 1 à 4 atomes de carbone, de préférence de 1 à 2 atomes de carbone.

6. Procédé selon la revendication 1, de préparation de composés répondant à la formule suivante :

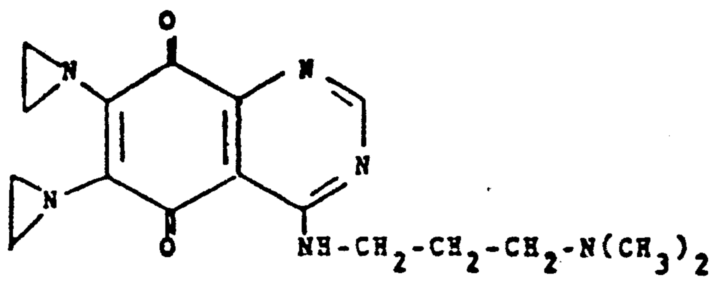

(63)

7. Procédé selon la revendication 3, de préparation de composés de formule III, caractérisés en ce que $R_6$ et $R_7$ sont identiques et représentent simultanément

. un groupe aziridine de formule

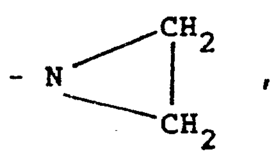

. ou un groupe aziridine mono, bi, tri ou tétrasubstitué par des groupes alkyle, identiques ou différents, de 1 à 4 atomes de carbone, de préférence de 1 à 2 atomes de carbone.

8. Procédé selon la revendication 3, de préparation de composés de formule III, caractérisés en ce que A...A représente une chaîne moléculaire de formule

- NH - Y - NH-, dans laquelle Y représente une chaîne alkyle linéaire de 2 à 10 atomes de carbone, de préférence de 2 à 7 atomes de carbone.

9. Procédé selon la revendication 3, de préparation de composés de formule III, caractérisés en ce que A...A représente une chaîne moléculaire de formule - NH - Y - NH-, dans laquelle Y représente une chaîne moléculaire de formule

$-(CH_2)_m - W - (CH_2)_n$, dans laquelle m et n sont identiques ou différents et varient de 2 à 5, et W représente un groupe amine de formule

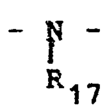

dans laquelle $R_{17}$ représente un atome d'hydrogène, ou un groupe alkyle de 1 à 3 atomes de carbone.

10. Procédé selon la revendication 3, de préparation de composés de formule III, caractérisés en ce que A...A représente une chaîne de formule -NH-Y-NH-, dans laquelle Y représente $-(CH_2)-(CH_2OCH_2)_q-CH_2-$, q variant de 1 à 5, et valant, de préférence 2.

11. Procédé selon les revendications 1 ou 3, de préparation de composés répondant aux formules suivantes :

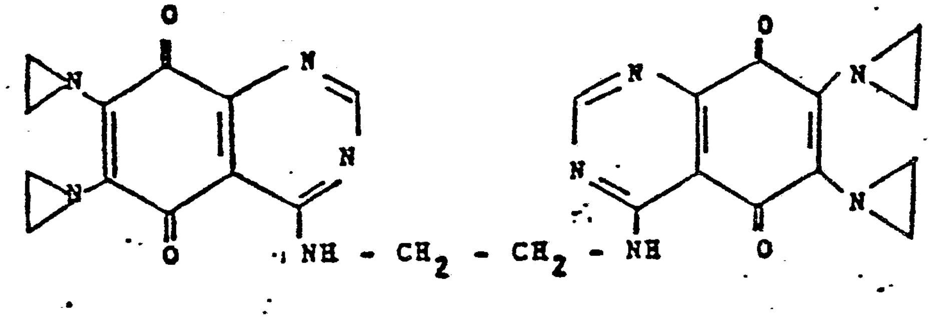

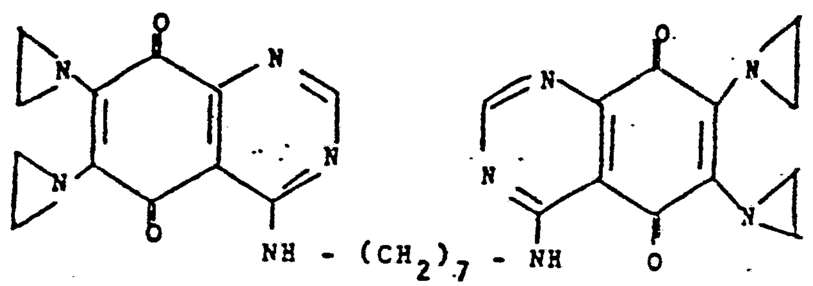

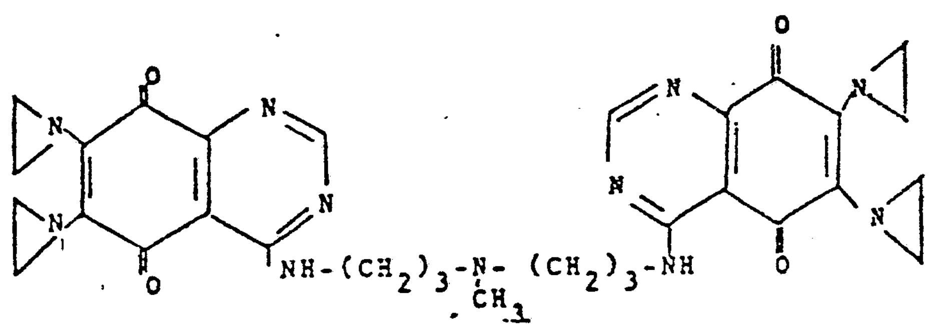

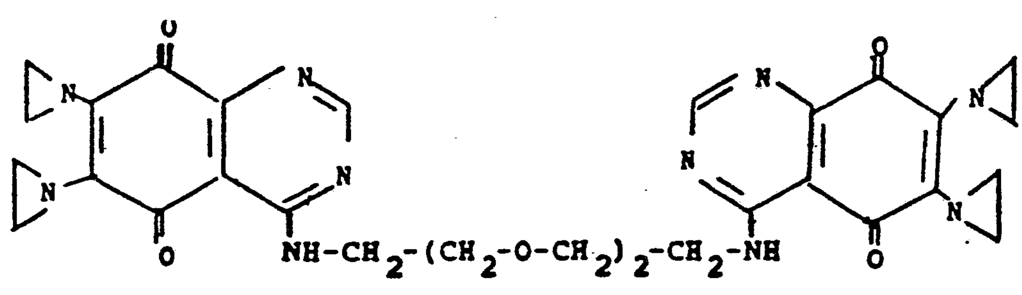

12. Procédé de préparation de composés de formule 2 :

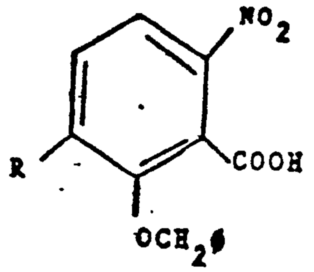

2

dans laquelle
- Ø représente un cycle benzénique,
- R représente un groupe alcoxy de 1 à 5 atomes de carbone,
caractérisés en ce que on oxyde à l'aide d'un oxydant, notamment $KMnO_4$ le composé de formule 1 :

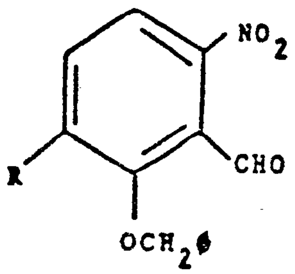

1

dans laquelle
- Ø représente un cycle benzénique,
- R représente un groupe alcoxy de 1 à 5 atomes de carbone.

13. Procédé de préparation du composé de formule 3 :

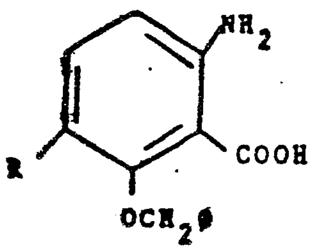

3

dans laquelle
- Ø représente un cycle benzénique,
- R représente un groupe alcoxy de 1 à 5 atomes de carbone,
caractérisés en ce que on oxyde à l'aide d'un oxydant, notamment $KMnO_4$ le composé de formule 1 :

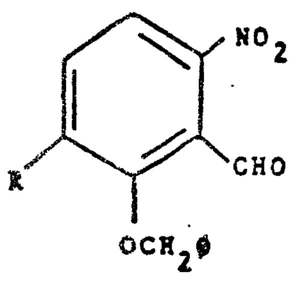

1

dans laquelle
- Ø représente un cycle benzénique,

- R représente un groupe alcoxy de 1 à 5 atomes de carbone,
pour obtenir un composé de formule 2 :

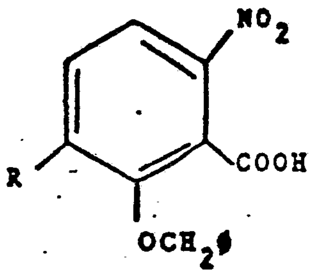

2

on réduit le groupe $NO_2$ du composé précédent en groupe $NH_2$, notamment à l'aide de $FeSO_4$.

14. Procédé de préparation du composé de formule 4 :

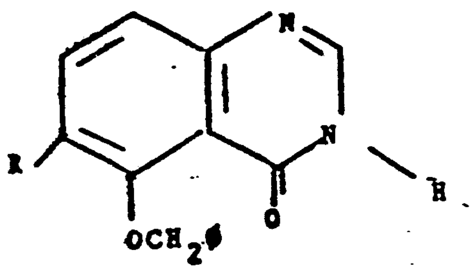

dans laquelle
- Ø représente un cycle benzénique,
- R représente un groupe alcoxy de 1 à 5 atomes de carbone,
caractérisés en ce que on oxyde à l'aide d'un oxydant, notamment $KMnO_4$ le composé de formule 1 :

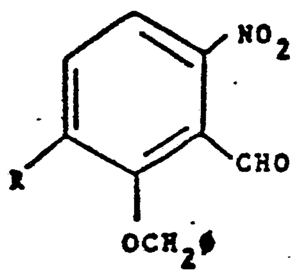

1

dans laquelle
- Ø représente un cycle benzénique,
- R représente un groupe alcoxy de 1 à 5 atomes de carbone,
pour obtenir un composé de formule 2 :

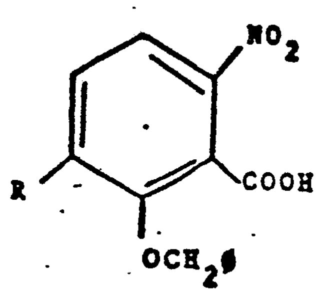

2

on réduit le groupe $NO_2$ du composé précédent en groupe $NH_2$, notamment à l'aide de $FeSO_4$, pour obtenir un composé de formule 3 :

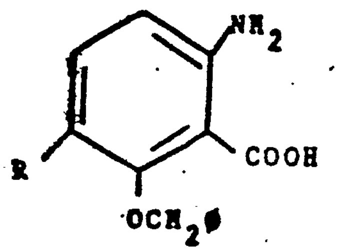

3

on condense le composé précédent avec la triazine symétrique.

15. Procédé de préparation du composé de formule 5 :

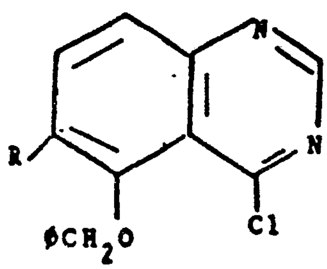

5

dans laquelle
- Ø représente un cycle benzénique,
- R représente un groupe alcoxy de 1 à 5 atomes de carbone.

caractérisés en ce que on oxyde à l'aide d'un oxydant, notamment $KMnO_4$ le composé de formule 1 :

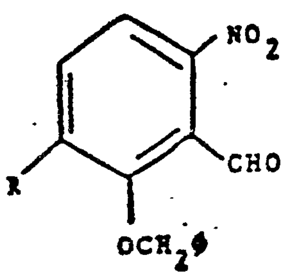

1

dans laquelle
- Ø représente un cycle benzénique,
- R représente un groupe alcoxy de 1 à 5 atomes de carbone,

pour obtenir un composé de formule 2 :

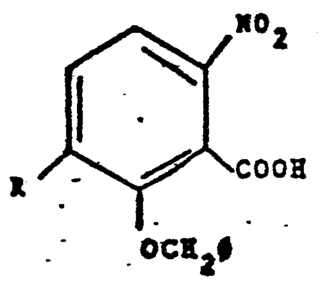

2

on réduit le groupe $NO_2$ du composé précédent en groupe $NH_2$, notamment à l'aide de $FeSO_4$, pour obtenir un composé de formule 3 :

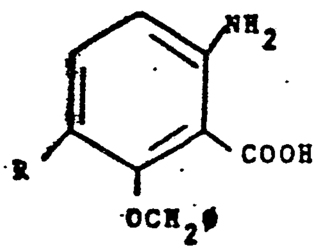

3

on condense le composé précédent avec la triazine symétrique pour obtenir un composé de formule 4 :

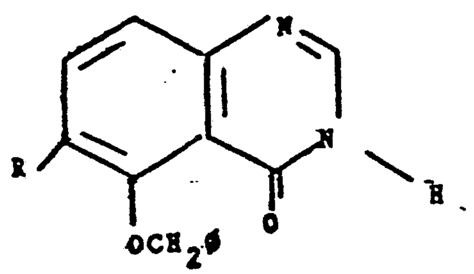

4

on chlore le composé précédent, notamment à l'aide d'oxychlorure de phosphore.

16. Procédé de préparation du composé de formule 6 :

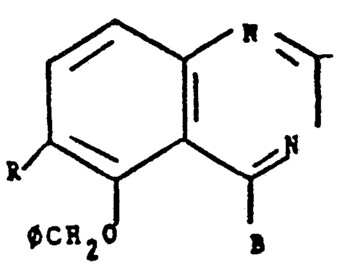

6

dans laquelle
- Ø représente un cycle benzénique,
- R représente un groupe alcoxy de 1 à 5 atomes de carbone,
- B a la signification indiquée à la revendication 1, caractérisés en ce que on oxyde à l'aide d'un oxydant, notamment $KMnO_4$ le composé de formule 1 :

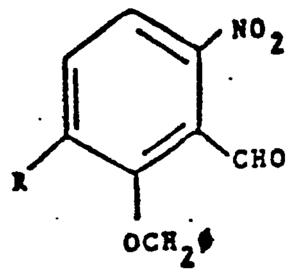

1

dans laquelle
- Ø représente un cycle benzénique,
- R représente un groupe alcoxy de 1 à 5 atomes de carbone,
pour obtenir un composé de formule 2 :

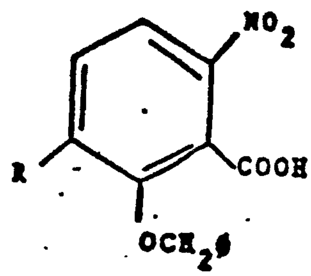

2

on réduit le groupe $NO_2$ du composé précédent en groupe $HN_2$, notamment à l'aide de $FeSO_4$,
pour obtenir un composé de formule 3 :

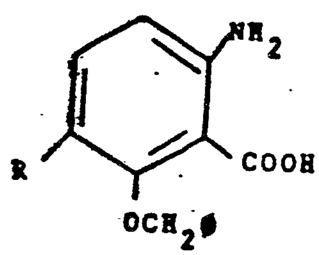

3

on condense le composé précédent avec la triazine symétrique pour obtenir un composé de formule 4 :

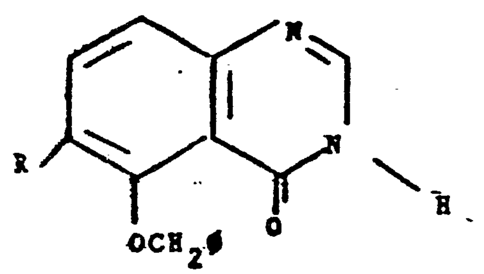

4

on chlore le composé précédent, notamment à l'aide d'oxychlorure de phosphore,
pour obtenir un composé de formule 5,
on condense le composé précédent sur un composé de formule BH dans lequel B a les significations indiquées à la revendication 1.

17. Procédé de préparation du composé de formule 7 :

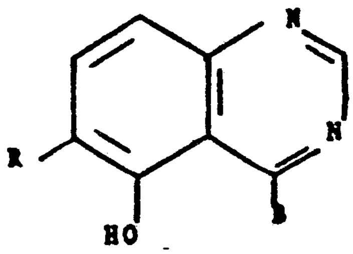

7

dans laquelle
- Ø représente un cycle benzénique,
- R représente un groupe alcoxy de 1 à 5 atomes de carbone,
- B a la signification indiquée à la revendication 1,

caractérisés en ce que on oxyde à l'aide d'un oxydant, notamment $KMnO_4$ le composé de formule 1 :

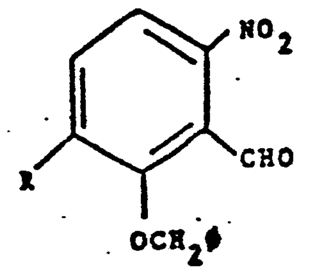

1

dans laquelle
- Ø représente un cycle benzénique,
- R représente un groupe alcoxy de 1 à 5 atomes de carbone,

pour obtenir un composé de formule 2 :

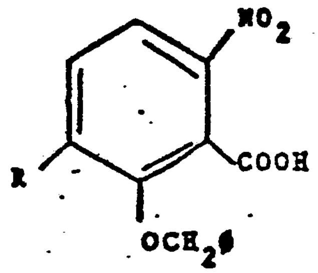

2

on réduit le groupe $NO_2$ du composé précédent en groupe $NH_2$, notamment à l'aide de $FeSO_4$, pour obtenir un composé de formule 3 :

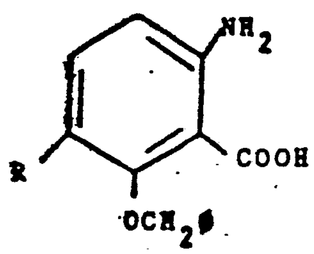

3

on condense le composé précédent avec la triazine symétrique pour obtenir un composé de formule 4 :

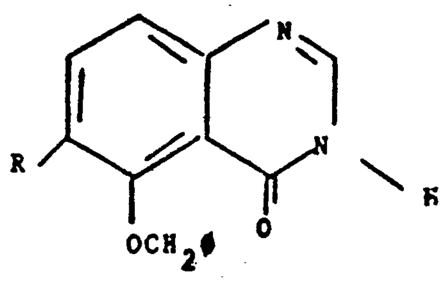

4

on chlore le composé précédent, notamment à l'aide d'oxychlorure de phosphore,
pour obtenir un composé de formule 5,
on condense le composé précédent sur un composé de formule BH dans lequel B a les significations indiquées à la revendicaiton 1,
pour obtenir un composé de formule 6,

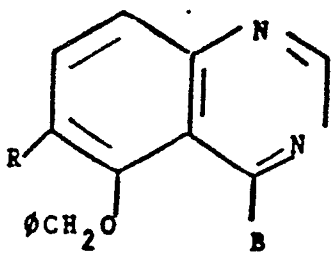

6

on élimine le groupe benzyle du groupe précédent.

18. Procédé de préparation du composé de formule IV :

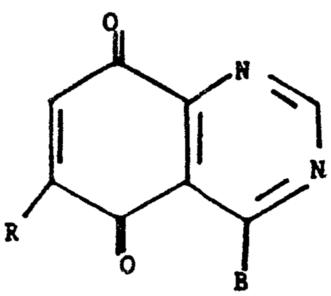

IV

dans laquelle
- R représente un groupe alcoxy de 1 à 5 atomes de carbone,
- B a la signification indiquée à la revendication 1,

caractérisés en ce que on oxyde à l'aide d'un oxydant, notamment $KMnO_4$ le composé de formule 1 :

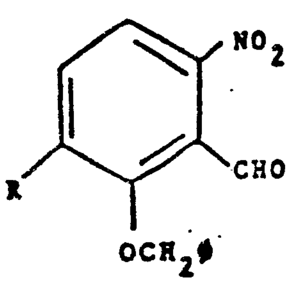

1

dans laquelle
- Ø représente un cycle benzénique,
- R représente un groupe alcoxy de 1 à 5 atomes de carbone,

pour obtenir un composé de formule 2 :

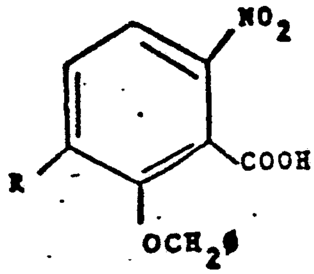

2

on réduit le groupe $NO_2$ du composé précédent en groupe $NH_2$, notamment à l'aide de $FeSO_4$, pour obtenir un composé de formule 3 :

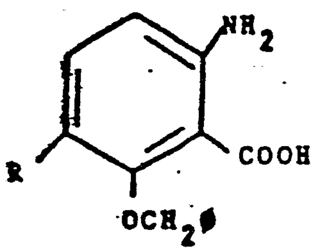

on condense le composé précédent avec la triazine symétrique pour obtenir un composé de formule 4 :

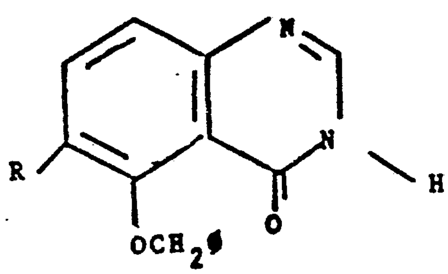

4

on chlore le composé précédent, notamment à l'aide d'oxychlorure de phosphore,
pour obtenir un composé de formule 5,
on condense le composé précédent sur un composé de formule BH dans lequel B a les significations indiquées à la revendication 1,
pour obtenir un composé de formule 6,

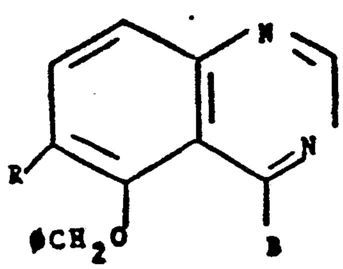

on élimine le groupe benzyle du groupe précédent, pour obtenir un composé de formule 7

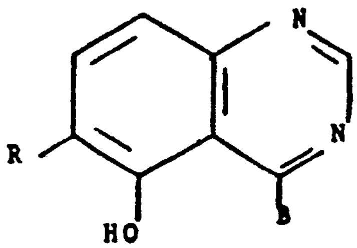

7

on oxyde le composé précédent.

19. Procédé selon la revendication 18, de préparation de composés répondant aux formules suivantes :

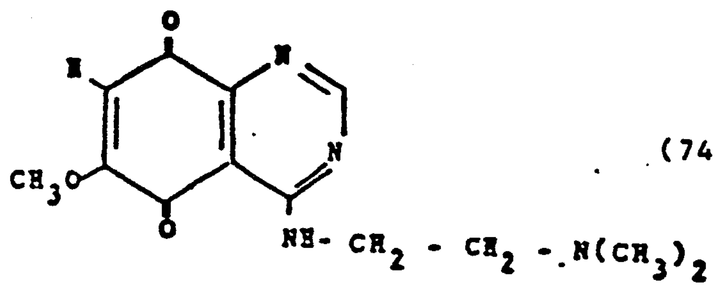

(74)

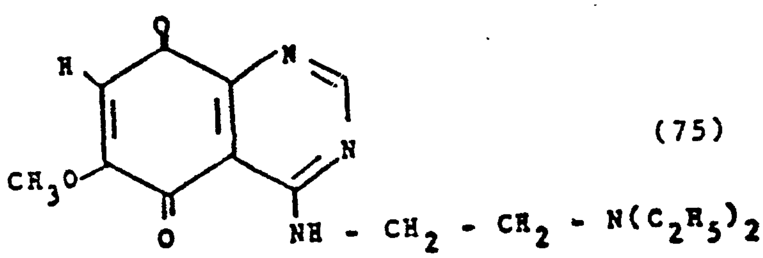

(75)

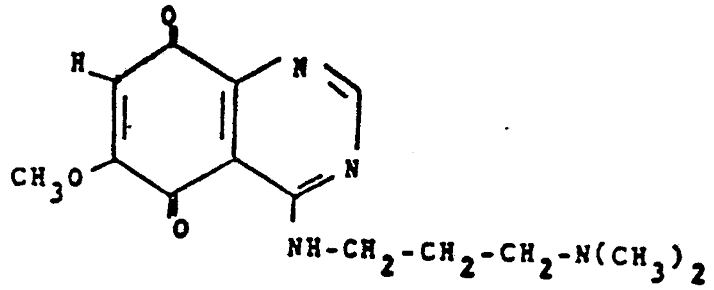

(62)

(76)

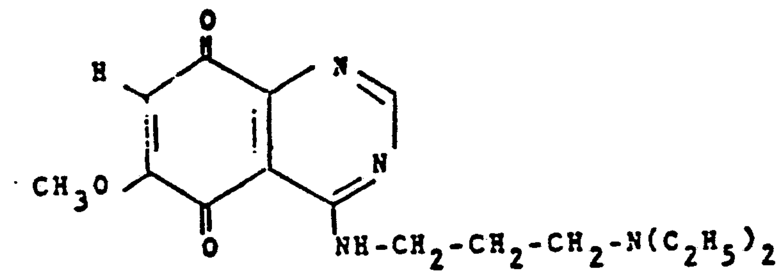

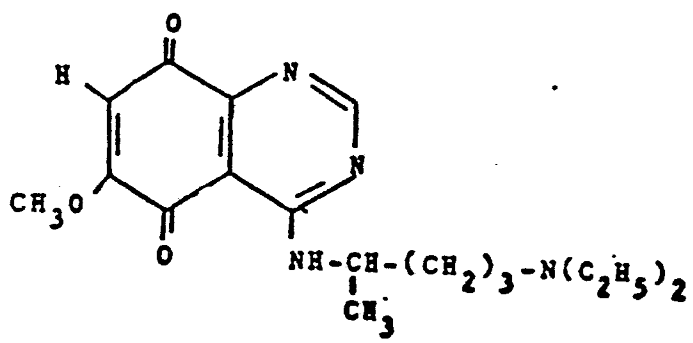

(77)

20. Procédé de préparation du composé de formule 11 :

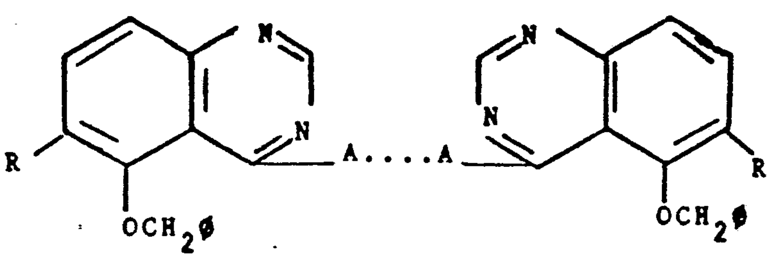

11

dans laquelle

- Ø représente un cycle benzénique,
- R représente un groupe alcoxy de 1 à 5 atomes de carbone,
- A...A a la signification indiquée à la revendication 1,
- caractérisé en ce que on condense le composé de formule 5

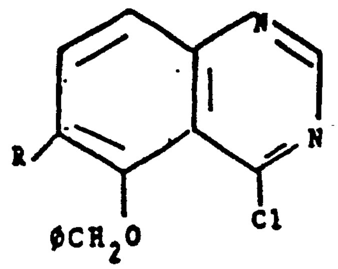

5

dans laquelle
- Ø représente un cycle benzénique,
- R représente un groupe alcoxy de 1 à 5 atomes de carbone
sur le composé de formule HA...AH,
dans laquelle A...A a la signification indiquée à la revendication 1.

21. Procédé de préparation du composé de formule 12 :

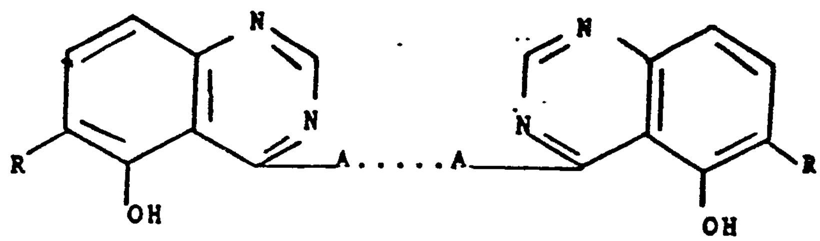

12

caractérisé en ce que on condense le composé de formule 5

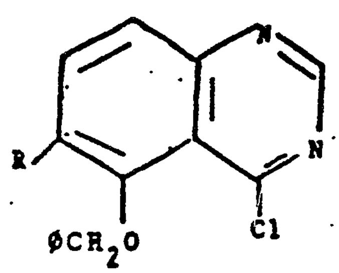

5

dans laquelle
- Ø représente un cycle benzénique,
- R représente un groupe alcoxy de 1 à 5 atomes de carbone
sur le composé de formule HA...AH,
dans laquelle A...A a la signification indiquée à la revendication 1,
pour obtenir le composé de formule 11 :

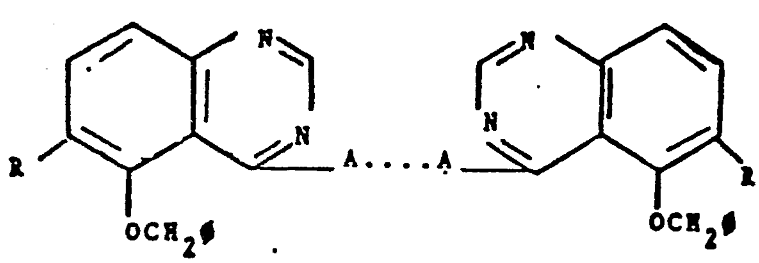

11

puis on élimine les groupes benzyle du composé précédent.

22. Procédé de préparation du composé de formule V :

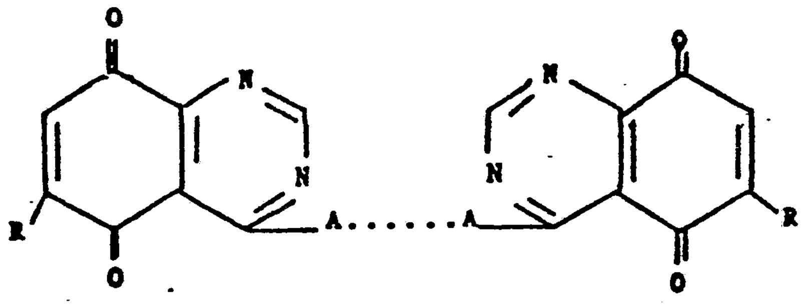

V

- R représente un groupe alcoxy de 1 à 5 atomes de carbone
- A...A a la signification indiquée à la revendication 1,

caractérisé en ce que on condense le composé de formule

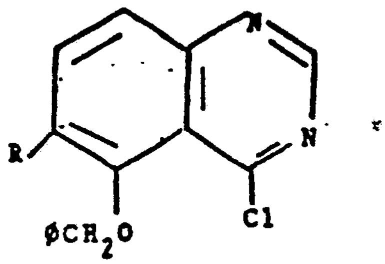

5

dans laquelle
- Ø représente un cycle benzénique,
- R représente un groupe alcoxy de 1 à 5 atomes de carbone

sur le composé de formule HA...AH,
dans laquelle A...A a la signification indiquée à la revendication 1,
pour obtenir le composé de formule 11 :

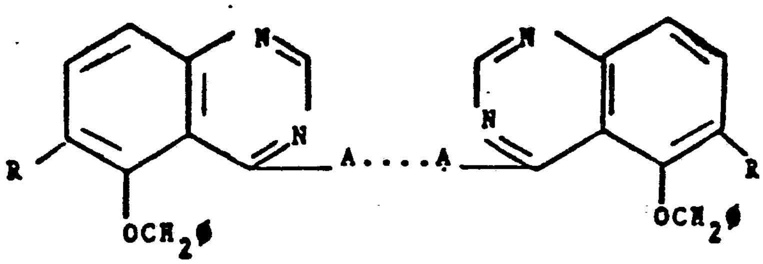

11

puis on élimine les groupes benzyle du composé précédent,
pour obtenir le composé de formule 12 :

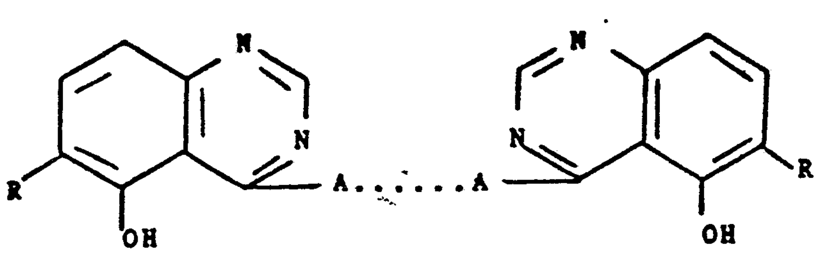

12

puis on oxyde le composé précédent.